(19) Europäisches Patentamt
European Patent Office
Office européen des brevets

(11) EP 4 574 023 A1

(12) EUROPEAN PATENT APPLICATION

(43) Date of publication:
25.06.2025 Bulletin 2025/26

(21) Application number: 24150461.2

(22) Date of filing: 05.01.2024

(51) International Patent Classification (IPC):
A61B 5/00 (2006.01)    A61B 5/145 (2006.01)
A61B 5/0245 (2006.01)

(52) Cooperative Patent Classification (CPC):
A61B 5/14532; A61B 5/0006; A61B 5/0245;
A61B 5/7264; A61B 5/7267; A61B 5/28;
G16H 50/20

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 21.12.2023 TW 112150091

(71) Applicant: Singular Wings Medical Co., Ltd.
Zhubei City, Hsinchu County 302 (TW)

(72) Inventors:
• LEE, Wei-Chung
Hsinchu County 302 (TW)
• CHEN, Cheng-I
Hsinchu County 302 (TW)
• LIU, Hsin-Che
Hsinchu County 302 (TW)
• LIU, Chang-Yuan
Hsinchu County 302 (TW)

(74) Representative: DTS Patent- und Rechtsanwälte PartmbB
Brienner Straße 1
80333 München (DE)

(54) METHOD AND COMPUTING DEVICE FOR NON-INVASIVELY ESTIMATING BLOOD GLUCOSE, DEVICE FOR NON-INVASIVELY MEASURING ELECTROCARDIOGRAM SIGNAL, AND NON-TRANSITORY COMPUTER READABLE STORAGE MEDIUM

(57) A method for non-invasively estimating blood glucose for estimating a blood glucose value of a user by a computing device (400A, 400B, 2600). The method includes receiving a plurality of electrocardiogram (ECG) waveforms of the user, extracting at least two first ECG features from each of the plurality of ECG waveforms of the user, respectively determining a first feature peak position corresponding to each of the first ECG features, calculating at least one peak distance between the plurality of the first feature peak positions, and estimating the blood glucose value of the user based on the peak distance. The first ECG features are selected from the group consisting of a P-wave, a Q-wave, an R-wave, an S-wave, a T-wave, and a U-wave. Furthermore, a computing device (2600) for non-invasively estimating blood glucose, a device (2700) for non-invasively measuring ECG signal, and a non-transitory computer readable storage medium are utilized for the method.

| | |
|---|---|
| Receiving a plurality of ECG waveforms | S610 |
| Extracting at least two first ECG features | S620 |
| Determining a first feature peak position corresponding to each of the first ECG features, respectively | S630 |
| Calculating at least one peak distance | S640 |
| Estimating the blood glucose value of the user based on the peak distance | S650 |

FIG. 6

EP 4 574 023 A1

**Description**

BACKGROUND OF THE INVENTION

1. Field of the Invention

The present disclosure relates to a method and a computing device for estimating, a device for measuring, and a computer-readable storage medium, and in particular to a method and a computing device for non-invasively estimating blood glucose, a device for non-invasively measuring electrocardiogram (ECG) signals, and a non-transitory computer readable storage medium.

2. Description of the Related Art

According to World Health Organization (WHO), the number of people with diabetes has increased from 108 million in 1980 to 422 million in 2014. In the U.S. alone 30 million adults are afflicted with diabetes, from which 7.2 million are not aware that they suffer from this condition. Therefore, the measurement and monitoring of blood glucose is an important topic in the medical field.

Conventional blood glucose measurements, whether measured continuously or not, are invasive. For instance, when people use a blood glucose meter or a continuous blood glucose monitor to measure blood glucose, the needle must be inserted into the body. One of the problems with invasive measurements is that the user suffers from physical pain causing a lot of discomfort. Therefore, users of blood glucose measurement devices are usually limited to diabetics who must control their blood glucose. In addition to the cost of the treatment medication, the cost of test strips, measuring devices, and readers must be added to the total cost. Thus, developing a method and a device for non-invasive estimating blood glucose will be of great benefit to diabetic patients.

In terms of conventional non-invasive blood glucose measurement, both hands of the user must be in contact with the measuring device as electrodes to form the V1 lead, so as to obtain a single-lead ECG signal. Imagine that every 15 minutes while eating, an eater would have to put down the cutlery and spend 1 minute with their hands taking an ECG signal. If the quality of the signal is not satisfactory during this process, the user needs to take another 1 minute to measure the signal again until the quality of the signal is adequate. Although it is a non-invasive measurement, it cannot achieve the purpose of continuous measurement, and therefore cannot know the detailed trend of changes in blood glucose.

In addition, the method of using ECG signals to estimate the blood glucose value of the user has extremely high requirements on the quality of the signals, and it is even more necessary to accurately detect P-waves, Q-waves, R-waves, S-waves, and T-waves for feature extraction to obtain precise results.

There are two main methods for measuring the blood glucose change based on ECG signals. One is the method based on features extracted from the morphology of ECG waveforms, such as QT interval, and the commonly used analysis ways are statistical analysis and machine learning. In the results of statistical analysis, QT interval and ST interval are highly correlated with hypoglycemia, while PR interval and ST interval are highly correlated with hyperglycemia. The other method is based on ECG signals itself as features. However, this method shows that the performance with different people varies greatly. Therefore, using the morphology of ECG signals as features will be more stable in performance than the direct use of ECG signals.

Although the method based on features extracted from the morphology of ECG waveforms is a superior method, it still only shows the relationship between hypoglycemia and QT interval (and/or ST interval) and the relationship between hyperglycemia and PR interval (and/or ST interval). That is, this method is still not able to estimate the blood glucose value of the user even if ST interval is highly correlated with both hypoglycemia and hyperglycemia.

Thus, it is desirable to have improvements to the non-invasive blood glucose measurement methods in order to estimate the blood glucose value of the user based on the ECG features extracted from ECG waveforms.

BRIEF SUMMARY OF THE INVENTION

An objective of the present disclosure is to provide technology conducive to non-invasively estimating a blood glucose value of a user with a plurality of electrocardiogram (ECG) waveforms. This technology provides an enhancement for accurately and non-invasively estimating the blood glucose value of the user with a plurality of ECG waveforms.

To achieve at least the above objective and more, the present disclosure provides a method for non-invasively estimating blood glucose which is suitable for estimating a blood glucose value of a user by a computing device. The method includes receiving a plurality of electrocardiogram (ECG) waveforms of a user, extracting at least two first ECG features from each of the plurality of ECG waveforms of the user, respectively determining a first feature peak position corresponding to each of the first ECG features, calculating at least one peak distance between the plurality of the first feature peak positions for each of the plurality of ECG waveforms, and estimating the blood glucose value of the user based on the peak distance. The first ECG features are selected from the group consisting of a P-wave, a Q-wave, an R-wave, an S-wave, a T-wave, and a U-wave.

In an embodiment, the method for non-invasively estimating blood glucose further includes calculating a calibrated blood glucose value of the user based on the blood glucose value of the user by an equation of calibrated blood glucose value.

In an embodiment, the method for non-invasively estimating blood glucose further includes calculating at least one peak-to-peak slope between the plurality of the first feature peak positions for each of the plurality of ECG waveforms. The step of estimating the blood glucose value of the user is further based on the peak-to-peak slope.

In an embodiment, the method for non-invasively estimating blood glucose further includes extracting at least three second ECG features from each of the plurality of ECG waveforms of the user, and calculating at least one occurrence rate based on the second ECG features. The step of estimating the blood glucose value of the user is further based on the occurrence rate. The second ECG features are selected from the group consisting of a P-wave, a Q-wave, an R-wave, an S-wave, a T-wave, and a U-wave.

In an embodiment, the method for non-invasively estimating blood glucose further includes extracting at least four third ECG features from each of the plurality of ECG waveforms of the user, and calculating at least one amplitude ratio based on the third ECG features. The step of estimating the blood glucose value of the user is further based on the amplitude ratio. The third ECG features are selected from the group consisting of a P-wave, a Q-wave, an R-wave, an S-wave, a T-wave, and a U-wave.

In an embodiment, the method for non-invasively estimating blood glucose further includes extracting at least one fourth ECG feature from each of the plurality of ECG waveforms of the user, and calculating at least one sharpness result based on the fourth ECG feature. The step of estimating the blood glucose value of the user is further based on the sharpness result. The fourth ECG feature is selected from the group consisting of a P-wave, a Q-wave, an R-wave, an S-wave, a T-wave, and a U-wave.

In an embodiment, the method for non-invasively estimating blood glucose further includes evaluating the quality of the plurality of ECG waveforms of the user, outputting an immediate blood glucose value of the user when the plurality of ECG waveforms of the user is evaluated as a normal ECG signal, and outputting a previous blood glucose value of the user when the plurality of ECG waveforms of the user is evaluated as a noise signal. The immediate blood glucose value of the user is the blood glucose value that is estimated immediately. The previous blood glucose value of the user is the blood glucose value that was estimated previously.

In an embodiment, the method for non-invasively estimating blood glucose further includes determining whether the number of the plurality of ECG waveforms of the user is less than a default value, and re-receiving the plurality of ECG waveforms of the user when the number of the plurality of ECG waveforms of the user is less than the default value.

Furthermore, the present disclosure provides a computing device for non-invasively estimating blood glucose which is suitable for electrically coupling with an electrocardiogram (ECG) measuring device in order to receive a plurality of ECG waveforms of a user from the ECG measuring device. The computing device includes a storage module and a blood glucose estimating module. The blood glucose estimating module is configured to be electrically coupled with the storage module. A plurality of codes is stored in the storage module. The blood glucose estimating module performs the steps of any one of the methods for non-invasively estimating blood glucose after the blood glucose estimating module executes the plurality of codes stored in the storage module.

Furthermore, the present disclosure provides a device for non-invasively measuring electrocardiogram (ECG) signal which is suitable for electrically coupling with a computing device in order to output a plurality of ECG waveforms of a user to the computing device. The device includes a measuring module and a signal transmitting module. The measuring module has a plurality of electrodes that are electrically connected to the user. The measuring module is configured to be used to measure the plurality of ECG waveforms of the user. The signal transmitting module is configured to be electrically coupled with the measuring module. The signal transmitting module is configured to be used to transmit the plurality of ECG waveforms of the user to the computing device and then make the computing device be able to perform the steps of any one of the methods for non-invasively estimating blood glucose.

A non-transitory computer readable storage medium is capable of completing any one of the methods for non-invasively estimating blood glucose after a computer device loads and executes a plurality of codes stored therein.

The present disclosure provides an improvement in the technical field about non-invasively estimating the blood glucose value of the user by utilizing a plurality of ECG waveforms and thus the present disclosure can accurately estimate the blood glucose value of the user with a non-invasive way.

BRIEF DESCRIPTION OF THE DRAWINGS

FIG. 1 is a waveform diagram illustrating two examples of a plurality of ECG waveforms that have been received from a user according to an embodiment of the present disclosure.

FIG. 2 is a waveform diagram illustrating an example of the plurality of the ECG features that are extracted from each of the plurality of ECG waveforms according to an embodiment of the present disclosure.

FIG. 3 is a waveform diagram illustrating calculating at least one peak distance between the plurality of the first feature peak positions for each of the plurality of ECG waveforms according to an embodiment of the present disclosure.

FIG. 4 is a schematic diagram illustrating an example of a computing device that is electrically coupled with an ECG measuring device and with at least one of a machine learning model, a neural network model, and a convolution neural network model according to an embodiment of the present disclosure.

FIG. 5 is a schematic diagram illustrating an example of the calculating unit that is electrically coupled with the determining unit and the estimating unit according to an embodiment of the present disclosure.

FIG. 6 is a flowchart illustrating a first example of the method for non-invasively estimating blood glucose according to an embodiment of the present disclosure.

FIG. 7A is a detailed flowchart illustrating a method of estimating the blood glucose value of the user based on the peak distance according to an embodiment of the present disclosure.

FIG. 7B is a detailed flowchart illustrating a method of estimating the blood glucose value of the user based on the peak distance according to an embodiment of the present disclosure.

FIG. 8A is a schematic diagram illustrating an example of the machine learning model according to an embodiment of the present disclosure.

FIG. 8B is a schematic diagram illustrating an example of the neural network model according to an embodiment of the present disclosure.

FIG. 8C is a schematic diagram illustrating an example of the convolution neural network model according to an embodiment of the present disclosure.

FIG. 8D is a schematic diagram illustrating an example of the convolution layer according to an embodiment of the present disclosure.

FIG. 9 is a flowchart illustrating a second example of the method for non-invasively estimating blood glucose according to an embodiment of the present disclosure.

FIG. 10 is a flowchart illustrating a third example of the method for non-invasively estimating blood glucose according to an embodiment of the present disclosure.

FIG. 11 is a flowchart illustrating a fourth example of the method for non-invasively estimating blood glucose according to an embodiment of the present disclosure.

FIG. 12 is a detailed flowchart illustrating a method of calculating at least one occurrence rate based on the second ECG features according to an embodiment of the present disclosure.

FIG. 13 is a waveform diagram illustrating calculating at least one occurrence rate based on the second ECG features according to an embodiment of the present disclosure.

FIG. 14A is a detailed flowchart illustrating a method of estimating the blood glucose value of the user based on the peak distance and the occurrence rate according to an embodiment of the present disclosure.

FIG. 14B is a detailed flowchart illustrating a method of estimating the blood glucose value of the user based on the peak distance and the occurrence rate according to an embodiment of the present disclosure.

FIG. 15 is a flowchart illustrating a fifth example of the method for non-invasively estimating blood glucose according to an embodiment of the present disclosure.

FIG. 16 is a detailed flowchart illustrating a method of calculating at least one amplitude ratio based on the third ECG features according to an embodiment of the present disclosure.

FIG. 17A is a waveform diagram illustrating an example of calculating at least one amplitude ratio based on the third ECG features according to an embodiment of the present disclosure.

FIG. 17B is a waveform diagram illustrating another example of calculating at least one amplitude ratio based on the third ECG features according to an embodiment of the present disclosure.

FIG. 18A is a detailed flowchart illustrating a method of estimating the blood glucose value of the user based on the peak distance and the amplitude ratio according to an embodiment of the present disclosure.

FIG. 18B is a detailed flowchart illustrating a method of estimating the blood glucose value of the user based on the peak distance and the amplitude ratio according to an embodiment of the present disclosure.

FIG. 19 is a flowchart illustrating a sixth example of the methods for non-invasively estimating blood glucose according to an embodiment of the present disclosure.

FIG. 20 is a detailed flowchart illustrating a method of calculating the sharpness result based on the fourth ECG feature according to an embodiment of the present disclosure.

FIG. 21 is a waveform diagram illustrating calculating the sharpness result based on the fourth ECG feature according to an embodiment of the present disclosure.

FIG. 22A is a detailed flowchart illustrating a method of estimating the blood glucose value of the user based on the peak distance and the sharpness result according to an embodiment of the present disclosure.

FIG. 22B is a detailed flowchart illustrating a method of estimating the blood glucose value of the user based on the peak distance and the sharpness result according to an embodiment of the present disclosure.

FIG. 23 is a schematic diagram illustrating another example of a computing device that is electrically coupled with an ECG measuring device and with at least one of a machine learning model, a neural network model, and a convolution neural network model according to an embodiment of the present disclosure.

FIG. 24 is a flowchart illustrating a seventh example of the method for non-invasively estimating blood glucose according to an embodiment of the present disclosure.

FIG. 25 is a flowchart illustrating an eighth example of the method for non-invasively estimating blood glucose according to an embodiment of the present disclosure.

FIG. 26 is a schematic diagram illustrating an example of a computing device for non-invasively estimating blood glucose that is electrically coupled with at least one of an ECG sensor, an ECG monitoring device, and a server and with at least one of a displaying device and a server according to an embodiment of the present disclosure.

FIG. 27 is a schematic diagram illustrating an example of a device for non-invasively measuring ECG signal that is electrically coupled with at least one of a non-invasively estimating blood glucose device, a computing device, and a server according to an embodiment of the present disclosure.

FIG. 28 is a schematic diagram illustrating that the blood glucose value of the user is estimated based on the plurality of ECG waveforms that have been received from the user according to an embodiment of the present disclosure.

FIG. 29A is a schematic diagram illustrating a comparison result for the blood glucose value that is estimated by the present disclosure compared to another device.

FIG. 29B is a schematic diagram illustrating another comparison result for the blood glucose value that is estimated by the present disclosure compared to another device.

## DETAILED DESCRIPTION OF THE INVENTION

The present disclosure is described in detail with the following embodiments and the accompanying drawings in order to help those who have ordinary knowledge and skill in the field of the technology understand the purposes, features, and efficacies of the present disclosure.

Before the present disclosure is described in detail, it should be noted that the same components or steps may be denoted by the same reference numeral in the following description.

It should be also noted that in the context of the present disclosure, the terms such as "first", "second", "third", "fourth", "fifth", and "sixth" are used to differentiate between components instead of being not used to limit the components themselves or to indicate a particular ordering of the components.

It should be also noted that the various steps described herein may be performed in sequence, in reverse order, or by appropriately changing or skipping the step during controlling and processing.

It should be also noted that the phrase "the first step may be performed in sequence after the second step is performed" may represent that after the second step is performed, the first step may be performed directly or be performed after performing another step (e.g., the third step) first.

Since a lot of different physiological signals may be generated by the human body itself and a lot of medical devices or sensors are capable of converting the physiological signals into a specific electric signal, it is possible to signally process the physiological signals by electronic devices in order to obtain important clinical information.

For example, since the heart itself consists of a plurality of muscles that beat spontaneously and contract rhythmically, a small amount of electrical current is generated when the plurality of muscles of the heart beat and contract, and then the electrical current is reflected to the surface of the body through the electrically conductive tissues and bodily fluids around the heart. Therefore, it is possible to convert the voltage changes caused by the plurality of muscles of the heart into a corresponding electrocardiogram by a related electronic device. More specifically, it is possible to receive and record the electrical signals that are caused by the plurality of muscles of the heart by the plurality of electrodes that are in contact with the skin of the human body.

The methods provided in the present disclosure are suitable for a signal processing for the plurality of ECG waveforms in order to estimate a blood glucose value based on the plurality of ECG waveforms. Since a plurality of ECG waveforms of a user can be received by a non-invasively measuring device, the methods provided in the present disclosure can estimate a blood glucose value of the user in a non-invasive manner.

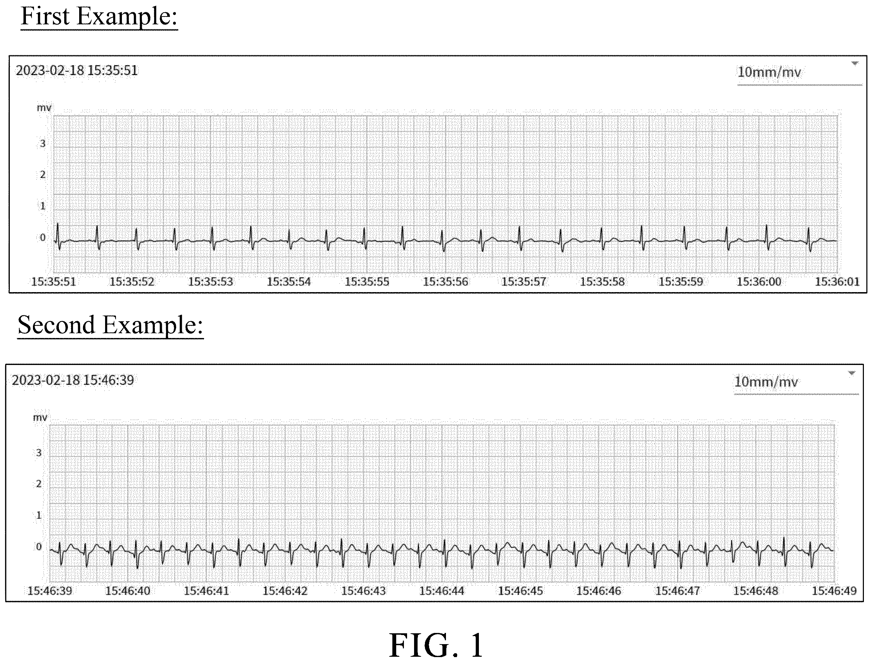

Referring to FIG. 1, FIG. 1 is a waveform diagram illustrating two examples of the plurality of ECG waveforms that have been received from the user. The horizontal axis in FIG. 1 represents the time (in seconds) and the vertical axis in FIG. 1 represents the amplitude value of the ECG waveform (in millivolts).

As shown in FIG. 1, the first example shows that the number of the plurality of ECG waveforms that have been received from the user during 10 seconds is 20. That is, the first example as shown in FIG. 1 can indicate that the heart rate of the user is 120 beats per minute. As shown in FIG. 1, the second example shows that the number of the plurality of ECG waveforms that have been received from the user during 10 seconds is 30. That is, the second example as shown in FIG. 1 can indicate that the heart rate of the user is 180 beats per minute.

The number of the plurality of ECG waveforms that have been received from the user during a period may vary depending on different users. For instance, the first example and the second example as shown in FIG. 1 may respectively indicate that the plurality of ECG waveforms is received from two different users. In addition, the number of the plurality of ECG waveforms that have been received during a period may also vary depending on the same user with different states. For instance, the first example may indicate that the plurality of ECG waveforms is received from a user in a normal state, and the second example may indicate that the plurality of ECG waveforms is received from the same user in a state of heightened emotion such as nervousness, excitement, or fear.

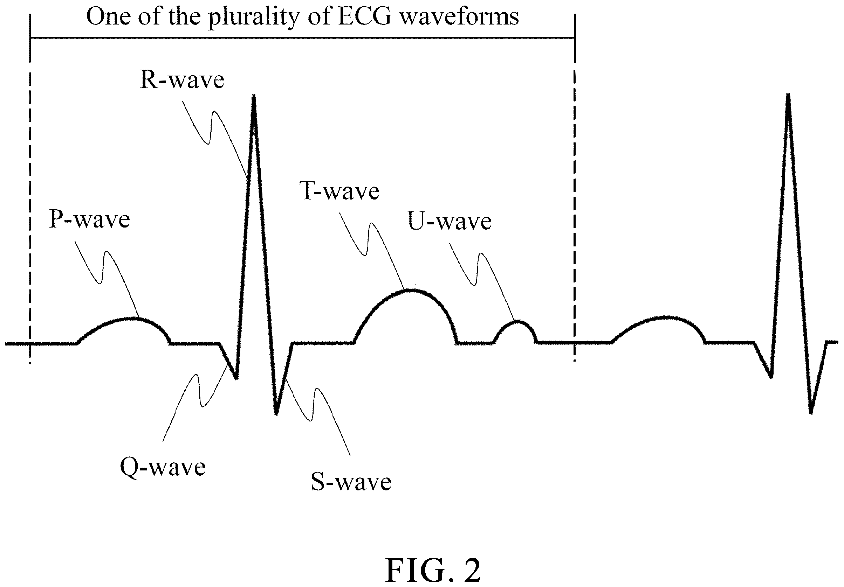

Referring to FIG. 2, FIG. 2 is a waveform diagram illustrating an example of the plurality of the ECG features that are extracted from each of the plurality of ECG waveforms. The horizontal axis in FIG. 2 represents the time (in seconds) and the vertical axis in FIG. 2 represents the amplitude value of the plurality of ECG waveform (in millivolts). As shown in FIG. 2, each of the plurality of ECG waveforms that is received may have a plurality of ECG features such as a P-wave, a Q-wave, an R-wave, an S-wave, a T-wave, and a U-wave, respectively. The plurality of ECG features may be used to represent the voltage changes caused by the plurality of muscles of the heart.

Since the plurality of muscles of the heart may be contracted due to an atrial depolarization, a first deviation (i.e., a first voltage change) occurs in an electrocardiogram waveform and it may be referred to as a P-wave. Since the plurality of muscles of the heart may be contracted due to a left ventricular depolarization and a right ventricular depolarization, the second deviation (i.e., the second voltage change) occurs in the ECG waveform and it may be referred to as a QRS waveform. The amount of change in the second deviation is significantly more than the amount of change in the first deviation. There are usually three deviations in the QRS wave group, namely, the first downward deviation, the first upward deviation, and the second downward deviation. The first downward deviation can be referred to as a Q-wave, the first upward deviation can be referred to as an R-wave, and the second downward deviation can be referred to as an S-wave. Since the plurality of muscles of the heart may be contracted due to a ventricular repolarization, the third deviation (i.e., the third voltage change) occurs in the ECG waveform can be referred to as a T-wave. The deviation (i.e., the fourth voltage change) immediately following the T-wave may be referred to as a U-wave.

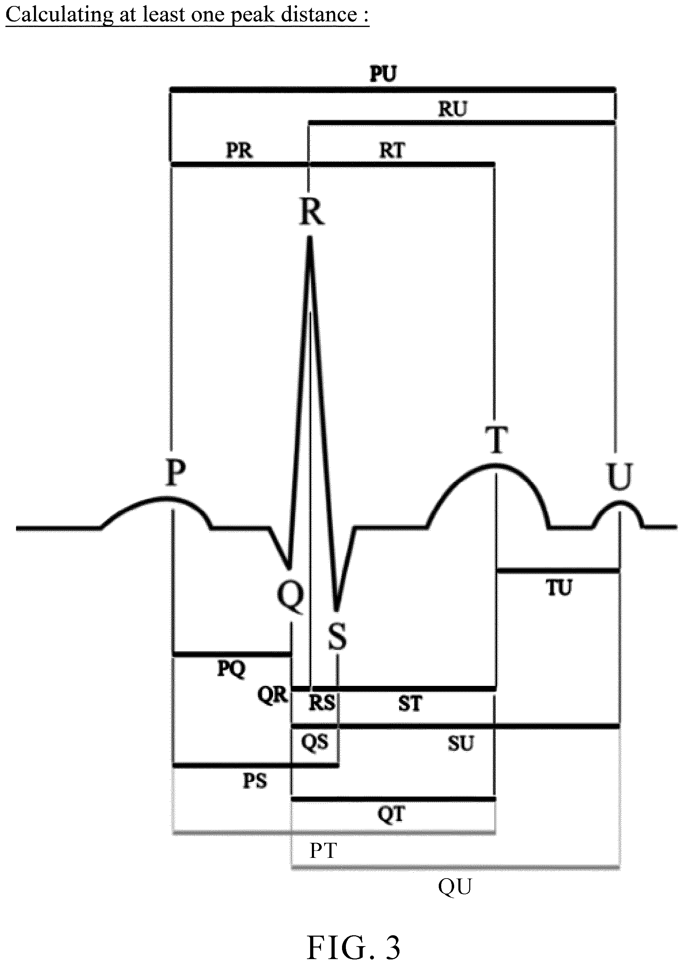

Referring to FIG. 3, FIG. 3 is a waveform diagram illustrating calculating at least one peak distance between the plurality of the first feature peak positions for each of the plurality of ECG waveforms according to an embodiment of the present disclosure. It should be noted that although FIG. 3 illustrates how the peak distance is calculated by using only one ECG waveform as an example, the peak distance for each of the plurality of ECG waveforms that have been received can be calculated in the same way as shown in FIG. 3.

As shown in FIG. 3, since the plurality of ECG waveforms that are received have the plurality of ECG features such as P-waves, Q-waves, R-waves, S-waves, T-waves, and U-waves, the peak distance can be calculated based on the plurality of ECG features. In order to calculate the peak distance, it is necessary to define the specific position of each of the plurality of ECG features first.

In some embodiments, the specific position of the P-wave may refer to a point where the amplitude value changes the most in the first voltage change, i.e., the maximum of the P-wave; the specific position of the Q-wave may refer to a point where the amplitude value changes the most in the first downward deviation that is in the second voltage change, i.e., the minimum of the Q-wave; the specific position of the R-wave may refer to a point where the amplitude value changes the most in the first upward deviation that is in the second voltage change, i.e., the maximum of the R-wave; the specific position of the S-wave may refer to a point where the amplitude value changes the most in the second downward deviation that is in the second voltage change, i.e., the minimum of the S-wave; the specific position of the T-wave may refer to a point where the amplitude value changes the most in the third voltage change, i.e., the maximum of the absolute value of the T-wave; the specific position of the U-wave may refer to a point where the amplitude value changes the most in the fourth voltage change, i.e., the maximum of the U-wave.

Based on the above, the peak distance between the P-wave and the Q-wave may be a difference between the specific position of the P-wave and the specific position of the Q-wave, i.e., a time difference between the maximum of the P-wave and the minimum of the Q-wave; the peak distance between the Q-wave and the S-wave may be a difference between the specific position of the Q-wave and the specific position of the S-wave, i.e., a time difference between the minimum of the Q-wave and the minimum of the S-wave; and the peak distance between the S-wave and the T-wave may be a difference between the specific position of the S-wave and the specific position of the T-wave, i.e., a time difference between the minimum of the S-wave and the maximum of the absolute value of the T-wave.

For example, as shown in FIG. 3, if the time point where the maximum of the R-wave occurs is 13:44:20.234 and the time point where the maximum of the absolute value of the T-wave occurs is 13:44:20.385, then the result of the calculation of the peak distance between the R-wave and the T-wave is 0.151 seconds (i.e., the time difference between the maximum of the R-wave and the maximum of the absolute value of the T-wave).

Therefore, a peak distance between each of the plurality of ECG features can be calculated by respectively calculating a time difference between two ECG features, wherein the units of the peak distance can be seconds or milliseconds.

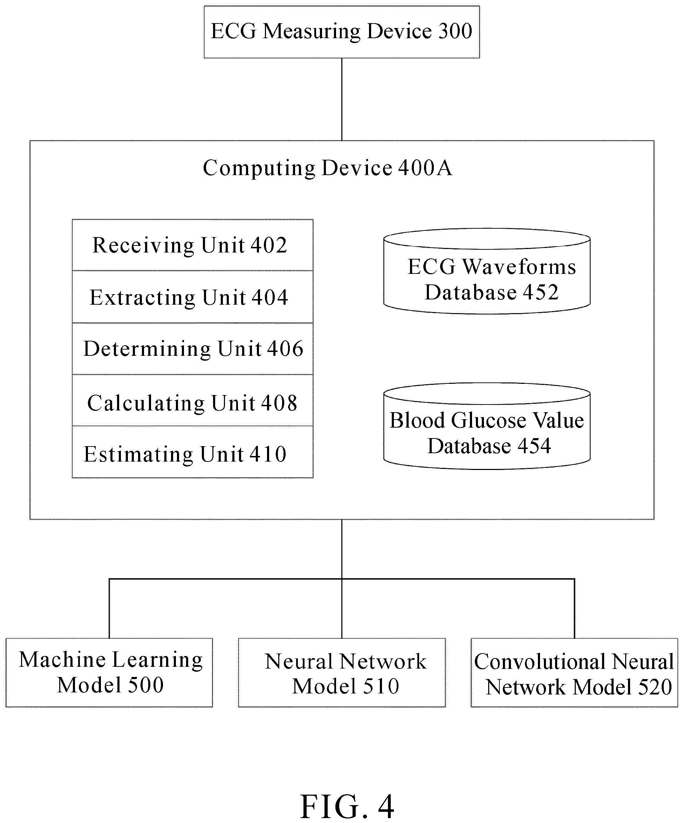

Referring to FIG. 4, FIG. 4 is a schematic diagram illustrating an example of a computing device 400A that is signally connected with an ECG measuring device 300 and with at least one of a machine learning model 500, a neural network model 510, and a convolution neural network model 520 according to an embodiment of the present disclosure.

Since the computing device 400A is electrically coupled with the ECG measuring device 300, the computing device 400A may receive the plurality of ECG waveforms of the user from the ECG measuring device 300. In some embodiments, the computing device 400A may receive the plurality of ECG waveforms of the user from the ECG measuring device 300 via a physical signal line connection. For example, the physical signal line connection may be a network signal line connection that conforms with an Internet Protocol (IP), but is not limited thereto. In addition, in some embodiments, the computing device 400A may also receive the plurality of ECG waveforms of the user from the ECG measuring device 300 via a virtual signal line connection. For example, the virtual signal line connection may be a Wi-Fi connection that conforms with the wireless network protocol, but is not limited thereto.

In some embodiments, the computing device 400A may also be electrically coupled with other devices that are capable of providing the plurality of ECG waveforms (e.g., wearable measurement devices) to receive the plurality of ECG waveforms of the user from other devices (not shown).

The computing device 400A may include a receiving unit 402, an extracting unit 404, a determining unit 406, a calculating unit 408, and an estimating unit 410, such that the computing device 400A may perform a series of specific signal processing on the plurality of ECG waveforms of the user that have been received. That is, after the computing device 400A receives the plurality of ECG waveforms of the user, the computing device 400A may estimate a blood glucose value of the user based on the plurality of ECG waveforms of the user.

The receiving unit 402 may be configured to receive the plurality of ECG waveforms of the user. That is, the computing device 400A may receive the plurality of ECG waveforms of the user from the ECG measuring device 300 or other devices capable of providing the plurality of ECG waveforms, via a wired or wireless signal transmission path, by the receiving unit 402.

The extracting unit 404 may be configured to extract the plurality of ECG features from each of the plurality of ECG waveforms. That is, the extracting unit 404 may extract from each of the plurality of ECG waveforms respectively in a time-sequential manner in order to extract the plurality of ECG features from each of the plurality of ECG waveforms. The plurality of ECG features that have been extracted may include P-waves, Q-waves, R-waves, S-waves, T-waves, and U-waves (as shown in FIG. 2), but are not limited thereto. In some embodiments, a plurality of first ECG features, a plurality of second ECG features, and/or a plurality of third ECG features may be respectively extracted from each of the plurality of ECG waveforms by the extracting unit 404. Thus, taking the plurality of first ECG features as an example, the extracting unit 404 may extract the plurality of first ECG features from each of the plurality of ECG waveforms, which may include a P-wave, a Q-wave, an R-wave, an S-wave, a T-wave, and a U-wave. In some embodiments, the extracting unit 404 may input the plurality of ECG waveforms of the user into the convolutional neural network model 520 and then each of the plurality of ECG features is extracted by the convolutional neural network model 520.

The determining unit 406 may be configured to determine a feature peak position corresponding to each of the plurality of ECG features. In some embodiments, the determining unit 406 may respectively determine a first feature peak position corresponding to each of the plurality of first ECG features, wherein the plurality of first ECG features may include a P-wave, a Q-wave, an R-wave, an S-wave, a T-wave, and a U-wave. For example, the determining unit 406 may respectively determine a position of the maximum of the P-wave, a position of the minimum of the Q-wave, a position of the maximum of the R-wave, a position of the minimum of the S-wave, a position of the maximum of the absolute value of the T-wave, and a position of the maximum of the U-wave.

The calculating unit 408 may be configured to calculate a peak distance between the plurality of feature peak positions. In some embodiments, the calculating unit 408 may respectively calculate the peak distance between two of the plurality of first feature peak positions, such as the peak distance between the position of the maximum of the P-wave and the position of the minimum of the Q-wave, the peak distance between the position of the minimum of the Q-wave and the position of the minimum of the S-wave and/or the peak distance between the position of the minimum of the S-wave and the position of the maximum of the absolute value of the T-wave, but is not limited thereto. The calculating unit 408 may respectively calculate the peak distance between any two of the plurality of first feature peak positions. For example, when the plurality of first feature peak positions include the position of the maximum of the P-wave, the position of the minimum of the Q-wave, the position of the maximum of the R-wave, the position of the minimum of the S-wave, the position of the maximum of the absolute value of the T-wave, and the position of the maximum of the U-wave, the calculating unit 408 may select any two of them in order to calculate the peak distance.

The estimating unit 410 may be configured to estimate a blood glucose value of the user based on at least one peak distance. That is, the estimating unit 410 may estimate the blood glucose value of the user based on a calculation of each of the peak distances. It may specifically be carried out by inputting the calculation of each of the peak distances into an estimating model and then the blood glucose value of the user is estimated by the estimating model. In some embodiments, the estimating unit 410 may input the calculation of each of the peak distances into the machine learning model 500 and then the blood glucose value of the user is estimated by the machine learning model 500. In other embodiments, the estimating unit 410 may input the calculation of each of the peak distances into the neural network model 510 and the blood glucose value of the user is estimated by the neural network model 510.

In some embodiments, the estimating unit 410 may also be configured to estimate the blood glucose value of the user based on at least one peak distance and at least one occurrence rate. In other embodiments, the estimating unit 410 may also be configured to estimate the blood glucose value of the user based on at least one peak distance and at least one amplitude ratio. In other embodiments, the estimating unit 410 may also be configured to estimate the blood glucose value of the user based on at least one peak distance, at least one occurrence rate, and at least one amplitude ratio.

The machine learning model 500 may be configured to estimate the blood glucose value of the user based on at least one peak distance. In some embodiments, machine learning model 500 may be configured to estimate the blood glucose value further based on at least one occurrence rate and/or at least one amplitude ratio. The machine learning model 500 may include or utilize a machine learning algorithm. In some embodiments, the machine learning model 500 may be a supervised learning model, and the machine learning model 500 is trained with the plurality of ECG data such that the machine learning model 500 can estimate the blood glucose value of the user. In some embodiments, each of the plurality of ECG data may include, at least one peak distance of each of the plurality of ECG waveforms and a corresponding blood glucose value, but is not limited thereto. In some embodiments, the machine learning model 500 may be implemented by a decision tree algorithm, a random forest algorithm incorporating bagging, an extreme gradient boosting (XGBoost) algorithm, or a support vector machine algorithm such that the machine learning model 500 can estimate the blood glucose value of the user based on at least one peak distance.

In some embodiments, the machine learning model 500 may also be a semi-supervised learning model, or an unsupervised learning model. For example, the machine learning model 500 may also be implemented by CycleGAN.

In some embodiments, the machine learning model 500 may also be integrated into the computing device 400A. That is, the computing device 400A may store the algorithms associated with the machine learning model 500 and may implement the functions of the machine learning model 500 by executing the algorithms that are stored in the computing device 400A.

The neural network model 510 may be configured to estimate the blood glucose value of the user based on at least one peak distance. In some embodiments, the neural network model 510 may be configured to estimate the blood glucose value of the user further based on at least one occurrence rate and/or at least one amplitude ratio. The neural network model 510 may include an input layer, a hidden layer, and an output layer. The input layer of the neural network model 510 may be configured to receive the plurality of ECG features input from outside of the neural network model 510, wherein the number of nodes of the input layer may depend on the number of the plurality of ECG features that have been extracted. For example, when the plurality of ECG features that have been extracted from each of the plurality of ECG waveforms include P-waves, Q-waves, R-waves, S-waves, T-waves, and U-waves, the number of nodes of the input layer may be, for example, six. The hidden layer of the neural network model 510 may be configured to be intermediate between the input layer and the output layer, and may be configured to perform a series of operating processes on the variables of the nodes of the input layer. In some embodiments, the number of layers of the hidden layer may be configured to be at least three layers so as to increase the computational complexity of the neural network model 510. The number of nodes for each of the layer of the hidden layer may be more than, less than, or equal to the number of nodes for the previous layer. It depends on the complexity of the calculation. For example, when the neural network model 510 is configured to have an input layer, three hidden layers, and an output layer, the number of nodes of the first hidden layer may be more than the number of nodes of the input layer, the number of nodes of the second hidden layer may be less than the number of nodes of the first hidden layer, and the number of nodes of the third hidden layer may be less than the number of nodes of the second hidden layer. More specifically, when the number of nodes of the input layer is configured to be six, the number of nodes of the first hidden layer may be more than six. In some embodiments, the activation function of the hidden layer may be a ReLU function, a Tanh function, or a Sigmoid function, etc., but is not limited thereto. In some embodiments, the hidden layer may be standardized in order to ensure that the values input to the hidden layer are between the sensitive regions of the activation functions. The output layer of the neural network model 510 may be configured to output a final operating result, wherein the final operating result may be obtained by performing a series of operating processes by the hidden layer. That is, after receiving each of the plurality of ECG features, the neural network model 510 performs a series of operating processes by the hidden layer between the input layer and the output layer, such that the output layer of the neural network model 510 can be configured to output the blood glucose value of the user (i.e., the final operating result).

In some embodiments, the neural network model 510 may also be integrated into the computing device 400A. That is, the computing device 400A may store the algorithms associated with the neural network model 510 and may implement the functions of the neural network model 510 by executing the algorithms that are stored in the computing device 400A.

The convolutional neural network model 520 may be configured to extract the plurality of ECG features based on the plurality of ECG waveforms. In some embodiments, the convolutional neural network model 520 may include a convolutional layer and a fully connected layer, wherein the convolutional layer may be configured to extract the plurality of ECG features from the plurality of ECG waveforms and the fully connected layer may be configured to be substantially the same structure and purpose as the neural network model 510. In some embodiments, the number of layers of the convolutional layer may be configured to be at least three. In some embodiments, since the fully connected layer of the convolutional neural network model 520 has substantially the same structure as the neural network model 510, the blood glucose value of the user can also be estimated by the convolutional neural network model 520 (i.e., each of the plurality of ECG features is extracted by the convolutional layer and then the blood glucose value of the user is estimated by the fully connected layer).

In some embodiments, the convolutional neural network model 520 may also be integrated into the computing device 400A. That is, the computing device 400A may store the algorithms associated with the convolutional neural network model 520 and may implement the functions of the convolutional neural network model 520 by executing the algorithms that are stored in the computing device 400A.

In some embodiments, the computing device 400A may also include an ECG waveform database 452 and a blood glucose value database 454. The ECG waveform database 452 may be configured to store the plurality of ECG waveforms of the user. That is, the computing device 400A may first store the plurality of ECG waveforms of the user received from the ECG measuring device 300 into the ECG waveform database 452, and then receive the plurality of ECG waveforms of the user from the ECG waveform database 452 by the receiving unit 402. The blood glucose value database 454 may be configured to store a blood glucose value of the user. That is, the computing device 400A may store the estimated blood glucose value of the user and/or the calibrated blood glucose value of the user into the blood glucose value database 454 after estimating the blood glucose value of the user and/or calculating the calibrated blood glucose value of the user.

Thereby, the computing device 400A provided in the present disclosure can be used to carry out the methods for non-invasively estimating blood glucose as described below, such that the computing device 400A, after receiving the plurality of ECG waveforms of the user, can estimate the blood glucose value of the user and/or calculate the calibrated blood glucose value of the user based on the plurality of ECG waveforms of the user.

By means of the computing device 400A as shown in FIG. 4, it is possible to not only provide a user with a means for non-invasively estimating the blood glucose value of the user, but also to estimate the blood glucose value of the user more accurately. In addition, by means of the computing device 400A as shown in FIG. 4, it is possible to improve the technical field regarding non-invasively estimating the blood glucose value of the user by utilizing a plurality of ECG waveforms.

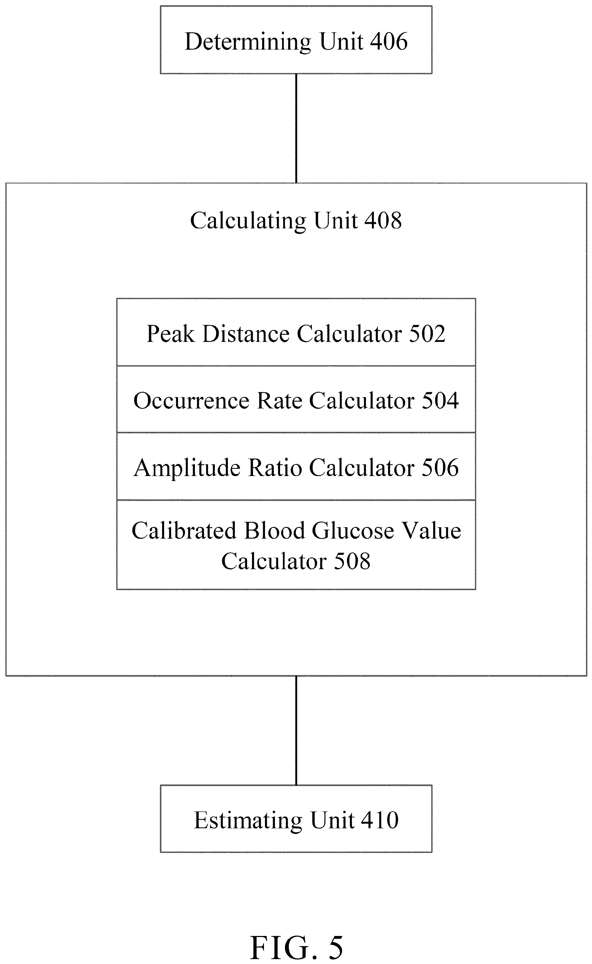

Referring to FIG. 5, FIG. 5 is a schematic diagram illustrating an example of the calculating unit 408 that is signally connected with the determining unit 406 and the estimating unit 410 according to an embodiment of the present disclosure. In some embodiments, the calculating unit 408 may be configured to calculate at least one peak distance between the plurality of feature peak position. In addition, the calculating unit 408 may also be configured to calculate at least one occurrence rate, at least one amplitude ratio, a calibrated blood glucose value, a peak-to-peak slope, and/or at least one sharpness result, but is not limited thereto. Therefore, the calculating unit 408 may include a peak distance calculator 502, and may further include an occurrence rate calculator 504, an amplitude ratio calculator 506, a calibrated blood glucose value calculator 508, a peak-to-peak slope calculator, and/or a sharpness result calculator.

The peak distance calculator 502 may be configured to calculate at least one peak distance between the plurality of feature peak positions. That is, the peak distance calculator 502 may be configured to calculate the peak distance between two of the plurality of feature peak positions, such as the time difference between the position of the maximum of the P-wave and the position of the minimum of the Q-wave (as shown in FIG. 3), but is not limited thereto.

The occurrence rate calculator 504 may be configured to calculate at least one occurrence rate of each of the plurality of ECG features among each of the plurality ECG waveforms. That is, the occurrence rate calculator 504 may be configured to calculate a percentage of the occurrence of a second ECG feature in each of the ECG waveforms, such as the occurrence rate of a P-wave, but is not limited thereto.

The amplitude ratio calculator 506 may be configured to calculate a ratio of amplitude values between each of the plurality of ECG features. That is, the amplitude ratio calculator 506 may be configured to calculate a ratio of peak-to-peak values between each of the plurality of third ECG features, such as an amplitude ratio of the P-wave, but is not limited thereto.

The calibrated blood glucose value calculator 508 may be configured to calculate a calibrated blood glucose value of the user. That is, after estimating the blood glucose value of the user by the estimating unit 410, the calibrated blood glucose value calculator 508 of the calculating unit 408 may receive the estimated blood glucose value of the user from the estimating unit 410 and further perform a calibration operation on the estimated blood glucose value of the user by the equation for calibrating blood glucose value in order to calculate the calibrated blood glucose value of the user.

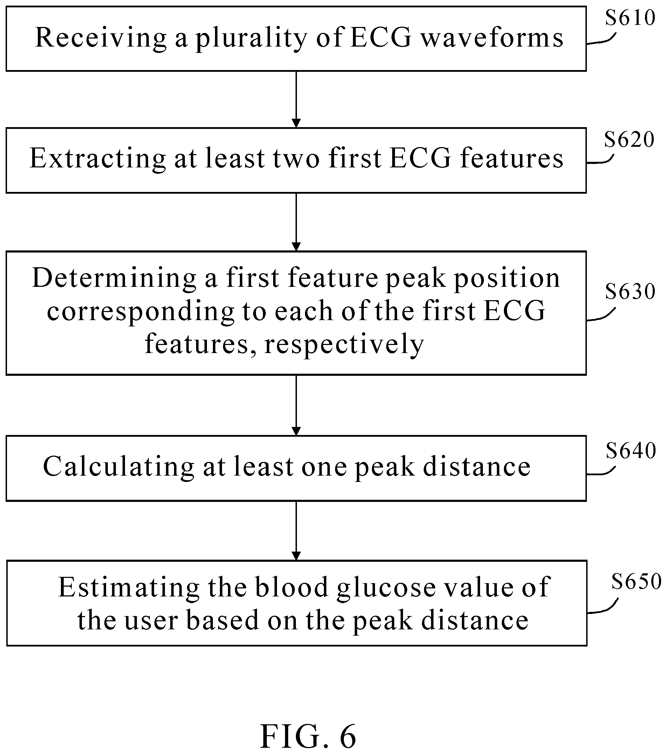

Referring to FIG. 6, FIG. 6 is a flowchart illustrating a first example of the method for non-invasively estimating blood glucose according to an embodiment of the present disclosure. The method as shown in FIG. 6 may include steps S610, S620, S630, S640, and S650.

In step S610, the plurality of ECG waveforms of the user are received. Step S610 may be performed by the receiving unit 402 of the computing device 400A as shown in FIG. 4. In some embodiments, by performing step S610, the plurality of ECG waveforms of the user may be received instantaneously from, for example, the ECG measuring device 300, an ECG sensor (not shown), or a wearable device (not shown), but is not limited thereto. In other embodiments, the plurality of ECG waveforms of the user may be received from the ECG waveform database 452 by performing step S610 to indirectly receive the plurality of ECG waveforms of the user.

In some embodiments, the number of the plurality of ECG waveforms may depend on an ECG receiving duration. That is, step S610 may receive the plurality of ECG waveforms of the user during the ECG receiving duration, wherein the ECG receiving duration may be set to at least one minute in order to ensure that a sufficient number of the plurality of ECG waveforms have been received to estimate the blood glucose value of the user and to generate a more accurate estimation and/or calculation of the blood glucose value.

In step S620, at least two first ECG features are extracted from each of the plurality of ECG waveforms of the user. Step S620 may be performed by the extracting unit 404 of the computing device 400A as shown in FIG. 4. In some embodiments, step S620 may be performed in sequence after step S610 is performed. At least two first ECG features may be extracted from each of the plurality of ECG waveforms by performing step S620, wherein the first ECG features may be selected from a group consisting of a P-wave, a Q-wave, an R-wave, an S-wave, a T-wave, and a U-wave.

In some examples, the P-wave and the Q-wave of each of the plurality of ECG waveforms may be extracted as the first ECG feature by performing step S620, respectively. In another embodiment, the S-wave and the T-wave of each of the plurality of ECG waveforms may be extracted as the first ECG feature by performing step S620, respectively. In another embodiment, the P-wave, the Q-wave, the R-wave, the S-wave, the T-wave, and the U-wave of each of the plurality of ECG waveforms may be extracted as the first ECG feature by performing step S620. That is, at least two of the P-wave, the Q-wave, the R-wave, the S-wave, the T-wave, and the U-wave of each of plurality of ECG waveforms may be extracted as the first ECG feature by performing step S620.

In some embodiments, the first ECG feature may be also extracted by the convolutional neural network model 520. That is, after the plurality of ECG waveforms of the user are input into the convolutional neural network model 520, the first ECG features can be extracted by the convolutional layer of the convolutional neural network model 520.

In step S630, a first feature peak position corresponding to each of the plurality of first ECG features is determined, respectively. Step S630 may be performed by the determining unit 406 of the computing device 400A as shown in FIG. 4. In some embodiments, step S630 may be performed in sequence after step S620 is performed. The first feature peak position corresponding to each of the plurality of first ECG features may be determined by performing step S630, wherein the first feature peak position is substantially the same as the content described in FIG. 3.

In some examples, when the extracted first ECG features are a P-wave and a Q-wave, the position of the maximum of the P-wave and the position of the minimum of the Q-wave in each of the plurality of ECG waveforms may be determined by performing step S630, respectively. In another embodiment, when the extracted first ECG features are an S-wave and a T-wave, the position of the minimum of the S-wave and the position of the maximum of the absolute value of the T-wave in each of the plurality of ECG waveforms may be determined by performing step S630, respectively. In another embodiment, when the extracted first ECG features are a P-wave, a Q-wave, an R-wave, an S-wave, a T-wave, and a U-wave, the position of the maximum of the P-wave, the position of the minimum of the Q-wave, the position of the maximum of the R-wave, the position of the minimum of the S-wave, the position of the maximum of the absolute value of the T-wave, and the position of the maximum of the U-wave in each of the plurality of ECG waveforms may be determined by performing step S630, respectively.

Since the first feature peak position is the position of the maximum or minimum of the first ECG feature, in some embodiments, the method for finding out the first feature peak position may be carried out by continuously comparing the values of two neighboring points; and in another embodiments, the method for finding out the first feature peak position may be carried out by calculating the tangent slopes of the points, respectively.

Compared with the existing technology, the first feature peak position is easier to be determined and utilized. It contributes to estimate the blood glucose value of the user.

In step S640, at least one peak distance between the plurality of first feature peak positions for each of the plurality of ECG waveforms is calculated. Step S640 may be performed by the peak distance calculator 502 of the calculating unit 408 as shown in FIG. 5. In some embodiments, step S640 may be performed in sequence after step S630 is performed. The time difference between any two of the plurality of first feature peak positions is calculated as the peak distance by performing step S640, wherein the peak distance is substantially the same as the content described in FIG. 3.

In some examples, when the extracted first ECG features are a P-wave and a Q-wave, the time difference between the position of the maximum of the P-wave and the position of the minimum of the Q-wave may be calculated as the peak distance by performing step S640. In another embodiment, when the extracted first ECG features are an S-wave and a T-wave, the time difference between the position of the minimum of the S-wave and the position of the maximum of the absolute value of the T-wave may be calculated as the peak distance by performing step S640. In another embodiment, when the extracted first ECG features are a P-wave, a Q-wave, an R-wave, an S-wave, a T-wave, and a U-wave, the time difference between any two of the position of a maximum of the P-wave, the position of a minimum of the Q-wave, the position of a maximum of the R-wave, the position of a minimum of the S-wave, the position of a maximum of the T-wave, and the position of a maximum of the U-wave may be calculated as the peak distance by performing step S640. That is, the peak distances may include the time difference between the P-wave and the Q-wave, the time difference between the P-wave and the R-wave, the time difference between the P-wave and the S-wave, the time difference between the P-wave and the T-wave, the time difference between the P-wave and the U-wave, the time difference between the Q-wave and the R-wave, the time difference between the Q-wave and the S-wave, the time difference between the Q-wave and the T-wave, the time difference between the Q-wave and the U-wave, the time difference between the R-wave and the S-wave, the time difference between the R-wave and the T-wave, the time difference between the R-wave and the U-wave, the time difference between the S-wave and the T-wave, the time difference between the S-wave and the U-wave, and/or the time difference between the T-wave and the U-wave, but are not limited thereto.

In step S650, the blood glucose value of the user is estimated based on the peak distance. Step S650 may be performed by the estimating unit 410 of the computing device 400A as shown in FIG. 4. In some embodiments, step S650 may be performed in sequence after step S640 is performed. The blood glucose value of the user is estimated based on the calculation of the peak distance by performing step S650. More specifically, the blood glucose value of the user is estimated by the estimation model after the calculation of the peak distance is input into the estimation model.

By means of the method for non-invasively estimating blood glucose as shown in FIG. 6, it is possible to not only provide a user with a means for non-invasively estimating the blood glucose value of the user, but also to estimate the blood glucose value of the user more accurately by utilizing the peak distance described above. In addition, by carrying out the method for non-invasively estimating blood glucose as shown in FIG. 6, it is possible to improve the technical field regarding non-invasively estimating the blood glucose value of the user by utilizing a plurality of ECG waveforms.

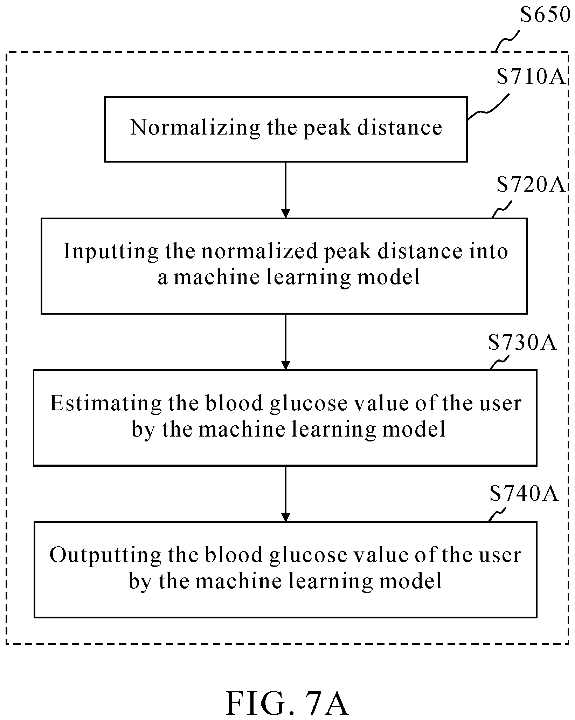

Referring to FIG. 7A, FIG. 7A is a detailed flowchart illustrating a method of estimating the blood glucose value of the user based on the peak distance according to an embodiment of the present disclosure. That is, step S650 as shown in FIG. 6 may include steps S710A, S720A, S730A, and S740A, and it may be accomplished by performing the steps S710A, S720A, S730A, and S740A, wherein the steps S710A, S720A, S730A, and S740A may be performed by the estimating unit 410 of the computing device 400A as shown in FIG. 4.

In step S710A, the peak distance is normalized. In some embodiments, step S710A may be performed in sequence after step S640 is performed. The calculation of the peak distance may be normalized to a corresponding value between a range (e.g., between 0 and 1) by performing step S710A.

In step S720A, the normalized peak distance is input into the machine learning model 500. In some embodiments, step S720A may be performed in sequence after step S710A is performed. The normalized peak distance may be input into the machine learning model 500, such as a decision tree algorithm, a random forest algorithm incorporating bagging, an extreme gradient boosting (XGBoost) algorithm, or a support vector machine algorithm, by performing step S720A.

In step S730A, the blood glucose value of the user is estimated by the machine learning model 500. In some embodiments, step S730A may be performed in sequence after step S720A is performed. The blood glucose value of the user may be estimated based on the normalized peak distance, by performing step S730A, after the normalized peak distance has been input into the machine learning model 500. Since the machine learning model 500 has already been trained with the plurality of data and validated with prediction results, the blood glucose value of the user can be estimated by the machine learning model 500 with a prediction accuracy of 0.80 or more, preferably 0.90 or more.

In step S740A, the blood glucose value of the user is output by the machine learning model 500. In some embodiments, step S740A may be performed in sequence after step S730A is performed. The estimated blood glucose value of the user may be output to a display device and/or a database by the machine learning model 500 by performing step S740A. In some examples, the blood glucose value of the user may be output to the blood glucose value database 454 of the computing device 400A and be synchronously stored in the blood glucose value database 454.

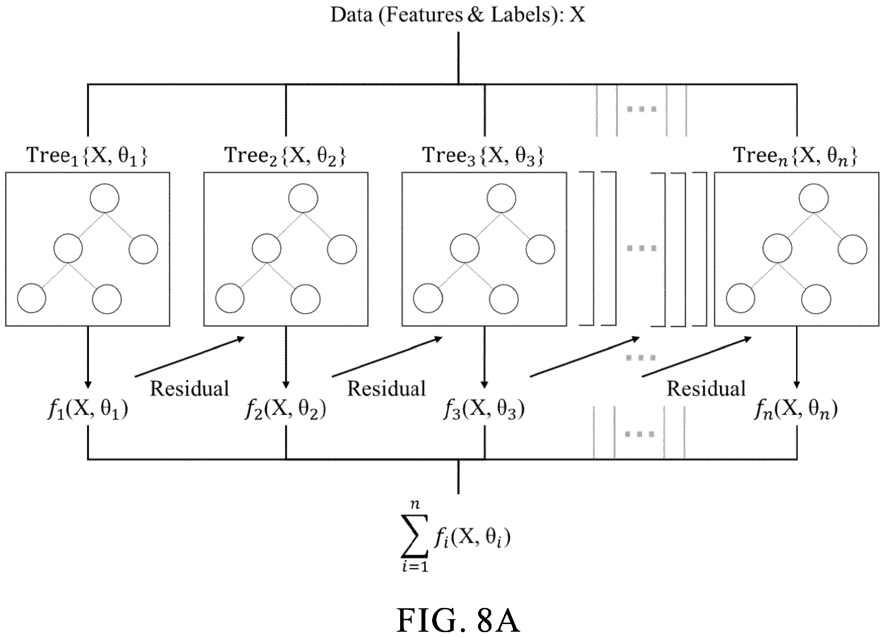

Referring to FIG. 8A, FIG. 8A is a schematic diagram illustrating an example of the machine learning model 500 according to an embodiment of the present disclosure. The machine learning model 500 may be implemented by means of an extreme gradient boosting (XGBoost) algorithm as shown in FIG. 8A. Since the XGBoost algorithm is designed by a plurality of decision trees and the result of the previous decision tree will affect the next decision tree, each of the plurality of decision trees is correlated with each other, so as to make the prediction results of the machine learning model 500 more accurate. In some embodiments, the machine learning model 500 may also be implemented by other algorithms such as a decision tree algorithm, a random forest algorithm incorporating bagging, or a support vector machine algorithm.

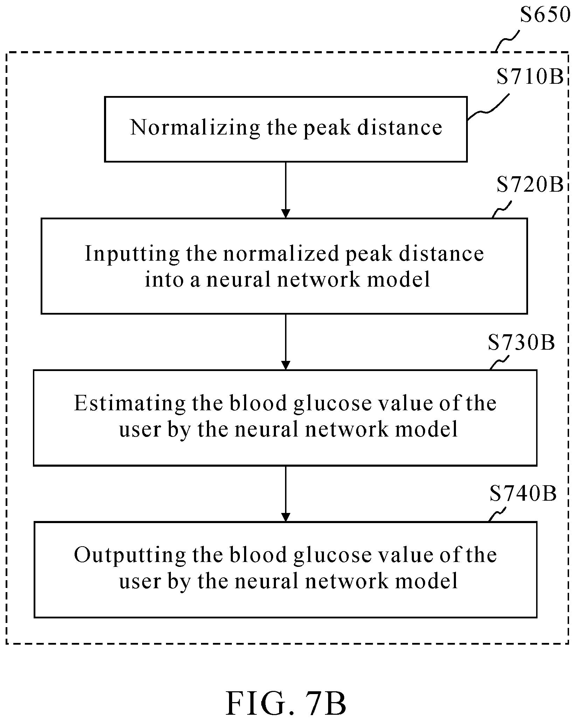

Referring to FIG. 7B, FIG. 7B is a detailed flowchart illustrating a method of estimating the blood glucose value of the user based on the peak distance according to an embodiment of the present disclosure. That is, step S650 as shown in FIG. 6 may include steps S710B, S720B, S730B, and S740B, and it may be accomplished by performing the steps S710B, S720B, S730B, and S740B, wherein the steps S710B, S720B, S730B, and S740B may be performed by the estimating unit 410 of the computing device 400A as shown in FIG. 4.

In step S710B, the peak distance is normalized. In some embodiments, step S710B may be performed in sequence after step S640 is performed. In some embodiments, step S710B may be substantially the same as step S710A.

In step S720A, the normalized peak distance is input into the neural network model 510. In some embodiments, step S720B may be performed in sequence after step S710B is performed. The normalized peak distance may be input into the neural network model 510 by performing step S720B.

In step S730B, the blood glucose value of the user is estimated by the neural network model 510. In some embodiments, step S730B may be performed in sequence after step S720B is performed. The blood glucose value of the user may be estimated based on the normalized peak distance, by performing step S730B, after the normalized peak distance has been input into the neural network model 510. Since the neural network model 510 has already been trained with the plurality of data and validated with prediction results, the blood glucose value of the user can be estimated by the neural network model 510 with a prediction accuracy of 0.80 or more, preferably 0.90 or more.

In step S740B, the blood glucose value of the user is output by the neural network model 510. In some embodiments, step S740B may be performed in sequence after step S730B is performed. The estimated blood glucose value of the user may be output to a display device and/or a database by the neural network model 510 by performing step S740B. In some examples, the estimated blood glucose value of the user may be output to the blood glucose value database 454 of the computing device 400A and be synchronously stored in the blood glucose value database 454.

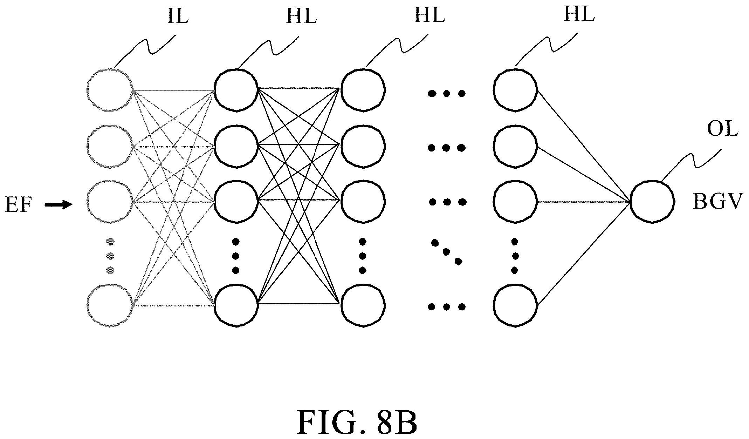

Referring to FIG. 8B, FIG. 8B is a schematic diagram illustrating an example of the neural network model 510 according to an embodiment of the present disclosure. The neural network model 510 may include an input layer IL, at least three layers of a hidden layer HL, and an output layer OL. In some embodiments, the neural network model 510, after receiving the peak distance, may perform a series of operating processes by each node of the hidden layer HL in order to estimate the blood glucose value of the user and output the estimated blood glucose value of the user by the output layer OL. In some embodiments, the neural network model 510 may also directly receive the information of each of the plurality of ECG features and then perform a series of operating processes on the information of each of the plurality of ECG features by the hidden layer HL in order to estimate the blood glucose value of the user.

In some embodiments, each of the plurality of ECG features in each of the plurality of ECG waveforms may be extracted by the convolutional neural network model 520. Furthermore, since the fully connected layer in the convolutional neural network model 520 may have substantially the same architecture as the neural network model 510, the convolutional neural network model 520, after extracting the plurality of ECG features in each of the plurality of ECG waveforms, may directly estimate the blood glucose value of the user based on each of the plurality of ECG features, in particular based on the peak distance between the plurality of ECG features.

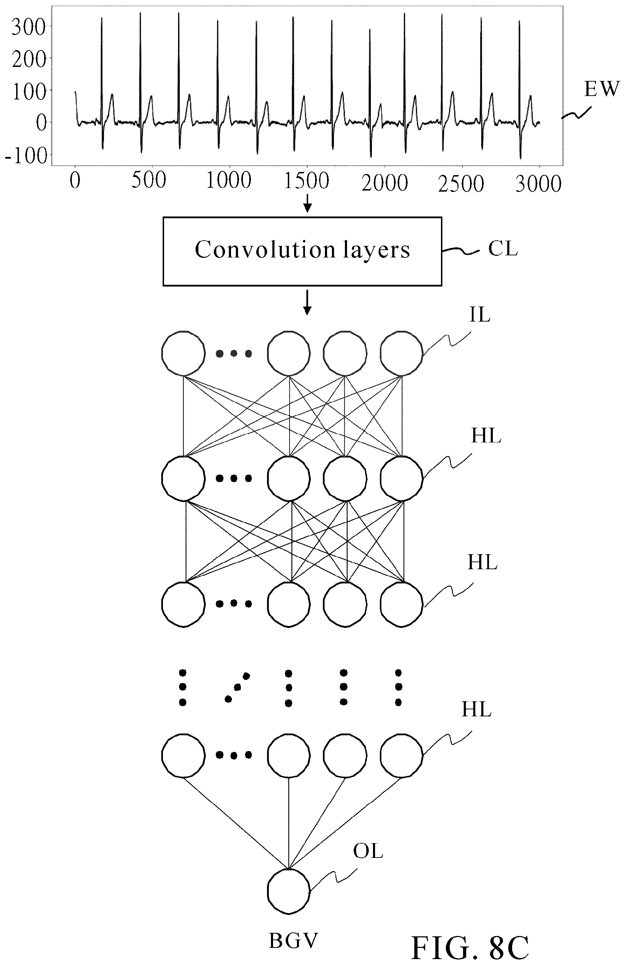

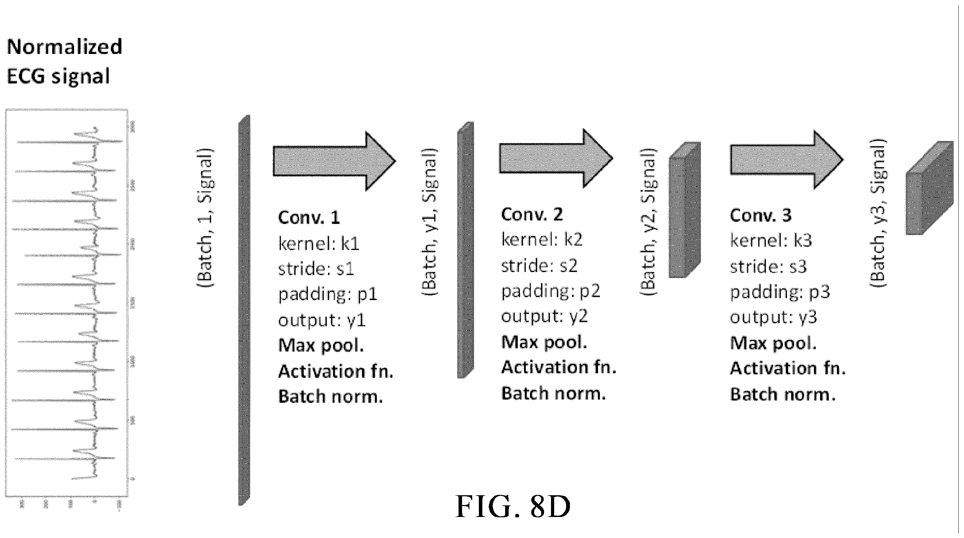

Referring to FIG. 8C, FIG. 8C is a schematic diagram illustrating an example of the convolution neural network model 520 according to an embodiment of the present disclosure. The convolutional neural network model 520 can extract each of the plurality of ECG features by the convolutional layer CL after receiving the plurality of ECG waveforms EW of the user. Referring also to FIG. 8D, FIG. 8D is a schematic diagram illustrating an example of the convolution layer CL according to an embodiment of the present disclosure. As shown in FIG. 8D, the convolutional neural network model 520 may include at least three (e.g., three) layers of the convolutional layer CL.

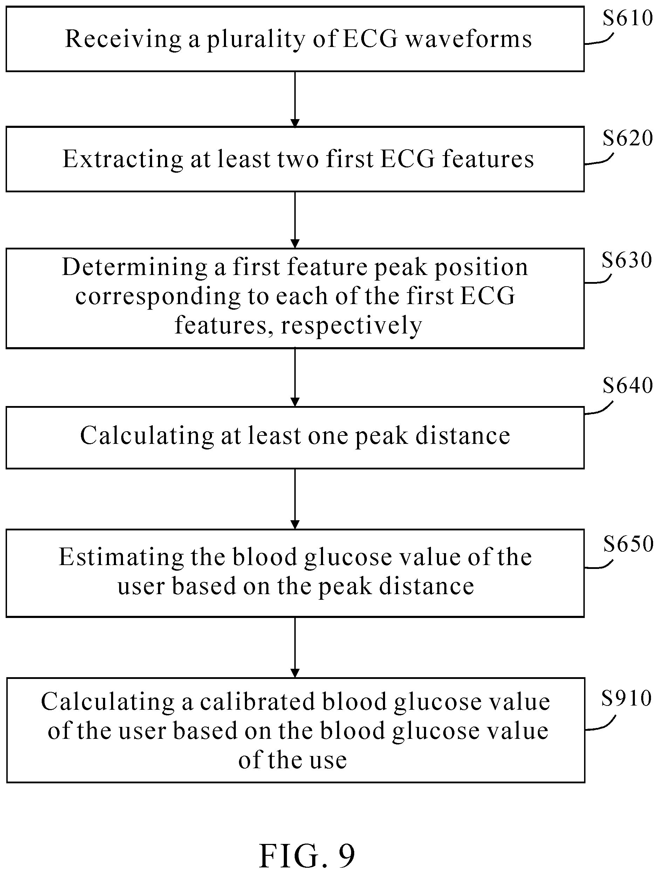

Referring to FIG. 9, FIG. 9 is a flowchart illustrating a second example of the method for non-invasively estimating blood glucose according to an embodiment of the present disclosure. The method as shown in FIG. 9 may include steps S610, S620, S630, S640, S650, and S910, wherein the steps S610, S620, S630, S640, and S650 are substantially the same as those shown in FIG. 6.

In step S910, the calibrated blood glucose value of the user is calculated based on the blood glucose value of the user. Step S910 may be performed by the calibrated blood glucose value calculator 508 of the calculating unit 408 as shown in FIG. 5. In some embodiments, step S910 may be performed in sequence after step S650 is performed. The calibrated blood glucose value of the user is calculated by using the equation of calibrated blood glucose value based on the blood glucose value of the user that is estimated by the estimating unit 410, by performing step S910.

In some embodiments, the equation of calibrated blood glucose value may be a unary cubic equation, such as "$a \times x^3 + b \times x^2 + c \times x + d = y$", but is not limited thereto. Each parameter (a, b, c, and d) in the equation of calibrated blood glucose value may be determined based on the estimated blood glucose value of the user (variable x) and the actual blood glucose value of the user (variable y) that is measured by a blood glucose meter.

In an example, the values of each parameter (a, b, c, and d) can be known by solving the simultaneous equations when the estimated blood glucose values of the user are 90, 135, and 180, respectively, in sequence, and the actual blood glucose values of the user measured by the blood glucose meter are 85, 120, and 190, respectively, in sequence.

$$\begin{cases} 90^3 \cdot a + 90^2 \cdot b + 90 \cdot c = 85 \\ 135^3 \cdot a + 135^2 \cdot b + 135 \cdot c = 120 \\ 180^3 \cdot a + 180^2 \cdot b + 180 \cdot c = 190 \end{cases}, a = \frac{1}{18225}, b = -\frac{11}{810}, c = \frac{31}{18}, d = 0$$

Therefore, the calibrated blood glucose value calculator 508 may perform a calculating operation on the estimated blood glucose of the user by the equation of calibrated blood glucose value "

$$\frac{1}{18225} \times x^3 - \frac{11}{810} \times x^2 + \frac{31}{18} \times x + 0 = y$$ " so as to calibrate the estimated blood glucose value of the user.

In some embodiments, the equation of calibrated blood glucose value may be a unary quadratic equation, such as " $a \times x^2 + b \times x + c = y$ ", but is not limited thereto. Each parameter (a, b, and c) in the equation may be determined based on the estimated blood glucose value of the user (variable x) and the actual blood glucose value of the user (variable y) that is measured by a blood glucose meter.

$$\begin{cases} 90^2 \cdot a + 90 \cdot b = 85 \\ 180^2 \cdot a + 180 \cdot b = 190 \end{cases}, a = \frac{1}{810}, b = \frac{5}{6}, c = 0$$

Therefore, the calibrated blood glucose value calculator 508 may perform a calculating operation on the estimated blood glucose of the user by the equation of calibrated blood glucose value "

$$\frac{1}{810} \times x^2 + \frac{5}{6} \times x + 0 = y$$ " 810 6 so as to calibrate the estimated blood glucose value of the user.

By means of the method for non-invasively estimating blood glucose as shown in FIG. 9, it is possible to not only provide a user with a means for non-invasively estimating the blood glucose value of the user, but also to calculate, by utilizing the equation of calibrated blood glucose value, the blood glucose value that is the same as or close to the result of the measurement by the blood glucose meter (i.e., the calibrated blood glucose value of the user). Thereby, the method as shown in FIG. 9 can compensate for the difference between the estimated result of the blood glucose value and the result of the measurement actually measured by the blood glucose meter in order to provide the user with a blood glucose value that is substantially the same as or close to the result of the measurement that is actually measured by the blood glucose meter.

In some embodiments, step S910 may be omitted when the blood glucose value that is estimated by the estimating unit 410 is accurate enough (e.g., the difference range being within 0.05 when comparing the estimated blood glucose value with the result of the measurement actually measured by the blood glucose meter). That is, whether or not to perform step S910 may depend on the user's need for precision in the estimating result of the blood glucose value. When the user desires a more accurate result of the blood glucose value, a more accurate result of the blood glucose value (i.e., the calibrated blood glucose value of the user) can be provided by the means of the method as shown in FIG. 9.

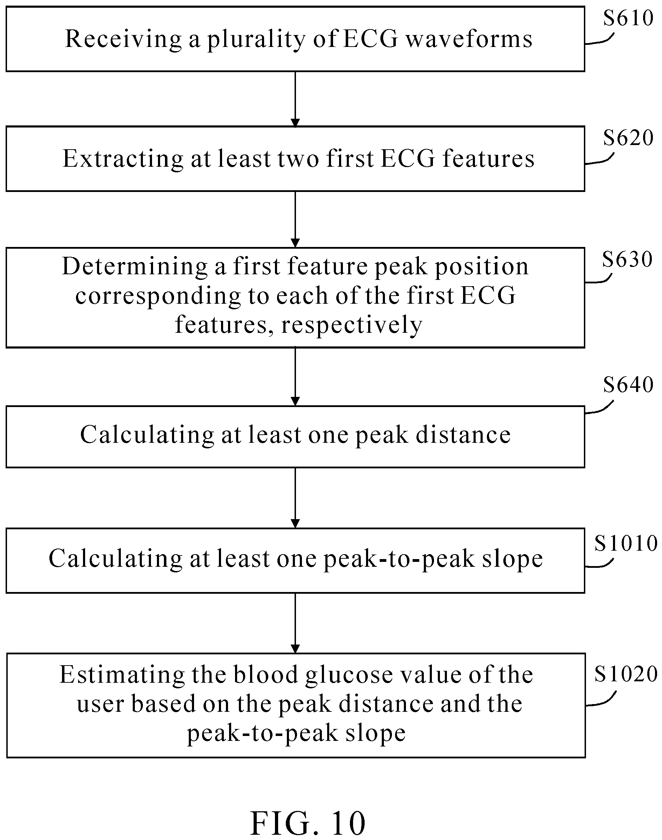

Referring to FIG. 10, FIG. 10 is a flowchart illustrating a third example of the method for non-invasively estimating blood glucose according to an embodiment of the present disclosure. The method as shown in FIG. 10 may include steps S610, S620, S630, S640, S1010, and S1020, wherein the steps S610, S620, S630, and S640 are substantially the same as those shown in FIG. 6.

In step S1010, at least one peak-to-peak slope between the plurality of the first feature peak positions for each of the plurality of ECG waveforms is calculated. Step S1010 may be performed by the calculating unit 408 of the computing device 400A as shown in FIG. 4. In some embodiments, the step S910 may be performed in sequence after step S630 is performed. In addition, step S1010 may be performed at the same time as step S640.

In some examples, when the extracted first ECG features are a P-wave and a Q-wave, the slope between the position of the maximum of the P-wave and the position of the minimum of the Q-wave may be calculated as the peak-to-peak slope by performing step S1010. In another embodiment, when the extracted first ECG features are an S-wave and a T-wave, the slope between the position of the minimum of the S-wave and the position of the maximum of the absolute value of the T-wave may be calculated as the peak-to-peak slope by performing step S640. In another embodiment, when the extracted first ECG features are a P-wave, a Q-wave, an R-wave, an S-wave, a T-wave, and a U-wave, the slope between any two of the position of a maximum of the P-wave, the position of a minimum of the Q-wave, the position of a maximum of the R-wave, the position of a minimum of the S-wave, the position of a maximum of the T-wave, and the position of a maximum of the U-wave may be calculated as the peak-to-peak slope by performing step S640. That is, the peak-to-peak slope may include the slope between the P-wave and the Q-wave, the slope between the P-wave and the R-wave, the slope between the P-wave and the S-wave, the slope between the P-wave and the T-wave, the slope between the P-wave and the U-wave, the slope between the Q-wave and the R-wave, the slope between the Q-wave and the S-wave, the slope between the Q-wave and the T-wave, the slope between the Q-wave and the U-wave, the slope between the R-wave and the S-wave, the slope between the R-wave and the T-wave, the slope between the R-wave and the U-wave, the slope between the S-wave and the T-wave, the slope between the S-wave and the U-wave, and/or the slope between the T-wave and the U-wave, but are not limited thereto.

In step S1020, the blood glucose value of the user is estimated based on the peak distance and the peak-to-peak slope. Step S1020 may be performed by the estimating unit 410 of the computing device 400A as shown in FIG. 4. In some embodiments, step S1130 may be performed in sequence after step S640 and step S1010 are performed. The blood glucose value of the user may be estimated based on the calculation of the peak distance and the calculation of the peak-to-peak slope by performing step S 1020. More specifically, the blood glucose value of the user is estimated by the estimation model after the calculation of the peak distance and the calculation of the peak-to-peak slope are input into the estimation model.

By means of the method for non-invasively estimating blood glucose as shown in FIG. 10, it is possible to not only provide a user with a means for non-invasively estimating the blood glucose value of the user, but also to estimate the blood glucose value of the user more accurately by utilizing the peak distance and the peak-to-peak slope that are described above. In addition, by carrying out the method for non-invasively estimating blood glucose as shown in FIG. 10, it is possible to improve the technical field regarding non-invasively estimating the blood glucose value of the user by utilizing a plurality of ECG waveforms.

Furthermore, in some embodiments, the method for non-invasively estimating blood glucose as shown in FIG. 10 may further include step S910 as shown in FIG. 9 and then it may calculate a calibrated blood glucose value of the user by performing step S910.

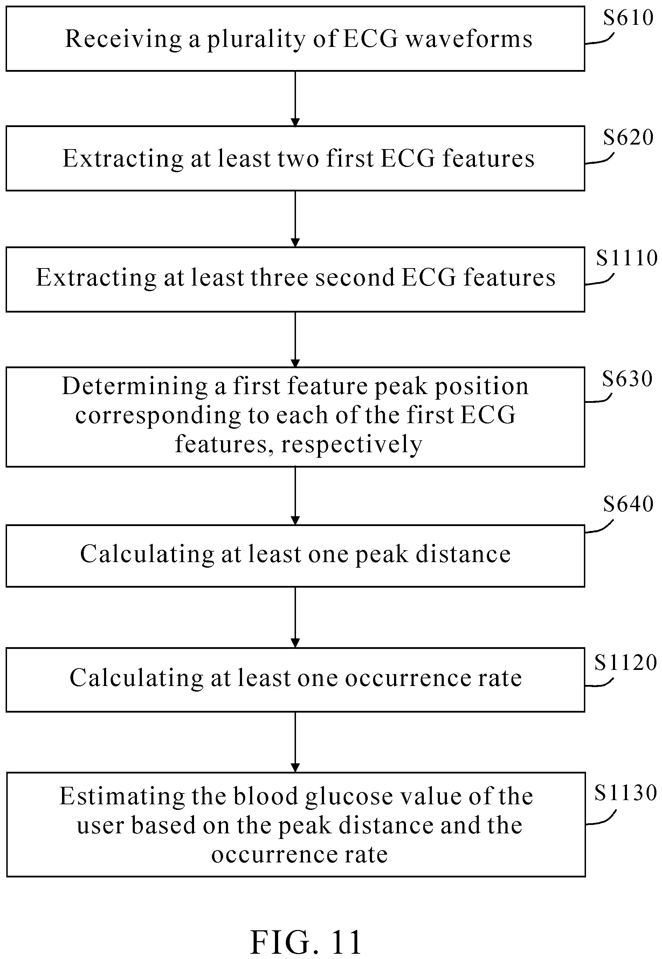

Referring to FIG. 11, FIG. 11 is a flowchart illustrating a fourth example of the method for non-invasively estimating blood glucose according to an embodiment of the present disclosure. The method as shown in FIG. 11 may include steps S610, S620, S630, S640, S1110, 51120, and 51130, wherein the steps S610, S620, S630, and S640 are substantially the same as those shown in FIG. 6.

In step S1110, at least three second ECG features are extracted from each of the plurality of ECG waveforms of the user. Step S1110 may be performed by the extracting unit 404 of the computing device 400A as shown in FIG. 4. In some embodiments, step S1110 may be performed in sequence after step S610 is performed. In addition, step S1110 may be performed at the same time as step S620. The at least three second ECG features may be extracted from each of the plurality of ECG waveforms by performing step S1110, wherein the second ECG features may be selected from a group consisting of a P-wave, a Q-wave, an R-wave, an S-wave, a T-wave, and a U-wave.

In some examples, the P-wave, the Q-wave, and the R-wave of each of the plurality of ECG waveforms may be extracted as the second ECG feature by performing step S1110, respectively. In another embodiment, the S-wave, the T-wave, and the U-wave of each of the ECG waveforms may be extracted as the second ECG feature by performing step S1110, respectively. In another embodiment, the P-wave, the Q-wave, the R-wave, the S-wave, the T-wave, and the U-wave of each of the plurality of ECG waveforms may be extracted as the second ECG feature by performing step S1 110, respectively. That is, at least three of the P-wave, the Q-wave, the R-wave, the S-wave, the T-wave, and the U-wave of each of the plurality of ECG waveforms may be extracted as the second ECG feature by performing step S1110.

In some embodiments, the second ECG feature may be also extracted by the convolutional neural network model 520. That is, after the plurality of ECG waveforms of the user are input into the convolutional neural network model 520, the second ECG features can be extracted by the convolutional layer of the convolutional neural network model 520.

In step S1120, at least one occurrence rate is calculated based on the second ECG feature. Step S1120 may be performed by the occurrence rate calculator 504 of the calculating unit 408 as shown in FIG. 5. In some embodiments, step 51120 may be performed in sequence after step S1110 is performed. At least one occurrence rate, e.g., the occurrence rate of a P-wave but not limited thereto, may be calculated based on the plurality of second ECG features by performing step S1120. Step S1120 may be a step for calculating the at least one occurrence rate based on an amplitude value (e.g., a maximum value, a minimum value, a root-mean-square value, or a peak-to-peak value, but not limited thereto) of the plurality of second ECG features. More specifically, the at least one occurrence rate may be calculated based on a comparison of the amplitude value of the plurality of second ECG features with a default value to determine whether each of the plurality of second ECG features occurs in each of the plurality of ECG waveforms, respectively. In addition, the amplitude values of the plurality of second ECG features may be compared with each other to determine whether each of the plurality of second ECG features occurs in each of the plurality of ECG waveforms, and then to calculate the at least one occurrence rate based on the result of the determination.

In some examples, when the extracted second ECG features are a P-wave, a Q-wave, and an R-wave, the occurrence rate of the P-wave, the occurrence rate of the Q-wave, and/or the occurrence rate of the R-wave may be calculated by performing step S1120. In another embodiment, when the extracted second ECG features are an S-wave, a T-wave, and a U-wave, the occurrence rate of the S-wave, the occurrence rate of the T-wave, and/or the occurrence rate of the U-wave may be calculated by performing step S1120. In another embodiment, when the extracted second ECG features are a P-wave, a Q-wave, an R-wave, an S-wave, a T-wave, and a U-wave, the occurrence rate of the P-wave, the occurrence rate of the Q-wave, the occurrence rate of the R-wave, the occurrence rate of the S-wave, the occurrence rate of the T-wave, and/or the occurrence rate of the U-wave may be computed by performing step S1120.

In step S1130, the blood glucose value of the user is estimated based on the peak distance and the occurrence rate. Step S1130 may be performed by the estimating unit 410 of the computing device 400A as shown in FIG. 4. In some embodiments, step S1130 may be performed in sequence after step S640 and step S1120 are performed. The blood glucose value of the user may be estimated based on the calculation of the peak distance and the calculation of the occurrence rate by performing step S1130. More specifically, the blood glucose value of the user is estimated by the estimation model after the calculation of the peak distance and the calculation of the occurrence rate are input into the estimation model.

By means of the method for non-invasively estimating blood glucose as shown in FIG. 11, it is possible to not only provide a user with a means for non-invasively estimating the blood glucose value of the user, but also to estimate the blood glucose value of the user more accurately by utilizing the peak distance and the occurrence rate that are described above. In addition, by carrying out the method for non-invasively estimating blood glucose as shown in FIG. 11, it is possible to improve the technical field regarding non-invasively estimating the blood glucose value of the user by utilizing a plurality of ECG waveforms.

Furthermore, in some embodiments, the method for non-invasively estimating blood glucose as shown in FIG. 11 may further include step S910 as shown in FIG. 9 and then it may calculate a calibrated blood glucose value of the user by performing step S910.

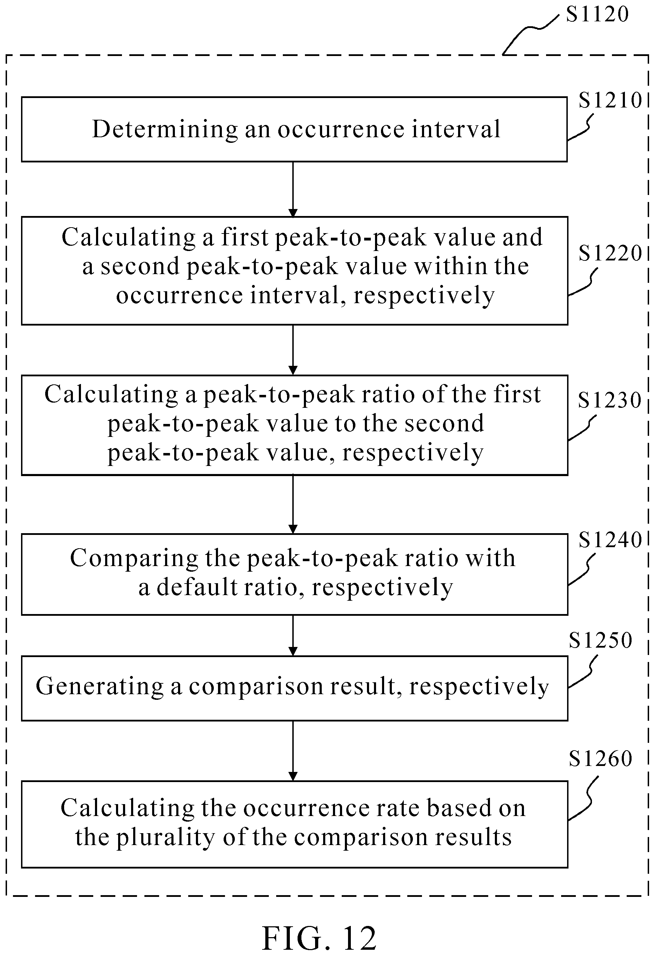

Referring to FIG. 12, FIG. 12 is a detailed flowchart illustrating a method of calculating at least one occurrence rate based on the second ECG features according to an embodiment of the present disclosure. That is, step S1120 as shown in FIG. 11 may include steps S1210, S1220, S1230, S1240, S1250, and S1260, and it may be accomplished by performing the steps S1210, S1220, S1230, S1240, S1250, and S1260, wherein the steps S1210, S1220, S1230, S1240, S1250, and S1260 may be performed by the occurrence rate calculator 504 of the calculating unit 408 as shown in FIG. 5.

In step S1210, an occurrence interval is determined. In some embodiments, step S1210 may be performed in sequence after step S1110 is performed. The occurrence interval may be determined based on one of the second ECG features by performing step S1210.

Step S1210 may be a step for determining the occurrence interval by using one of the second ECG features as a reference point forwards or backwards a certain occurrence spacing distance. In some embodiments, the occurrence spacing distance may depend on the distance between two adjacent ECG waveforms, i.e., the occurrence spacing distance may depend on the RR interval.

In some embodiments, the occurrence interval may be determined by taking the position of the maximum of the R-wave as the reference point and then being forward 0.33 times the RR interval, by performing step S1210. In another embodiment, the occurrence interval may be determined by taking the position of the maximum of the R-wave as the reference point and then being backward 0.67 times the RR interval, by performing step S1210. In another embodiment, the occurrence interval may be determined by taking the position of the maximum of the absolute value of the T-wave as the reference point and then being backward a certain occurrence spacing distance (i.e., until a new P-wave of the ECG waveform is encountered).

In step S1220, a first peak-to-peak value and a second peak-to-peak value are calculated within the occurrence interval, respectively. In some embodiments, step S1220 may be performed in sequence after step S1210 is performed. The first peak-to-peak value and the second peak-to-peak value within the occurrence interval may be calculated for each of the plurality of ECG waveforms by performing step S1220.

In some examples, when the extracted second ECG features are a P-wave, a Q-wave, and an R-wave, the peak-to-peak value between the maximum of the P-wave and the minimum of the Q-wave as the first peak-to-peak value and the peak-to-peak value between the maximum of the R-wave and the minimum of the Q-wave as the second peak-to-peak value, within the occurrence interval (i.e., 0.33 times the RR interval forward from the position of the maximum of the R-wave), may be calculated for each of the plurality of ECG waveforms by performing step S 1220, respectively. In another embodiment, when the extracted second ECG features are an R-wave, an S-wave, and a T-wave, the peak-to-peak value between the maximum of the absolute value of the T-wave and the minimum of the S-wave as the first peak-to-peak value and the peak-to-peak value between the maximum of the R-wave and the minimum of the S-wave as the second peak-to-peak value, within the occurrence interval (i.e., 0.67 times the RR interval backward from the position of the maximum of the R-wave), may be calculated for each of the plurality of ECG waveforms by performing step S 1220, respectively. In another embodiment, when the extracted second ECG features are an S-wave, a T-wave, and a U-wave, the peak-to-peak value between the maximum of the U-wave and the minimum of the S-wave as the first peak-to-peak value and the peak-to-peak value between the maximum of the absolute value of the T-wave and the minimum of the S-wave as the second peak-to-peak value, within the occurrence interval (i.e., a certain occurrence spacing distance, until a new P-wave of the ECG waveform is encountered, backward from the position of the maximum of the absolute value of the T-wave), may be calculated for each of the plurality of ECG waveforms by performing step S 1220, respectively.

In step S1230, a ratio of the first peak-to-peak to the second peak-to-peak is calculated, respectively. In some embodiments, step S1230 may be performed in sequence after step S1220 is performed. The ratio of the first peak-to-peak to the second peak-to-peak may be calculated as a peak-to-peak ratio for each of the plurality of ECG waveforms.

In some examples, when the extracted second ECG features are a P-wave, a Q-wave, and an R-wave, the ratio of the peak-to-peak value between the P-wave and the Q-wave to the peak-to-peak value between the R-wave and the Q-wave may be calculated as the peak-to-peak ratio (which may be referred to as a first peak-to-peak ratio) by performing step S1230, respectively. In another embodiment, when the extracted second ECG features are an R-wave, an S-wave, and a T-wave, the ratio of the peak-to-peak value between the T-wave and the S-wave to the peak-to-peak value between the R-wave and the S-wave may be calculated as the peak-to-peak ratio (which may be referred to as a second peak-to-peak ratio) by performing step S1230, respectively. In another embodiment, when the extracted second ECG features are an S-wave, a T-wave, and a U-wave, the ratio of the peak-to-peak value between the U-wave and the S-wave to the peak-to-peak value between the T-wave and the S-wave may be calculated as the peak-to-peak ratio (which may be referred to as a third peak-to-peak ratio) by performing step S1230, respectively.

In step S1240, the peak-to-peak ratio is compared with a default ratio, respectively. In some embodiments, step S1240 may be performed in sequence after step S1230 is performed. The peak-to-peak ratio may be compared with the default ratio (e.g., be compared by the ratio value of the two) for each of the plurality of ECG waveforms, respectively.

In some examples, when the extracted second ECG features are a P-wave, a Q-wave, and an R-wave, the first peak-to-peak ratio of each of the plurality of ECG waveforms may be compared with the default ratio by performing step S1240, respectively, wherein the default ratio may be set to a ratio value between 0.05 to 0.15, and specifically 0.1, so as to know whether the peak-to-peak value between the P-wave and the Q-waves is greater than the default ratio times the peak-to-peak value between the R-wave and the Q-waves. In another embodiment, when the extracted second ECG features are an R-wave, an S-wave, and a T-wave, the second peak-to-peak ratio of each of the plurality of ECG waveforms may be compared with the default ratio by performing step S1240, respectively, wherein the default ratio may be set to a ratio value between 0.2 to 0.4, and specifically 0.25, so as to know whether the peak-to-peak value between the T-wave and the S-wave is greater than the default ratio times the peak-to-peak value between the R-wave and the S-waves. In another embodiment, when the extracted second ECG features are an S-wave, a T-wave, and a U-wave, the third peak-to-peak ratio of each of the plurality of ECG waveforms may be compared with the default ratio by performing step S1240, respectively, wherein the default ratio may be set to a ratio value between 0.12 to 0.18, and specifically 0.15, so as to know whether the peak-to-peak value between the U-wave and the S-wave is greater than the default ratio times the peak-to-peak value between the T-wave and the S-wave.

In step S1250, a comparison result is generated, respectively. In some embodiments, step S1250 may be performed in sequence after step S1240 is performed.

In some examples, when the extracted second ECG features are a P-wave, a Q-wave, and an R-wave, the comparison result of each of the plurality of ECG waveforms may be generated by performing step S1250, wherein the comparison result may be a result that the first peak-to-peak ratio is greater than the default ratio or a result that the first peak-to-peak ratio is less than or equal to the default ratio. In another embodiment, when the extracted second ECG features are an R-wave, an S-wave, and a T-wave, the comparison result of each of the plurality of ECG waveforms may be generated by performing step S1250, wherein the comparison result may be a result that the second peak-to-peak ratio is greater than the default ratio or a result that the second peak-to-peak ratio is less than or equal to the default ratio. In another embodiment, when the extracted second ECG features are an S-wave, a T-wave, and a U-wave, the comparison result of each of the plurality of ECG waveforms may be generated by performing step S1250, wherein the comparison result may be a result that the third peak-to-peak ratio is greater than the default ratio or a result that the third peak-to-peak ratio is less than or equal to the default ratio.

In step S1260, the occurrence rate is calculated based on the plurality of comparison results. In some embodiments, step S1260 may be performed in sequence after step S1250 is performed. The occurrence rate may be calculated based on the comparison result of each of the plurality of ECG waveforms by performing step S1260.

In some examples, when the extracted second ECG features are a P-wave, a Q-wave, and an R-wave, the occurrence rate of the P-wave (i.e., the percentage of results that the first peak-to-peak ratio is greater than the default ratio) may be calculated based on the result that the first peak-to-peak ratio is greater than the default ratio and the result that the first peak-to-peak ratio is less than or equal to the preset ratio, by performing step S 1260. In another embodiment, when the extracted second ECG features are an R-wave, an S-wave, and a T-wave, the occurrence rate of the T-wave (i.e., the percentage of results that the second peak-to-peak ratio is greater than the default ratio) may be calculated based on the result that the second peak-to-peak ratio is greater than the default ratio and the result that the second peak-to-peak ratio is less than or equal to the preset ratio, by performing step S1260. In another embodiment, when the extracted second ECG features are an S-wave, a T-wave, and a U-wave, the occurrence rate of the U-wave (i.e., the percentage of results that the third peak-to-peak ratio is greater than the default ratio) may be calculated based on the result that the third peak-to-peak ratio is greater than the default ratio and the result that the third peak-to-peak ratio is less than or equal to the preset ratio, by performing step S1260.

By means of performing the steps as shown in FIG. 12, the plurality of comparison results may be generated by comparing the peak-to-peak ratio with the default ratio in order to more accurately calculate the occurrence rate, wherein the occurrence rate may include the occurrence rate of the P-wave, the occurrence rate of the Q-wave, the occurrence rate of the R-wave, the occurrence rate of the S-wave, the occurrence rate of the T-wave, and/or the occurrence rate of the U-wave, but is not limited thereto.

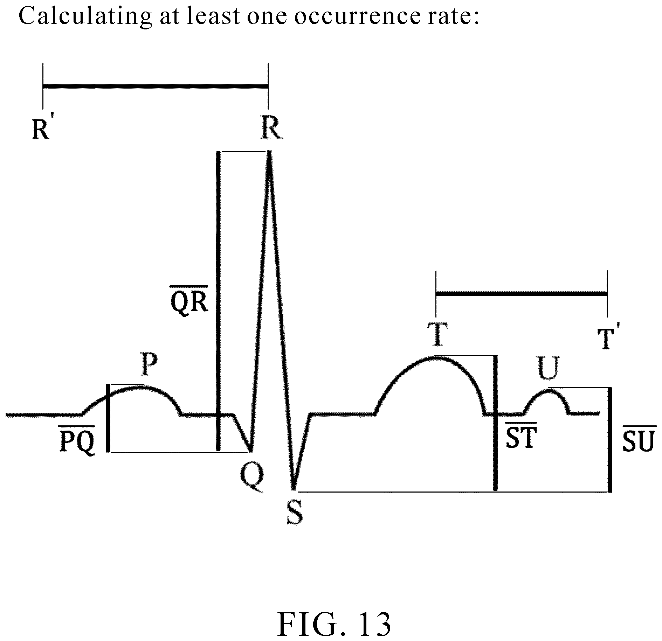

Referring to FIG. 13, FIG. 13 is a schematic diagram illustrating calculating at least one occurrence rate based on the second ECG features according to an embodiment of the present disclosure. As shown in FIG. 13, taking an ECG waveform as an example, when the extracted second ECG features are a P-wave, a Q-wave, an R-wave, an S-wave, a T-wave, and a U-wave, by performing each of the steps as shown in FIG. 12, the peak-to-peak value between the plurality of second ECG features and the peak-to-peak ratio between the plurality of the peak-to-peak values may be calculated, respectively, and the plurality of peak-to-peak ratios may be compared with the default ratio in order to calculate the occurrence rate, wherein the occurrence rate may be the occurrence rate of the P-wave, the occurrence rate of the Q-wave, the occurrence rate of the R-wave, the occurrence rate of the S-wave, the occurrence rate of the T-wave, and/or the occurrence rate of the U-wave, but is not limited thereto.

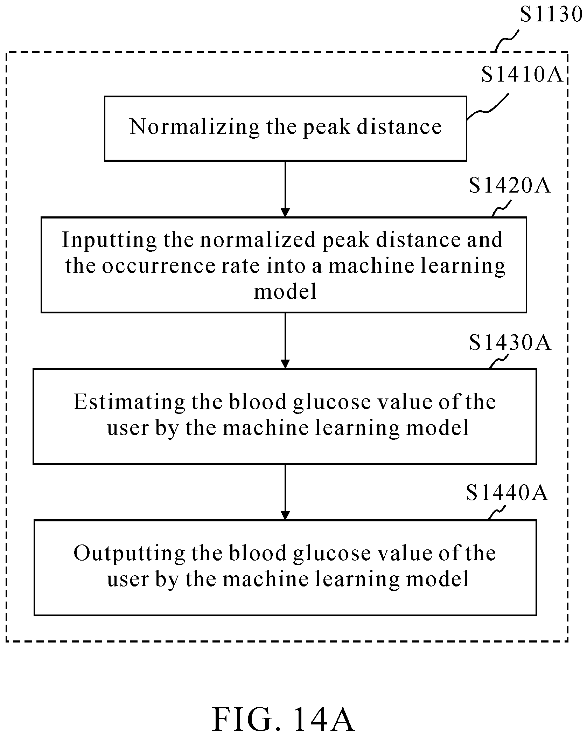

Referring to FIG. 14A, FIG. 14A is a detailed flowchart illustrating a method of estimating the blood glucose value of the user based on the peak distance and the occurrence rate according to an embodiment of the present disclosure. That is, step S1130 as shown in FIG. 11 may include steps S1410A, S1420A, S1430A, and S1440A, and it may be accomplished by performing the steps S1410A, S1420A, S1430A, and S1440A, wherein the steps S1410A, S1420A, S1430A, and S1440A may be performed by the estimating unit 410 of the computing device 400A as shown in FIG. 4.

In step S1410A, the peak distance is normalized. In some embodiments, step S1410A may be performed in sequence after step S640 and step S1120 are performed. In some embodiments, step S1410A may be substantially the same as step S710A as shown in FIG. 7A.

In step S1420A, the normalized peak distance and the occurrence rate are input into the machine learning model 500. In some embodiments, step S1420A may be performed in sequence after step S1410A is performed. The normalized peak distance and the occurrence rate may be input into the machine learning model 500, such as a decision tree algorithm, a random forest algorithm incorporating bagging, an extreme gradient boosting (XGBoost) algorithm, or a support vector machine algorithm, by performing step S1420A.

In step S1430A, the blood glucose of the user is estimated by the machine learning model 500. In some embodiments, step S1430A may be performed in sequence after step S1420A is performed. The blood glucose value of the user may be estimated based on the normalized peak distance and the occurrence rate, by performing step 1430A, after the normalized peak distance and the occurrence rate have been input into the machine learning model 500. Since the machine learning model 500 has already been trained with the plurality of data and validated with prediction results, the blood glucose value of the user can be estimated by the machine learning model 500 with a prediction accuracy of 0.80 or more, preferably 0.90 or more.

In step S1440A, the blood glucose value of the user is output by the machine learning model 500. In some embodiments, step S1440A may be performed in sequence after step S1430A is performed. In some embodiments, step S1440A may be substantially the same as step S740A as shown in FIG. 7A.

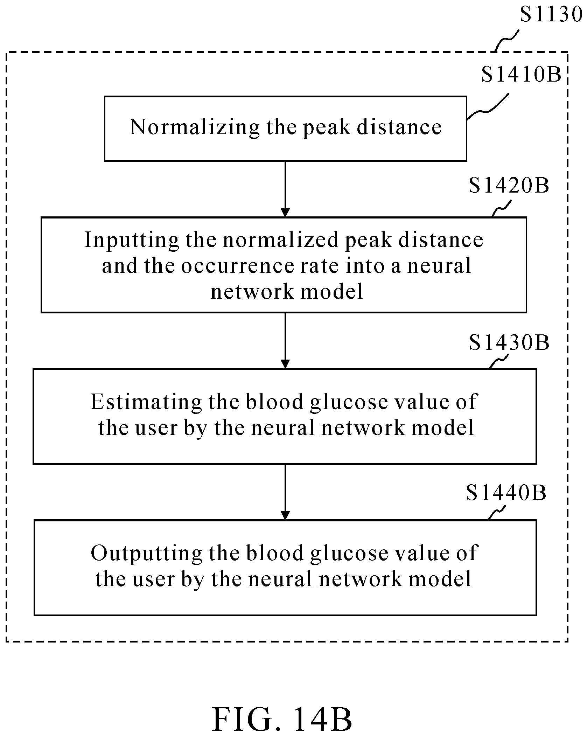

Referring to FIG. 14B, FIG. 14B is a detailed flowchart illustrating a method of estimating the blood glucose value of the user based on the peak distance and the occurrence rate according to an embodiment of the present disclosure. That is, step S1130 as shown in FIG. 11 may include steps S1410B, S1420B, S1430B, and S1440B, and it may be accomplished by performing the steps S1410B, S1420B, S1430B, and S1440B, wherein the steps S1410B, S1420B, S1430B, and S1440B may be performed by the estimating unit 410 of the computing device 400A as shown in FIG. 4.

In step S1410B, the peak distance is normalized. In some embodiments, step S1410B may be performed in sequence after step S640 and step S1120 are performed. In some embodiments, step S1410B may be substantially the same as step S710A as shown in FIG. 7A.

In step S1420B, the normalized peak distance and the occurrence rate are input into the neural network model 510. In some embodiments, step S1420B may be performed in sequence after step S1410B is performed. The normalized peak distance and the occurrence rate may be input into the neural network model 510 by performing step S1420B.

In step S1430B, the blood glucose of the user is estimated by the neural network model 510. In some embodiments, step S1430B may be performed in sequence after step S1420B is performed. The blood glucose value of the user is estimated based on the normalized peak distance and the occurrence rate, by performing step S1430B, after the normalized peak distance and the occurrence rate have been input into the neural network model 510. Since the neural network model 510 has already been trained with the plurality of data and validated with prediction results, the blood glucose value of the user can be estimated by the neural network model 510 with a prediction accuracy of 0.80 or more, preferably 0.90 or more.

In step S1440B, the blood glucose value of the user is output by the neural network model 510. In some embodiments, step S1440B may be performed in sequence after step S1430B is performed. In some embodiments, step S1440B may be substantially the same as step S740B as shown in FIG. 7B.

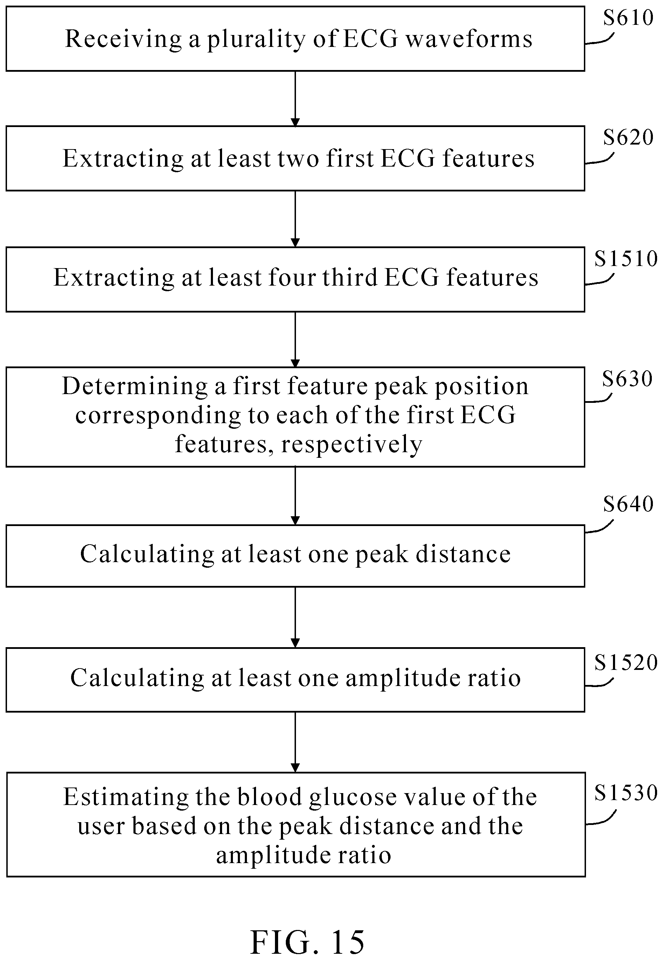

Referring to FIG. 15, FIG. 15 is a flowchart illustrating a fifth example of the method for non-invasively estimating blood glucose according to an embodiment of the present disclosure. The method as shown in FIG. 15 may include steps S610, S620, S630, S640, S1510, S1520, and S1530, wherein the steps S610, S620, S630, and S640 are substantially the same as those shown in FIG. 6.

In step S1510, at least four third ECG features are extracted from each of the plurality of ECG waveforms of the user. Step S1510 may be performed by the extracting unit 404 of the computing device 400A as shown in FIG. 4. In some embodiments, step S1510 may be performed in sequence after step S610 is performed. In addition, step S1510 may be performed at the same time as step S620. The at least four third ECG features may be extracted from each of the plurality of ECG waveforms by performing step S1510, wherein the third ECG features may be selected from a group consisting of a P-wave, a Q-wave, an R-wave, an S-wave, a T-wave, and a U-wave.

In some examples, the P-wave, the Q-wave, the R-wave, and the S-wave of each of the plurality of ECG waveforms may be extracted as the third ECG feature by performing step S1510, respectively. In another embodiment, the Q-wave, the R-wave, the S-wave, and the T-wave of each of the plurality of ECG waveforms may be extracted as the third ECG feature by performing step S1510, respectively. In another embodiment, the Q-wave, the R-wave, the S-wave, and the U-wave of each of the plurality of ECG waveforms may be extracted as the third ECG feature by performing step S1510, respectively. In another embodiment, the P-wave, the Q-wave, the R-wave, the S-wave, the T-wave, and the U-wave of each of the plurality of ECG waveforms may be extracted as the third ECG feature by performing step S1510, respectively. That is, at least four of the P-wave, the Q-wave, the R-wave, the S-wave, the T-wave, and the U-wave of each of the plurality of ECG waveforms may be extracted as the third ECG feature by performing step S1510.

In some embodiments, the third ECG feature may be also extracted by the convolutional neural network model 520. That is, after the plurality of ECG waveforms of the user are input into the convolutional neural network model 520, the third ECG features can be extracted by the convolutional layer of the convolutional neural network model 520.

In step S1520, at least one amplitude ratio is calculated based on the plurality of third ECG features. Step S1520 may be performed by the amplitude ratio calculator 506 of the calculating unit 408 as shown in FIG. 5. In some embodiments, step S1520 may be performed in sequence after step S1510 is performed. The at least one amplitude ratio, e.g., the amplitude ratio of the P-wave but not limited thereto, may be calculated based on the plurality of third ECG features by performing step S1520. Step S1520 may be a step for calculating the at least one amplitude ratio based on an amplitude value (e.g., a maximum value, a minimum value, a root-mean-square value, or a peak-to-peak value, but not limited thereto) of the plurality of third ECG features. More specifically, the at least one amplitude ratio is calculated by performing a series of calculating operation on the plurality of third ECG features.

In some examples, when the extracted third ECG features are a P-wave, a Q-wave, an R-wave, and an S-wave, the amplitude ratio of the P-wave may be calculated by performing step S1520. In another embodiment, when the extracted third ECG features are a Q-wave, an R-wave, an S-wave, and a T-wave, the amplitude ratio of the T-wave may be calculated by performing step S1520. In another embodiment, when the extracted third ECG features are a Q-wave, an R-wave, an S-wave, and a U-wave, the amplitude ratio of the U-wave may be calculated by performing step S1520. In another embodiment, when the extracted third ECG features are a P-wave, a Q-wave, an R-wave, an S-wave, a T-wave, and a U-wave, the amplitude ratio of the P-wave, the amplitude ratio of the T-wave, and/or the amplitude ratio of the U-wave may be calculated by performing step S1520, but is not limited thereto.

In step S1530, the blood glucose value the user is estimated based on the peak distance and the amplitude ratio. Step S1530 may be performed by the estimating unit 410 of the computing device 400A as shown in FIG. 4. In some embodiments, step S1530 may be performed in sequence after step S640 and step S1520 are performed. The blood glucose value of the user may be estimated based on the calculation of the peak distance and the calculation of the amplitude ratio by performing step S1530. More specifically, the blood glucose value of the user is estimated by the estimation model after the calculation of the peak distance and the calculation of the amplitude ratio are input into the estimation model.

By means of the method for non-invasively estimating blood glucose as shown in FIG. 15, it is possible to not only provide a user with a means for non-invasively estimating the blood glucose value of the user, but also to estimate the blood glucose value of the user more accurately by utilizing the peak distance and the amplitude ratio. In addition, by carrying out the method for non-invasively estimating blood glucose as shown in FIG. 15, it is possible to improve the technical field regarding non-invasively estimating the blood glucose value of the user by utilizing a plurality of ECG waveforms.

Furthermore, in some embodiments, the method for non-invasively estimating blood glucose as shown in FIG. 15 may further include step S910 as shown in FIG. 9 and then it may calculate a calibrated blood glucose value of the user by performing step S910.

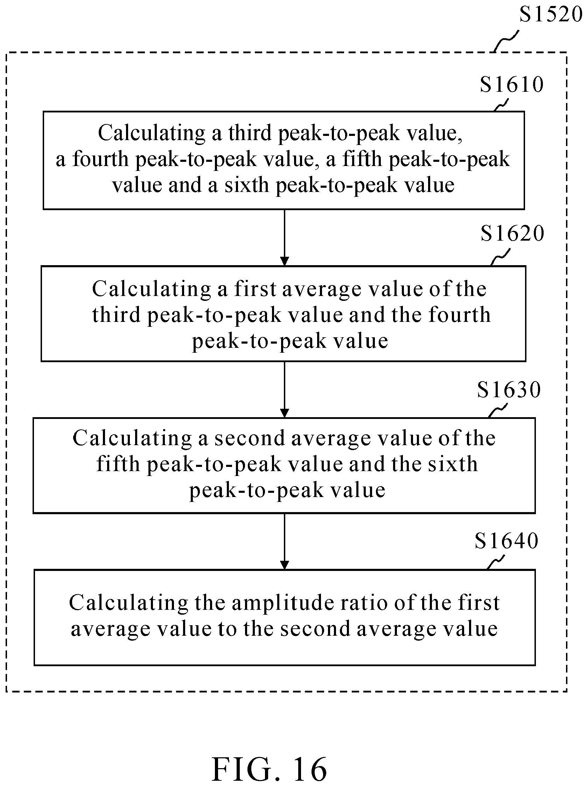

Referring to FIG. 16, FIG. 16 is a detailed flowchart illustrating a method of calculating at least one amplitude ratio based on the third ECG features according to an embodiment of the present disclosure. That is, step S1520 as shown in FIG. 15 may include steps S1610, S1620, S1630, and S1640, and it may be accomplished by performing the steps S1610, S1620, S1630, and S1640, wherein the steps S1610, S1620, S1630, and S1640 may be performed by the amplitude ratio calculator 506 of the calculating unit 408 as shown in FIG. 5.

In step S1610, a third peak-to-peak value, a fourth peak-to-peak value, a fifth peak-to-peak value, and a sixth peak-to-peak value are calculated, respectively. In some embodiments, step S1610 may be performed in sequence after step S1510 is performed. The third peak-to-peak value, the fourth peak-to-peak value, the fifth peak-to-peak value, and the sixth peak-to-peak value may be calculated for each of the plurality of ECG waveforms by performing step S1610.

In some embodiments, when the extracted third ECG features are a P-wave, a Q-wave, an R-wave, and an S-wave, the peak-to-peak value between the maximum of the P-wave and the minimum of the Q-wave as the third peak-to-peak value, the peak-to-peak value between the maximum of the P-wave and the minimum of the S-wave as the fourth peak-to-peak value, the peak-to-peak value between the maximum of the R-wave and the minimum of the Q-wave as the fifth peak-to-peak value, and the peak-to-peak value between the maximum of the R-wave and the minimum of the S-wave as the sixth peak-to-peak value may be calculated for each of the plurality of ECG waveforms by performing step S1610, respectively. In another embodiment, when the extracted third ECG features are a Q-wave, an R-wave, an S-wave, and a T-wave, the peak-to-peak value between the maximum of the absolute value of the T-wave and the minimum of the Q-wave as the third peak-to-peak value, the peak-to-peak value between the maximum of the absolute value of the T-wave and the minimum of the S-wave as the fourth peak-to-peak value, the peak-to-peak value between the maximum of the R-wave and the minimum of the Q-wave as the fifth peak-to-peak value, and the peak-to-peak value between the maximum of the R-wave and the minimum of the S-wave as the sixth peak-to-peak value may be calculated for each of the plurality of ECG waveforms by performing step S 16 10, respectively. In another embodiment, when the extracted third ECG features are a Q-wave, an R-wave, an S-wave, and a U-wave, the peak-to-peak value between the maximum of the U-wave and the minimum of the Q-wave as the third peak-to-peak value, the peak-to-peak value between the maximum of the U-wave and the minimum of the S-wave as the fourth peak-to-peak value, the peak-to-peak value between the maximum of the R-wave and the minimum of the Q-wave as the fifth peak-to-peak value, and the peak-to-peak value between the maximum of the R-wave and the minimum of the S-wave as the sixth peak-to-peak value may be calculated for each of the plurality of ECG waveforms by performing step S 1610, respectively.

In some embodiments, the third peak-to-peak values of each of the plurality of ECG waveforms may be further summed, and then the summed calculation may be divided by the number of third peak-to-peak values in order to calculate an average value of the third peak-to-peak value. Similarly, the fourth peak-to-peak values, the fifth peak-to-peak values and/or the sixth peak-to-peak values may be calculated by the same calculating operation so as to calculate an average value of the fourth peak-to-peak values, an average value of the fifth peak-to-peak values and/or an average value of the sixth peak-to-peak values, respectively. Thereby, the following steps S1620, S1630 and S1640 may be performed in sequence based on the average value of the third peak-to-peak values, the average value of the fourth peak-to-peak values, the average value of the fifth peak-to-peak values and the average value of the sixth peak-to-peak values, so that the amplitude ratios can be calculated with fewer calculating operations (i.e., avoiding calculating for each of the plurality of ECG waveforms in the steps S1620, S1630 and S1640).

In step S1620, a first average value of the third peak-to-peak value and the fourth peak-to-peak value is calculated. In some embodiments, step S1620 may be performed in sequence after step S1610 is performed. The third peak-to-peak value and the fourth peak-to-peak value may be summed, and then the summed calculation may be divided by two in order to calculate the first average value by performing step S1620. In some embodiments, the average value of the third peak-to-peak values and the average value of the fourth peak-to-peak values may be summed, and then the summed calculation may be divided by two in order to calculate the first average value by performing step S1620.

In some embodiments, when the extracted second ECG features are a P-wave, a Q-wave, an R-wave, and an S-wave, the peak-to-peak value between the maximum of the P-wave and the minimum of the Q-wave (i.e., the third peak-to-peak value) and the peak-to-peak value between the maximum of the P-wave and the minimum of the S-wave (i.e., the fourth peak-to-peak value) may be summed, and then the summed calculation may be divided by two in order to calculate the first average value by performing step S 1620. In an embodiment, when the extracted second ECG features are a Q-wave, an R-wave, an S-wave, and a T-wave, the peak-to-peak value between the maximum of the absolute value of the T-wave and the minimum of the Q-wave (i.e., the third peak-to-peak value) and the peak-to-peak value between the maximum of the absolute value of the T-wave and the minimum of the S-wave (i.e., the fourth peak-to-peak value) may be summed, and then the summed calculation may be divided by two in order to calculate the first average value by performing step S 1620. In an embodiment, when the extracted second ECG features are a Q-wave, an R-wave, an S-wave, and a U-wave, the peak-to-peak value between the maximum of the U-wave and the minimum of the Q-wave (i.e., the third peak-to-peak value) and the peak-to-peak value between the maximum of the U-wave and the minimum of the S-wave (i.e., the fourth peak-to-peak value) may be summed, and then the summed calculation may be divided by two in order to calculate the first average value by performing step S 1620.

In step S1630, a second average value of the fifth peak-to-peak value and the sixth peak-to-peak value is calculated. In some embodiments, step S1630 may be performed in sequence after step S1620 is performed. In addition, step S1630 may be performed at the same time as step S1620. The fifth peak-to-peak value and the sixth peak-to-peak value may be summed, and then the summed calculation may be divided by two in order to calculate the second average value by performing step S1630. In some embodiments, the average value of the fifth peak-to-peak values and the average value of the sixth peak-to-peak values may be summed, and then the summed calculation may be divided by two in order to calculate the second average value by performing step S1630.

In some embodiments, when the extracted second ECG features are a P-wave, a Q-wave, an R-wave, and an S-wave, the peak-to-peak value between the maximum of the R-wave and the minimum of the Q-wave (i.e., the fifth peak-to-peak value) and the peak-to-peak value between the maximum of the R-wave and the minimum of the S-wave (i.e., the sixth peak-to-peak value) may be summed, and then the summed calculation may be divided by two in order to calculate the second average value by performing step S1630. In an embodiment, when the extracted second ECG features are a Q-wave, an R-wave, an S-wave, and a T-wave, the peak-to-peak value between the maximum of the R-wave and the minimum of the Q-wave (i.e., the fifth peak-to-peak value) and the peak-to-peak value between the maximum of the R-wave and the minimum of the S-wave (i.e., the sixth peak-to-peak value) may be summed, and then the summed calculation may be divided by two in order to calculate the second average value by performing step S1630. In an embodiment, when the extracted second ECG features are a Q-wave, an R-wave, an S-wave, and a U-wave, the peak-to-peak value between the maximum of the R-wave and the minimum of the Q-wave (i.e., the fifth peak-to-peak value) and the peak-to-peak value between the maximum of the R-wave and the minimum of the S-wave (i.e., the sixth peak-to-peak value) may be summed, and then the summed calculation may be divided by two in order to calculate the second average value by performing step S1630.

In step S1640, a ratio of the first average value to the second average value is calculated as the amplitude ratio. In some embodiments, step S1640 may be performed in sequence after step S1630 is performed. The ratio of the first average value to the second average value may be calculated as the amplitude ratio by performing step S1640.

By means of performing the steps as shown in FIG. 16, the at least one amplitude ratio can be calculated more accurately based on the ratio of the first average value to the second average value by converting the peak-to-peak values between the plurality of third ECG features into the first average value and the second average value, wherein the amplitude ratio may include the amplitude ratio of the P-wave, the amplitude ratio of the T-wave, and/or the amplitude ratio of the U-wave, but is not limited thereto.

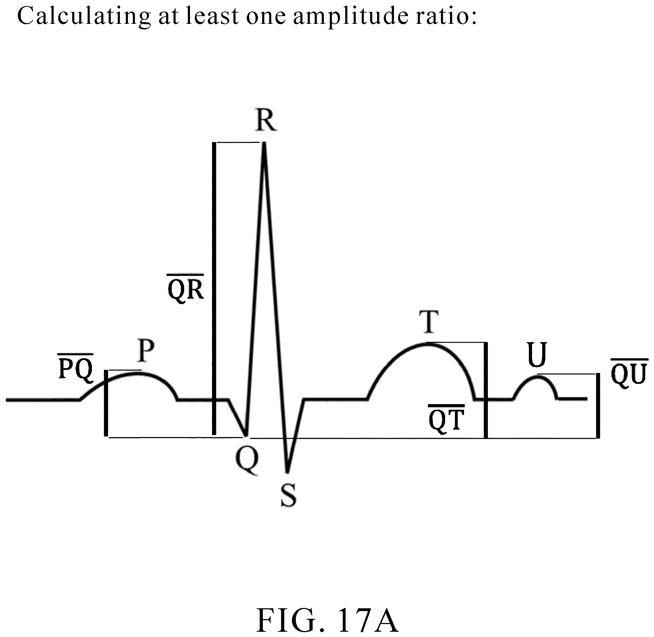

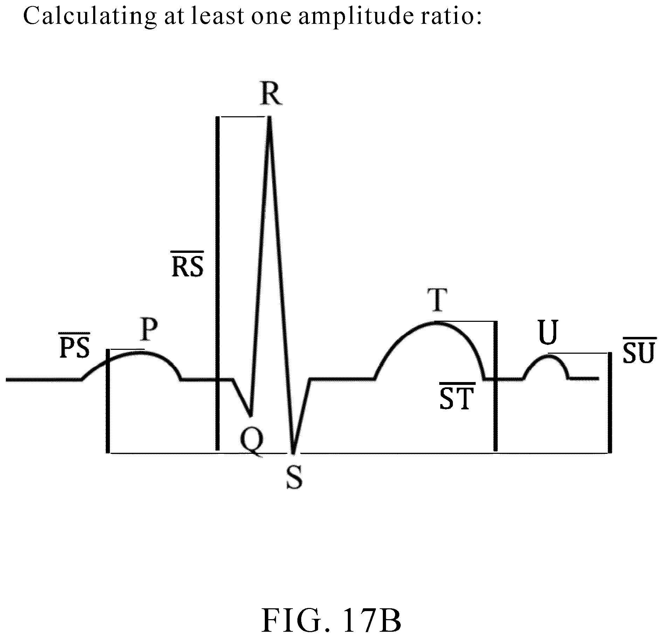

Referring to both FIG. 17A and FIG. 17B, FIG. 17A is a waveform diagram illustrating an example of calculating at least one amplitude ratio based on the third ECG features according to an embodiment of the present disclosure, and FIG. 17B is a waveform diagram illustrating another example of calculating at least one amplitude ratio based on the third ECG features according to an embodiment of the present disclosure. As shown in FIGS. 17A and 17B, for example, taking an ECG waveform as an example, when the extracted third ECG features are a P-wave, a Q-wave, an R-wave, an S-wave, a T-wave, and a U-wave, by performing the steps as shown in FIG. 16, the peak-to-peak values between the plurality of third ECG features may be calculated, respectively, and the first average value and the second average value may be calculated based on the peak-to-peak values, respectively, in order to calculate the at least one amplitude ratio.

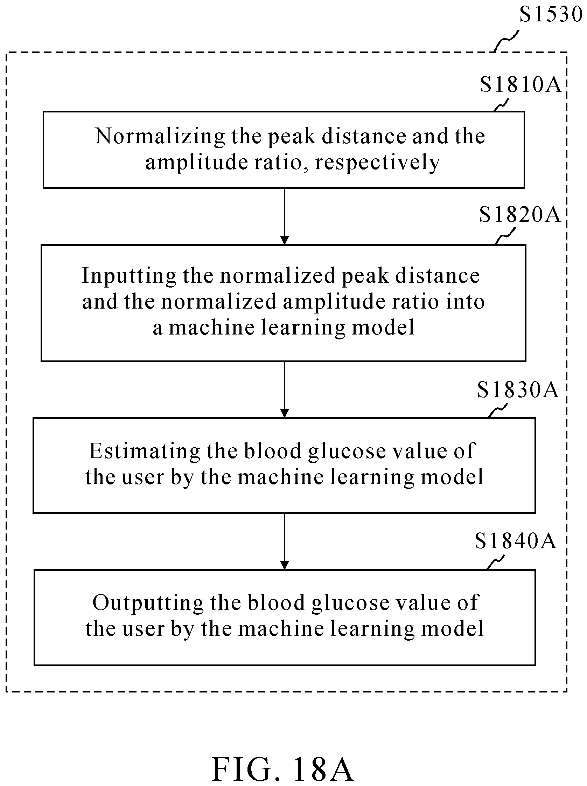

Referring to FIG. 18A, FIG. 18A is a detailed flowchart illustrating a method of estimating the blood glucose value of the user based on the peak distance and the amplitude ratio according to an embodiment of the present disclosure. That is, step S1530 as shown in FIG. 15 may include steps S1810A, S1820A, S1830A, and S1840A, and it may be accomplished by performing the steps S1810A, S1820A, S1830A, and S1840A, wherein the steps S1810A, S1820A, S1830A, and S1840A may be performed by the estimating unit 410 of the computing device 400A as shown in FIG. 4.

In step S1810A, the peak distance and the amplitude ratio are normalized, respectively. In some embodiments, step S1810A may be performed in sequence after step S640 and step S1520 are performed. The calculation of the peak distance may be normalized to a corresponding value between a range (e.g., between 0 and 1) and the calculation of the amplitude ratio may be normalized to a corresponding value between a range (e.g., between 0 and 1) by performing step S1810A.

In step S1820A, the normalized peak distance and the normalized amplitude ratio are input into the machine learning model 500. In some embodiments, step S1820A may be performed in sequence after step S1810A is performed. The normalized peak distance and the normalized amplitude ratio may be input into the machine learning model 500, such as a decision tree algorithm, a random forest algorithm incorporating bagging, an extreme gradient boosting (XGBoost) algorithm, or a support vector machine algorithm, by performing step S1820A.

In step S1830A, the blood glucose value of the user is estimated by the machine learning model 500. In some embodiments, step S1830A may be performed in sequence after step S1820A is performed. The blood glucose value of the user may be estimated based on the normalized peak distance and the normalized amplitude ratio, by performing step 1830A, after the normalized peak distance and the normalized amplitude ratio have been input into the machine learning model 500. Since the machine learning model 500 has already been trained with the plurality of data and validated with prediction results, the blood glucose value of the user can be estimated by the machine learning model 500 with a prediction accuracy of 0.80 or more, preferably 0.90 or more.

In step S1840A, the blood glucose value of the user is output by the machine learning model 500. In some embodiments, step S1840A may be performed in sequence after step S1830A is performed. In some embodiments, step S1840A may be substantially the same as step S740A as shown in FIG. 7A.

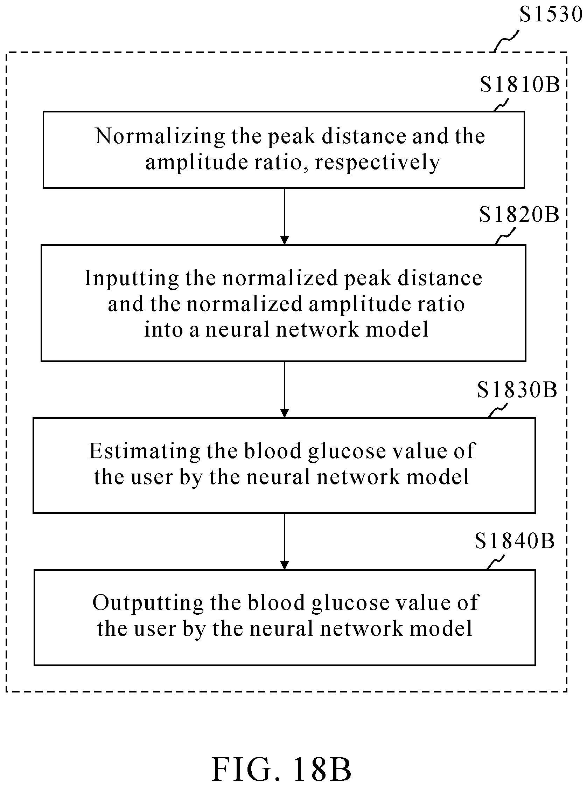

Referring to FIG. 18B, FIG. 18B is a detailed flowchart illustrating a method of estimating the blood glucose value of the user based on the peak distance and the amplitude ratio according to an embodiment of the present disclosure. That is, step S1530 as shown in FIG. 15 may include steps S1810B, S1820B, S1830B, and S1840B, and it may be accomplished by performing the steps S1810B, S1820B, S1830B, and S1840B, wherein the steps S1810B, S1820B, S1830B, and S1840B may be performed by the estimating unit 410 of the computing device 400A as shown in FIG. 4.

In step S1810B, the peak distance and the amplitude ratio are normalized, respectively. In some embodiments, step S1810B may be performed in sequence after step S640 and step S 1520 are performed. In some embodiments, step S1810B may be substantially the same as step S1810A as shown in FIG. 18A.

In step S1420B, the normalized peak distance and the normalized amplitude ratio are input into the neural network model 510. In some embodiments, step S1820B may be performed in sequence after step S1810B is performed.

The normalized peak distance and the normalized amplitude ratio may be input into the neural network model 510 by performing step S1820B.

In step S1830B, the blood glucose value of the user is estimated by the neural network model 510. In some embodiments, step S1830B may be performed in sequence after step S1820B is performed. The blood glucose value of the user is estimated based on the normalized peak distance and the normalized amplitude ratio, by performing step S1430B, after the normalized peak distance and the normalized amplitude ratio have been input into the neural network model 510. Since the neural network model 510 has already been trained with the plurality of data and validated with prediction results, the blood glucose value of the user can be estimated by the neural network model 510 with a prediction accuracy of 0.80 or more, preferably 0.90 or more.

In step S1840B, the blood glucose value of the user is output by the neural network model 510. In some embodiments, step S1840B may be performed in sequence after step S1830B is performed. In some embodiments, step S1840B may be substantially the same as step S740B as shown in FIG. 7B.

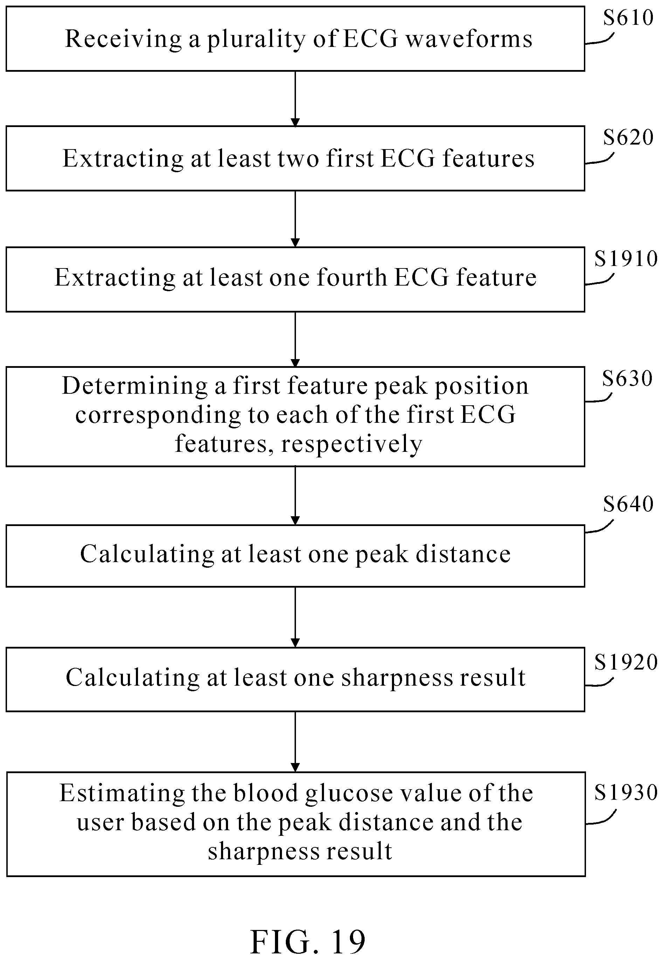

Referring to FIG. 19, FIG. 19 is a flowchart illustrating a sixth example of the method for non-invasively estimating blood glucose according to an embodiment of the present disclosure. The method as shown in FIG. 19 may include steps S610, S620, S630, S640, S1910, S1920 and S1930, wherein the steps S610, S620, S630, and S640 are substantially the same as those shown in FIG. 6.

In step S1910, at least one fourth ECG feature is extracted from each of the plurality of ECG waveforms of the user. Step S1910 may be performed by the extracting unit 404 of the computing device 400A as shown in FIG. 4. In some embodiments, step S 1910 may be performed in sequence after step S610 is performed. In addition, step S1910 may be performed at the same time as step S620. The fourth ECG feature may be selected from a group consisting of a P-wave, a Q-wave, an R-wave, an S-wave, a T-wave, and a U-wave. In some embodiments, step S1910 may be substantially similar to step S620 as shown in FIG. 6.

In some embodiments, the fourth ECG feature may be also extracted by the convolutional neural network model 520. That is, after the plurality of ECG waveforms of the user are input into the convolutional neural network model 520, the fourth ECG feature can be extracted by the convolutional layer of the convolutional neural network model 520.

In step S1920, at least one sharpness result is calculated based on the fourth ECG feature. Step S1920 may be performed by the calculating unit 408 of the computing device 400A as shown in FIG. 4. In some embodiments, step S1920 may be performed in sequence after step S1910 is performed. Step S1920 may be a step for calculating the sharpness result based on an amplitude value (e.g., a maximum value, or a minimum value, but not limited thereto) of the fourth ECG feature. More specifically, the sharpness result is calculated by performing a series of calculating operation on the fourth ECG feature.

In step S1930, the blood glucose value the user is estimated based on the peak distance and the sharpness result. Step S1930 may be performed by the estimating unit 410 of the computing device 400A as shown in FIG. 4. In some embodiments, step S1930 may be performed in sequence after step S640 and step S1920 are performed. The blood glucose value of the user may be estimated based on the calculation of the peak distance and the calculation of the sharpness result by performing step S1930. More specifically, the blood glucose value of the user is estimated by the estimation model after the calculation of the peak distance and the calculation of the sharpness result are input into the estimation model.

By means of the method for non-invasively estimating blood glucose as shown in FIG. 19, it is possible to not only provide a user with a means for non-invasively estimating the blood glucose value of the user, but also to estimate the blood glucose value of the user more accurately by utilizing the peak distance and the sharpness result. In addition, by carrying out the method for non-invasively estimating blood glucose as shown in FIG. 19, it is possible to improve the technical field regarding non-invasively estimating the blood glucose value of the user by utilizing a plurality of ECG waveforms.

Moreover, by means of the method for non-invasively estimating blood glucose as shown in FIG. 19, if the plurality of ECG waveforms with an inverted T-wave is received, it can estimate the blood glucose value of the user more accurately by utilizing the sharpness result.

Furthermore, in some embodiments, the method for non-invasively estimating blood glucose as shown in FIG. 19 may further include step S910 as shown in FIG. 9 and then it may calculate a calibrated blood glucose value of the user by performing step S910.

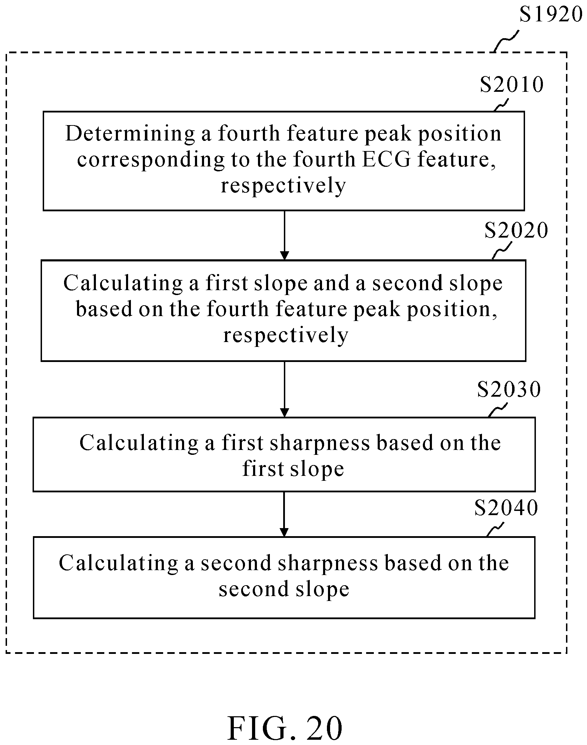

Referring to FIG. 20, FIG. 20 is a detailed flowchart illustrating a method of calculating the sharpness result based on the fourth ECG feature according to an embodiment of the present disclosure. That is, step S1920 as shown in FIG. 19 may include steps S2010, S2020, S2030, and S2040, and it may be accomplished by performing the steps S2010, S2020, S2030, and S2040, wherein the steps S2010, S2020, S2030, and S2040 may be performed by the calculating unit 408 of the computing device 400A as shown in FIG. 4.

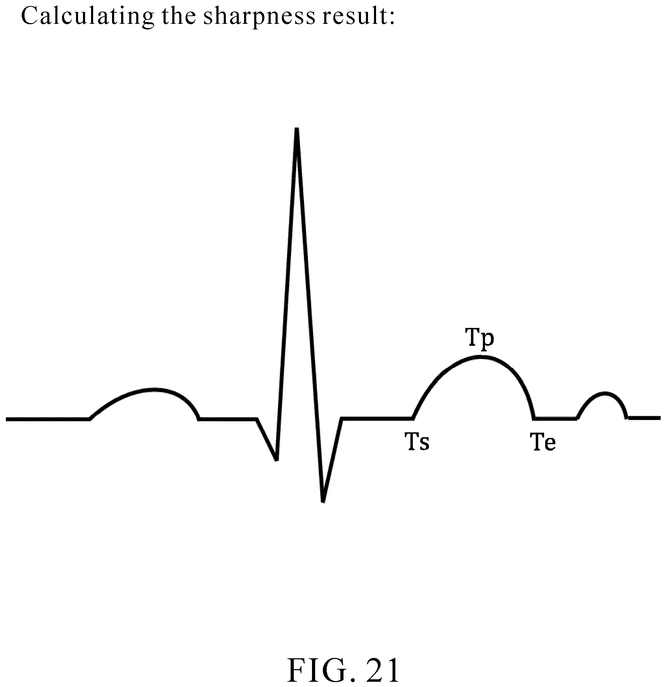

In step S2010, a fourth feature peak position corresponding to the fourth ECG feature is determined, respectively. In some embodiments, step S2010 may be performed in sequence after step S 1910 is performed. In some embodiments, step S2010 may be substantially similar to step S630 as shown in FIG. 6. In some embodiments, the fourth feature peak position may be referred to the position of the maximum of the P-wave, the position of the minimum of the Q-wave, the position of the maximum of the R-wave, the position of the minimum of the S-wave, the position of the maximum of the absolute value of the T-wave, and/or the position of the maximum of the U-wave. In some examples, when the extracted fourth ECG feature is a T-wave, the position of the maximum of the absolute value of the T-wave (i.e., "$T_P$" as shown in FIG. 21) in each of the plurality of ECG waveforms may be determined by performing step S2010, respectively.

In step S2020, a first slope and a second slope are calculated based on the fourth feature peak position, respectively. In some embodiments, step S2020 may be performed in sequence after step S2010 is performed.

The first slope is calculated based on the fourth feature peak position. More specifically, the first slope is calculated by an equation. In some embodiments, when the extracted fourth ECG feature is a T-wave, the equation

$$SL1 = \frac{T0 - \sum_{i=1}^{N} Ti/N}{N/2}$$

described above is that , wherein the "SL1" is the first slope, the "$T_0$" is the amplitude value of the T-wave position, and the "N" is the number of sampling points. For example, the "N" is 10.

The second slope is calculated based on the fourth feature peak position. More specifically, the second slope is calculated by an equation. In some embodiments, when the extracted fourth ECG feature is a T-wave, the equation

$$SL2 = \frac{T0 - \sum_{i=-N}^{-1} Ti/N}{N/2}$$

described above is that , wherein the "SL2" is the second slope, the "$T_0$" is the amplitude value of the fourth feature peak position (i.e., the amplitude value of the "$T_P$"), and the "N" is the number of sampling points. For example, the "N" is 10.

In step S2030, a first sharpness is calculated based on the first slope. In some embodiments, step S2030 may be performed in sequence after step S2020 is performed. The first sharpness is calculated by an equation. In some embodiments, when the extracted fourth ECG feature is a T-wave, the equation described above is

$$SP1 = \sum_{i=-N}^{-1} Ti - (T0 + SL1 \times i)$$

, wherein "SP1" is the first sharpness, "$T_0$" is the amplitude value of the fourth feature peak position (i.e., the amplitude value of "$T_P$"), "SL1" is the first slope, and "N" is the number of sampling points. For example, "N" is 10.

In step S2040, a second sharpness is calculated based on the second slope. In some embodiments, step S2040 may be performed in sequence after step S2030 is performed. In addition, step S2040 may be performed at the same time as step S2030. The second sharpness is calculated by an equation. In some embodiments, when the extracted fourth ECG feature is a T-wave, the equation described above is $$SP2 = \sum_{i=1}^{N} Ti - (T0 - SL2 \times i)$$ , wherein "SP2" is the second sharpness, "$T_0$" is the amplitude value of the fourth feature peak position (i.e., the amplitude value of "$T_P$"), "SL2" is the second slope, and "N" is the number of sampling points. For example, "N" is 10.

The first sharpness and the second sharpness are treated as the sharpness result. Therefore, the sharpness result can be calculated by performing the steps S2010, S2020, S2030, and S2040.

Referring to FIG. 21, FIG. 21 is a waveform diagram illustrating calculating the sharpness result based on the fourth ECG feature according to an embodiment of the present disclosure. In some embodiments, the sharpness result may be calculated by a differential calculation and an integral calculation.

The first slope is calculated based on the position of the maximum of the absolute value of the T-wave. More specifically, the first slope is calculated by a differential equation. The differential equation described above is

$$SL1 = \left| \frac{d(\overrightarrow{TpTs}(x))}{dx} \right|$$

, wherein "SL1" is the first slope, "$T_P$" is the amplitude value of the fourth feature peak position (i.e., the amplitude value of "$T_P$"), and "$T_S$" is the amplitude value of the start point of the T-wave.

The second slope is calculated based on the position of the maximum of the absolute value of the T-wave. More specifically, the second slope is calculated by a differential equation. The differential equation described above is

$$SL2 = \left| \frac{d(\overrightarrow{TpTe}(x))}{dx} \right|$$

, wherein "SL2" is the second slope, "$T_P$" is the amplitude value of the fourth feature peak position (i.e., the amplitude value of "$T_P$"), and "$T_e$" is the amplitude value of the end point of the T-wave.

The first sharpness is calculated based on the position of the maximum of the absolute value of the T-wave. More specifically, the first sharpness is calculated by an integral equation. The integral equation described above is

$$SP1 = \int_{Ts}^{Tp} ECG(x) - \overline{TsTp}(x) \, dx$$

, wherein "SP1" is the first sharpness, "$T_P$" is the amplitude value of the fourth feature peak position (i.e., the amplitude value of "$T_P$"), "$T_S$" is the amplitude value of the start point of the T-wave, and "ECG(x)" is the plurality of ECG waveforms.

The second sharpness is calculated based on the position of the maximum of the absolute value of the T-wave. More specifically, the second sharpness is calculated by an integral equation. The integral equation described above is

$$SP2 = \int_{Tp}^{Te} ECG(x) - \overline{TpTe}(x)\, dx$$

, wherein "SP2" is the second sharpness, "$T_P$" is the amplitude value of the fourth feature peak position (i.e., the amplitude value of "$T_P$"), "$T_e$" is the amplitude value of the end point of the T-wave, and "ECG(x)" is the plurality of ECG waveforms.

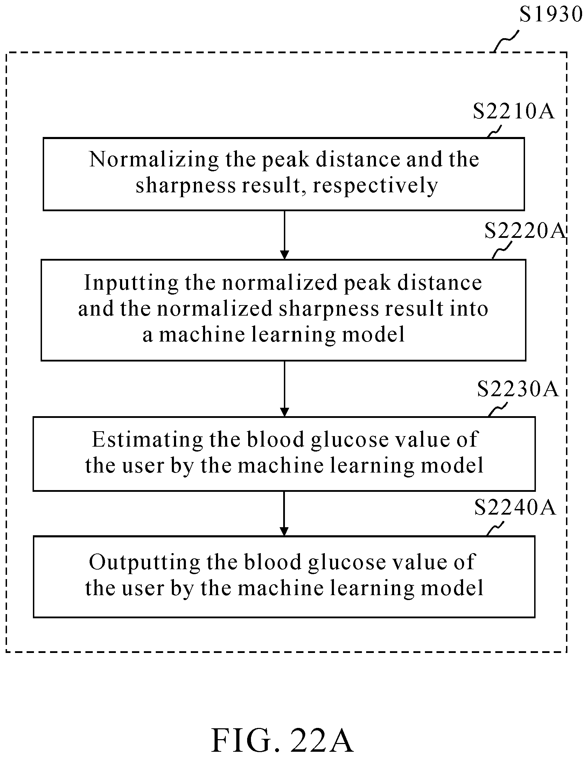

Referring to FIG. 22A, FIG. 22A is a detailed flowchart illustrating a method of estimating the blood glucose value of the user based on the peak distance and the sharpness result according to an embodiment of the present disclosure.

In step S2210A, the peak distance and the sharpness result are normalized, respectively. In some embodiments, step S2210A may be performed in sequence after step S640 and step S1920 are performed. The calculation of the peak distance may be normalized to a corresponding value between a range (e.g., between 0 and 1) and the calculation of the sharpness result may be normalized to a corresponding value between a range (e.g., between -2,000 and 2,000 by logarithm) by performing step S2210A.

In step S2220A, the normalized peak distance and the normalized sharpness result are input into the machine learning model 500. In some embodiments, step S2220A may be performed in sequence after step S2210A is performed. The normalized peak distance and the normalized sharpness result may be input into the machine learning model 500, such as a decision tree algorithm, a random forest algorithm incorporating bagging, an extreme gradient boosting (XGBoost) algorithm, or a support vector machine algorithm, by performing step S2220A.

In step S2230A, the blood glucose value of the user is estimated by the machine learning model 500. In some embodiments, step S2230A may be performed in sequence after step S2220A is performed. The blood glucose value of the user may be estimated based on the normalized peak distance and the normalized sharpness result, by performing step 2230A, after the normalized peak distance and the normalized sharpness result have been input into the machine learning model 500. Since the machine learning model 500 has already been trained with the plurality of data and validated with prediction results, the blood glucose value of the user can be estimated by the machine learning model 500 with a prediction accuracy of 0.80 or more, preferably 0.90 or more.

In step S2240A, the blood glucose value of the user is output by the machine learning model 500. In some embodiments, step S2240A may be performed in sequence after step S2230A is performed. In some embodiments, step S2240A may be substantially the same as step S740A as shown in FIG. 7A.

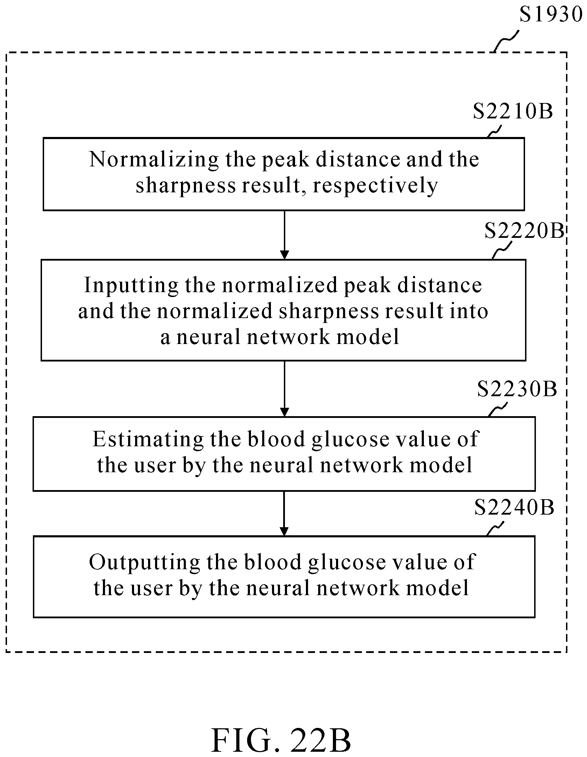

Referring to FIG. 22B, FIG. 22B is a detailed flowchart illustrating a method of estimating the blood glucose value of the user based on the peak distance and the sharpness result according to an embodiment of the present disclosure.

In step S2210B, the peak distance and the sharpness result are normalized, respectively. In some embodiments, step S2210B may be performed in sequence after step S640 and step S1920 are performed. In some embodiments, step S2210B may be substantially the same as step S2210A as shown in FIG. 22A.

In step S2220B, the normalized peak distance and the normalized sharpness result are input into the neural network model 510. In some embodiments, step S2220B may be performed in sequence after step S2210B is performed. The normalized peak distance and the normalized sharpness result may be input into the neural network model 510 by performing step S2220B.

In step S2230B, the blood glucose value of the user is estimated by the neural network model 510. In some embodiments, step S2230B may be performed in sequence after step S2220B is performed. The blood glucose value of the user is estimated based on the normalized peak distance and the normalized sharpness result, by performing step S2230B, after the normalized peak distance and the normalized sharpness result have been input into the neural network model 510. Since the neural network model 510 has already been trained with the plurality of data and validated with prediction results, the blood glucose value of the user can be estimated by the neural network model 510 with a prediction accuracy of 0.80 or more, preferably 0.90 or more.

In step S1840B, the blood glucose value of the user is output by the neural network model 510. In some embodiments, step S1840B may be performed in sequence after step S1830B is performed. In some embodiments, step S1840B may be substantially the same as step S740B as shown in FIG. 7B.

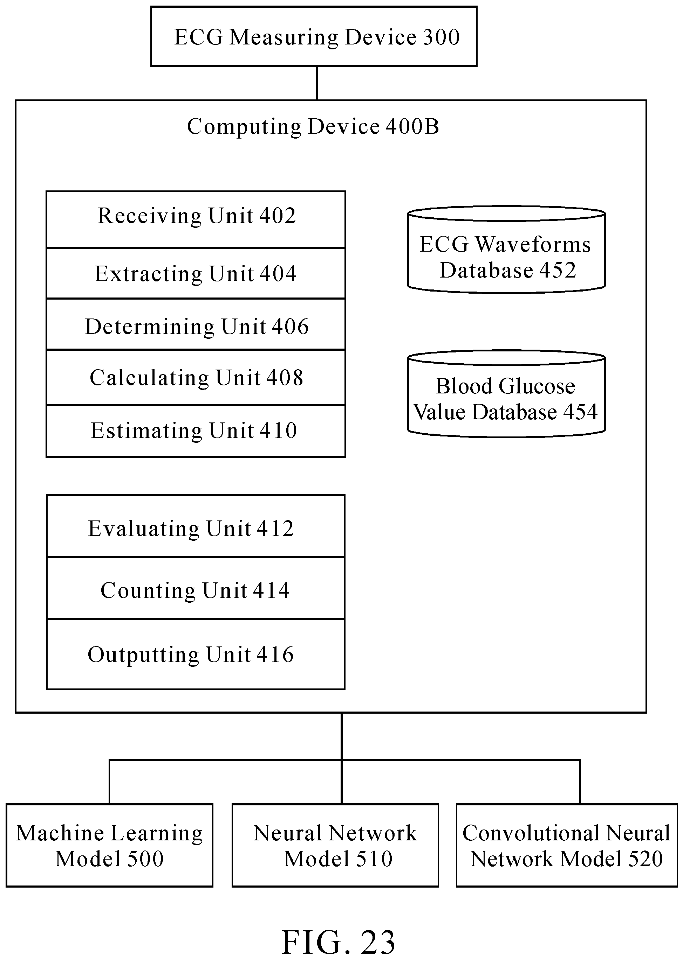

Referring to FIG. 23, FIG. 23 is a schematic diagram illustrating a computing device 400B that is electrically coupled with an ECG measuring device 300 and with at least one of a machine learning model 500, a neural network model 510, and a convolution neural network model 520 according to an embodiment of the present disclosure. The computing device 400B may have the same configuration and function as the computing device 400A as shown in FIG. 4, and further include an evaluating unit 412, a counting unit 414, and an outputting unit 416.

The evaluating unit 412 may be configured to evaluate the quality of the plurality of ECG waveforms. That is, the computing device 400B may evaluate the quality of the plurality of ECG waveforms that have been received by the evaluating unit 412. More specifically, the evaluating unit 412 may evaluate whether the plurality of ECG waveforms with the noise or not. If the result of the evaluation of the plurality of ECG waveforms for the noise is less than a default noise threshold, it may be determined that the quality of the complex ECG waveforms is a good ECG waveform (i.e., a normal ECG signal). Conversely, if the result of the evaluation of the plurality of ECG waveforms for the noise is greater than or equal to a default noise threshold, it may be determined that the quality of the plurality of ECG waveforms is a bad ECG waveform (i.e., noise).

In some embodiments, the evaluating unit 412 may evaluate the quality of the plurality of ECG waveforms that have been received by calculating the distribution of the value of RR intervals between adjacent ECG waveforms, the duration of each of the plurality of ECG waveforms, the heart rate of the user, the distribution of each of the plurality of ECG features, the amplitude value of each of the plurality of ECG features, and/or the degree of the similarity between the plurality of ECG waveforms.

In some embodiments, when the values of RR intervals between adjacent ECG waveforms differ from each other by a small amount (e.g., by a difference ratio within 0.1) and the percentage of outliers is less than a default value (e.g., 0.1), the quality of the plurality of ECG waveforms may be determined to be good ECG waveforms. In some embodiments, when the durations of each of the plurality of ECG waveforms differ from each other by a small amount (e.g., by a difference ratio within 0.1) and the percentage of outliers is less than a default value (e.g., 0.1), the quality of the plurality of ECG waveforms may be determined to be good ECG waveforms. In some embodiments, when the heart rate of the user falls within the normal range, the quality of the plurality of ECG waveforms may be determined to be good ECG waveforms. In some embodiments, when the distribution of each of the plurality of ECG features differ from each other by a small amount (e.g., by a difference ratio that the time difference between the P-wave and the R-wave of each of the plurality of ECG waveforms is within 0.1) and the percentage of outliers is less than a default value (e.g., 0.1), the quality of the plurality of ECG waveforms may be determined to be good ECG waveforms. In some examples, when the amplitude values of each of the plurality of ECG features differ from each other by a small amount (e.g., by a difference ratio within 0.1) and the percentage of outliers is less than a default value (e.g., 0.1), the quality of the plurality of ECG waveforms may be determined to be good ECG waveforms. In some embodiments, when the degree of similarity between the plurality of ECG waveforms is large (e.g., the degree of similarity is more than 0.9) and the percentage of outliers is less than a default value (e.g., 0.1), the quality of the plurality of ECG waveforms may be determined to be good ECG waveforms.

The counting unit 414 may be configured to count the number of the plurality of ECG waveforms of the user that have been received. That is, when the computing device 400B begins receiving the plurality of ECG waveforms, the computing device 400B may start to receive the amplitude value of each point for the plurality of ECG waveforms, convert the amplitude value of each point into a corresponding waveform, and count the number of the corresponding waveforms.

In some embodiments, the counting unit 414 may be further configured to compare the number of the corresponding waveforms (i.e., the plurality of ECG waveforms that have been received) with the default value in order to determine whether the computing device 400B has received an enough number of the plurality of ECG waveforms. In some embodiments, the default value may be set to 60 or more, but is not limited thereto.

The outputting unit 416 may be configured to output the blood glucose value of the user. That is, the outputting unit 416 may output the blood glucose value of the user that is estimated by the estimating unit 410 and/or the calibrated blood glucose value of the user that is calculated by the calibrated blood glucose value calculator 508. In some embodiments, since the blood glucose value of the user that is estimated by the estimating unit 410 and/or the calibrated blood glucose value of the user that is calculated by the calibrated blood glucose value calculator 508 may be synchronously stored in the blood glucose value database 454, the outputting unit 416 may output the blood glucose value that is stored in the blood glucose value database 454.

Thereby, the computing device 400B provided in the present disclosure can be used to carry out the methods for non-invasively estimating blood glucose, such that the computing device 400B, after receiving the plurality of ECG waveforms of the user, can estimate the blood glucose value of the user and/or calculate the calibrated blood glucose value of the user based on the plurality of ECG waveforms of the user.

In addition, the computing device 400B provided in the present disclosure can further evaluate the quality and the quantity of the plurality of ECG waveforms that have been received to ensure that the methods for non-invasively estimating blood glucose are carried out in a certain quantity and/or a certain quality of the plurality of ECG waveforms so as to estimate the blood glucose value of the user and/or calculate the calibrated blood glucose value of the user more accurately.

By means of the computing device 400B as shown in FIG. 23, it is possible to not only provide a user with a means for non-invasively estimating the blood glucose value of the user, but also to estimate the blood glucose value of the user more accurately. In addition, by means of the computing device 400B as shown in FIG. 23, it is possible to improve the technical field regarding non-invasively estimating the blood glucose value of the user by utilizing a plurality of ECG waveforms.

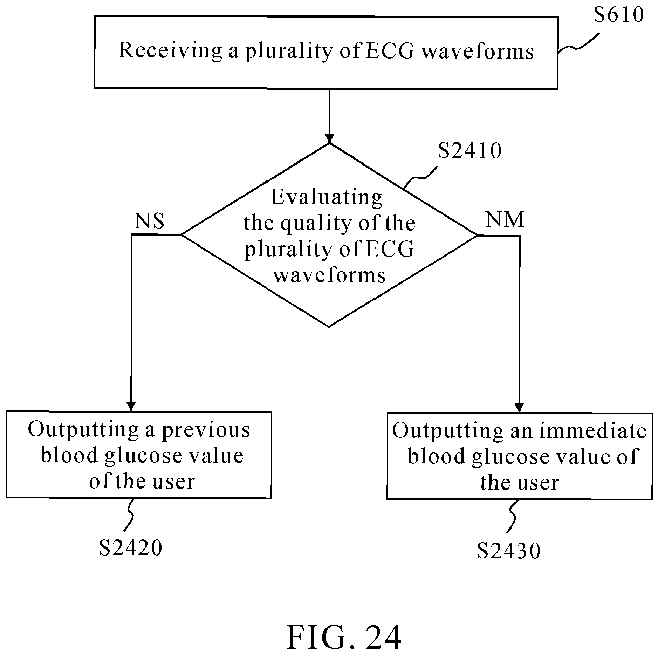

Referring to FIG. 24, FIG. 24 is a flowchart illustrating a seventh example of the method for non-invasively estimating blood glucose according to an embodiment of the present disclosure. The method as shown in FIG. 24 may include steps S610, S2410, S2420, and S2430, wherein step S610 is substantially the same as that shown in FIG. 6.

In step S2410, the quality of the plurality of ECG waveforms is evaluated. Step S2410 may be performed by the evaluating unit 412 of the computing device 400B as shown in FIG. 23. In some embodiments, step S2410 may be performed in sequence after step S610 is performed. The quality of the plurality of ECG waveforms received by the receiving unit 402 may be evaluated by performing step S2410.

When the plurality of ECG waveforms is evaluated as the noise NS (i.e., the result of the evaluation of the plurality of ECG waveforms for the noise is more than or equal to the default noise threshold), the previous blood glucose value of the user may be output (i.e., performing step S2420). When the plurality of ECG waveforms is evaluated as normal ECG signal NM (i.e., the result of the evaluation of the plurality of ECG waveforms for the noise is less than or equal to the default noise threshold), the immediate blood glucose value of the user may be output (i.e., performing step S2430).

In step S2420, the previous blood glucose value of the user is output. Step S2420 may be performed by the outputting unit 416 of the computing device 400B as shown in FIG. 23. In some embodiments, step S2420 may be performed in sequence after step S2410 is performed. The previous blood glucose value of the user may be output by performing step S2420.

In some embodiments, the method provided in the present disclosure may be a method to estimate the blood glucose value of the user in a period of the time (e.g., per second), such that the previous blood glucose value of the user may be output when step S2420 is performed due to the plurality of ECG waveforms being evaluated as the noise NS at the time point A and then the previous blood glucose value may refer to a blood glucose value of the user obtained prior to the time point A (e.g., a blood glucose value estimated at the time point A-1 or a blood glucose value estimated at the time point of the latest evaluation being a normal ECG signal NM). In some embodiments, the previous blood glucose value may refer to a blood glucose value that is stored in the blood glucose value database 454, and the previous blood glucose value may be the blood glucose value of the user estimated by the estimating unit 410 and/or the calibrated blood glucose value of the user calculated by the calibrated blood glucose value calculator 508.

In step S2430, the immediate blood glucose value of the user is output. Step S2430 may be performed by the outputting unit 416 of the computing device 400B as shown in FIG. 23. In some embodiments, step S2430 may be performed in sequence after step S2410 is performed. In some embodiments, the blood glucose value of the user estimated by the estimating unit 410 and/or the calibrated blood glucose value of the user calculated by the calibrated blood glucose value calculator 508 may be output immediately. In other embodiments, the blood glucose value of the user that is synchronously stored in the blood glucose value database 454 may be immediately output.

By means of the method for non-invasive estimation of blood glucose as shown in FIG. 24, it is possible to not only provide the user with a means for non-invasively estimating the blood glucose value of the user, but also to estimate the blood glucose value of the user more accurately by utilizing a certain quality of the plurality of ECG waveforms. That is, by carrying out the method for non-invasively estimating blood glucose as shown in FIG. 24, it can ensure that the method described above estimate the blood glucose value of the user (i.e., the result of the estimation of the blood glucose value of the user will not be significantly biased due to the plurality of ECG waveforms with noise) based on the certain quality of the plurality of ECG waveforms. In addition, by carrying out the method for non-invasively estimating blood glucose as shown in FIG. 24, it is possible to improve the technical field regarding non-invasively estimating the blood glucose value of the user by utilizing a plurality of ECG waveforms.

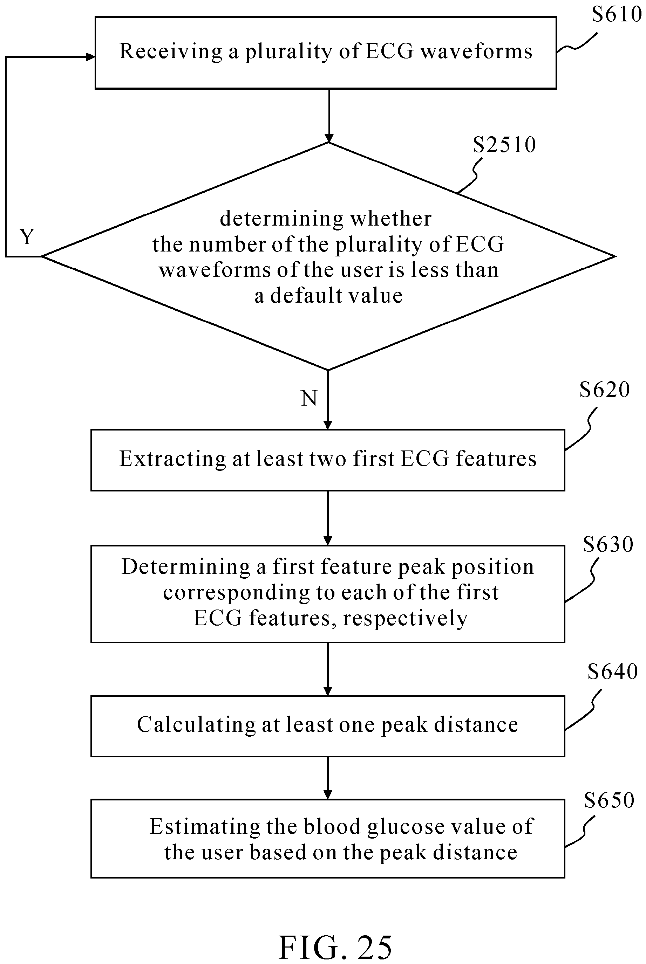

Referring to FIG. 25, FIG. 25 is a flowchart illustrating an eighth example of the method for non-invasively estimating blood glucose according to an embodiment of the present disclosure. The method as shown in FIG. 25 may include steps S610, S620, S630, S640, S650, and S2510, wherein the steps S610, S620, S630, S640, and S650 are substantially the same as those shown in FIG. 6.

In step S2510, determine whether the number of the plurality of ECG waveforms of the user is less than a default value. Step S2510 may be performed by the counting unit 414 of the computing device 400B as shown in FIG. 23. In some embodiments, step S2510 may be performed in sequence after step S610 is performed. Step S2510 may be performed to compare the number of the plurality of ECG waveforms that have been received with the default value in order to determine whether the number of the plurality of ECG waveforms of the user is less than a default value.

If the number of the plurality of ECG waveforms that have been received is more than or equal to the default value (i.e., "N" as shown in FIG. 25), then step S620 may be performed to complete the subsequent steps of the method for non-invasively estimating blood glucose. If the number of the plurality of ECG waveforms that have been received is less than the default value (i.e., "Y" as shown in FIG. 25), then the plurality of ECG waveforms of the user may be received again (i.e., return to step S610) until the number of the plurality of ECG waveforms that have been received is greater than the default value.

By means of the method for non-invasively estimating blood glucose as shown in FIG. 25, it is possible to not only provide the user with a means for non-invasively estimating the blood glucose value of the user, but also to estimate the blood glucose value of the user more accurately by utilizing an enough quantity of the plurality of ECG waveforms. That is, by carrying out the method for non-invasively estimating blood glucose as shown in FIG. 25, it can ensure that the method described above estimate the blood glucose value of the user (i.e., the result of the estimation of the blood glucose value of the user will not be significantly biased due to the deficiency of the plurality of ECG waveforms) based on the enough quantity of the plurality of ECG waveforms. In addition, by carrying out the method non-invasively estimating blood glucose as shown in FIG. 25, it is possible to improve the technical field regarding non-invasively estimating the blood glucose value of the user by utilizing a plurality of ECG waveforms.

In some embodiments, the method for non-invasively estimating blood glucose may also estimate the blood glucose value of the user based on at least one peak distance, at least one occurrence rate, at least one amplitude ratio, and the sharpness result, such that it is possible to not only provide a user with a means for non-invasively estimating the blood glucose value of the user, but also to estimate the blood glucose value of the user more accurately by utilizing the peak distance, the occurrence rate, the amplitude ratio, and the sharpness result. In addition, by carrying out the method for non-invasively estimating blood glucose described above, it is possible to improve the technical field regarding non-invasively estimating the blood glucose value of the user by utilizing a plurality of ECG waveforms.

Furthermore, in some embodiments, the method for non-invasively estimating blood glucose described above may further include step S910 as shown in FIG. 9, and then it may calculate a calibrated blood glucose value of the user by performing step S910.

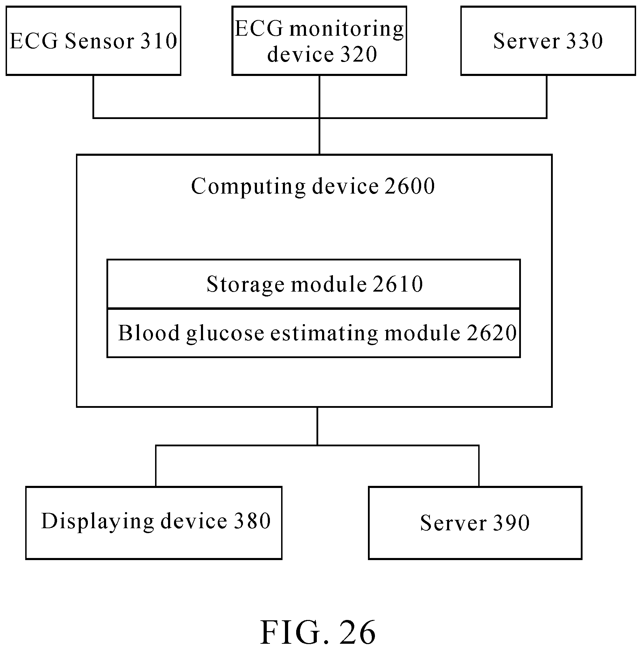

Referring to FIG. 26, FIG. 26 is a schematic diagram illustrating an example of a computing device 2600 for non-invasively estimating blood glucose that is electrically coupled with at least one of an ECG sensor 310, an ECG monitoring device 320, and a server 330 and with at least one of a displaying device 380 and a server 390 according to an embodiment of the present disclosure.

Since the computing device 2600 is electrically coupled with at least one of the ECG sensors 310, the ECG monitoring device 320, and the server 330, the computing device 2600 may receive the plurality of ECG waveforms of the user from at least one of the ECG sensors 310, the ECG monitoring device 320, and the server 330. In some embodiments, the computing device 2600 may receive the plurality of ECG waveforms of the user from at least one of the ECG sensors 310, the ECG monitoring device 320, and the server 330 via a physical signal line connection. For example, the physical signal line connection may be a network signal line connection that conforms with an Internet Protocol (IP), but is not limited thereto. In addition, in some embodiments, the computing device 2600 may also receive the plurality of ECG waveforms of the user from at least one of the ECG sensors 310, the ECG monitoring device 320, and the server 330 via a virtual signal line connection. For example, the virtual signal line connection may be a Wi-Fi connection that conforms with the wireless network protocol, but is not limited thereto.

In some embodiments, the computing device 2600 may also be electrically coupled with other devices that are capable of providing the plurality of ECG waveforms (e.g., wearable measurement devices) to receive the plurality of ECG waveforms of the user from other devices (not shown).

The computing device 2600 may include a storage module 2610 and a blood glucose estimating module 2620 such that the computing device 2600 may perform the specific signal processing on the plurality of ECG waveforms that have been received. That is, after the computing device 2600 receives the plurality of ECG waveforms of the user, the computing device 2600 may estimate a blood glucose value of the user and/or calculate a calibrated blood glucose value of the user based on the plurality of ECG waveforms of the user, and output the estimated blood glucose value of the user and/or the calibrated blood glucose value of the user.

Since the computing device 2600 is electrically coupled with at least one of the display device 380 and the server 390, the computing device 2600 may output the estimated blood glucose value of the user and/or the calibrated blood glucose value of the user to at least one of the display device 380 and the server 390.

In some embodiments, the computing device 2600 may also be electrically coupled with other devices that is capable of receiving, utilizing, storing, and/or displaying the estimated blood glucose value of the user and/or the calibrated blood glucose value of the user to the other devices (not shown) for subsequent use.

The storage module 2610 may be configured to store a program, a series of codes, and/or a series of instruction sets. In some embodiments, the storage module 2610 may include one or more non-volatile memories, and one or more volatile memories, but is not limited thereto. The non-volatile memory may be, for example, read-only memory, flash memory, or non-volatile random access memory, but is not limited thereto. The volatile memory may be, for example, dynamic random access memory or static random access memory, but is not limited thereto. In some embodiments, the storage module 2610, especially the non-volatile memory, can store a program, a series of codes, and/or a series of instruction sets regarding the methods for non-invasively estimating blood glucose as described above.

The blood glucose estimating module 2620 may be configured to be electrically coupled with the storage module 2610, and it may be configured to estimate the blood glucose value of the user based on the plurality of ECG waveforms of the user. That is, after the plurality of ECG waveforms of the user have been received, the blood glucose estimating module 2620 can performs the steps of any one of the methods for non-invasively estimating blood glucose as described above, such that the blood glucose estimating module 2620 can estimate the blood glucose value of the user based on the plurality of ECG waveforms of the user. In some embodiments, the blood glucose estimating module 2620 can also calculate the calibrated blood glucose value of the user.

In some embodiments, the blood glucose estimating module 2620 may include one or more processors, but is not limited thereto. The processor may be, for example, a central processing unit, but is not limited thereto.

Since the storage module 2610 stores a program, a series of codes, and/or a series of instruction sets regarding the methods for non-invasively estimating blood glucose as described above, the blood glucose estimating module 2620, after loading and executing the program, the codes, and/or the instruction sets, can carry out any one of the methods for non-invasively estimating blood glucose as described above by the blood glucose estimating module, especially by the processor.

In some embodiments, the computing device 2600 may further include a signal outputting module (not shown). The signal outputting module may be configured to be electrically coupled with the blood glucose estimating module 2620 and it can be configured to output the blood glucose value of the user that is estimated and/or the calibrated blood glucose value of the user that is calculated by the blood glucose estimating module 2620.

Thereby, the computing device 2600 provided in the present disclosure can be used to perform the steps of any of the methods for non-invasive glucose estimation as described above, such that the computing device 2600 can estimate the blood glucose value of the user based on the plurality of ECG waveforms of the user after the plurality of ECG waveforms of the user have been received.

By means of the computing device 2600 as shown in FIG. 26, it is possible to not only provide a user with a means for non-invasively estimating the blood glucose value of the user, but also to estimate the blood glucose value of the user more accurately. In addition, by means of the computing device 2600 as shown in FIG. 26, it is possible to improve the technical field regarding non-invasively estimating the blood glucose value of the user by utilizing a plurality of ECG waveforms.

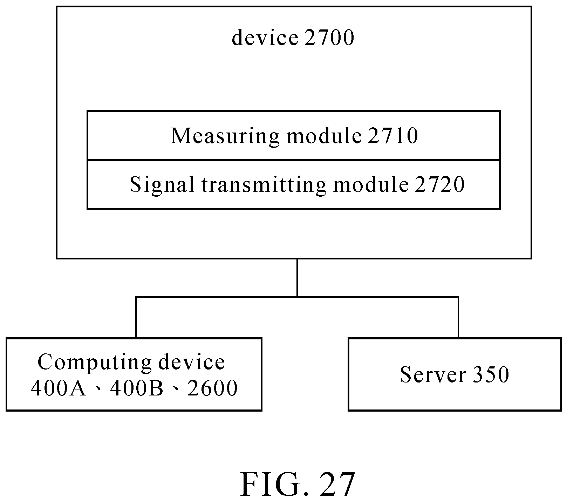

Referring to FIG. 27, FIG. 27 is a schematic diagram illustrating an example of a device 2700 for non-invasively measuring ECG signal that is electrically coupled with at least one of a computing device 2200, a computing device 400A, a computing device 400B, and a server 350 according to an embodiment of the present disclosure.

Since the device 2700 is signally connected with at least one of the computing device 2600, the computing device 400A, the computing device 400B, and the server 350, the device 2700 may provide the user with the plurality of ECG waveforms to at least one of the computing device 2600, the computing device 400A, the computing device 400B, and the server 350. In some embodiments, the device 2700 may provide the plurality of ECG waveforms of the user to at least one of the computing device 2600, the computing device 400A, the computing device 400B, and the server 350 via a physical signal line connection. For example, the physical signal line connection may be a network signal line connection that conforms with an Internet Protocol (IP), but is not limited thereto. In addition, in some embodiments, the device 2700 may also provide the plurality of ECG waveforms of the user to at least one of the computing device 2600, the computing device 400A, the computing device 400B, and the server 350 via a virtual signal line connection. For example, the virtual signal line connection may be a Wi-Fi connection that conforms with the wireless network protocol, but is not limited thereto.

The device 2700 may include a measuring module 2710 and a signal transmitting module 2720, such that the device 2700 may provide the plurality of ECG waveforms of the user to at least one of the computing device 2600, the computing device 400A, the computing device 400B, and the server 350 so as to make at least one of the computing device 2600, the computing device 400A, the computing device 400B, and the server 350 perform the steps of any of the methods for non-invasively estimating blood glucose as described above to estimate the blood glucose value of the user based on the plurality of ECG waveforms that is measured by the device 2700.

The measuring module 2710 may be configured to have a plurality of electrodes that are electrically connected to the user, and may be configured to measure the plurality of ECG waveforms of the user. Since the depolarization process of the heart causes the voltage changes on the surface of the body, the voltage changes on the surface of the skin of the user may be measured by the plurality of electrodes electrically connected to the surface of the skin of the user in order to obtain the plurality of ECG waveforms of the user.

The signal transmitting module 2720 may be configured to be electrically coupled with the measuring module 2710 and may be configured to transmit the plurality of ECG waveforms of the user to at least one of the computing device 2600, the computing device 400A, the computing device 400B, and the server 350, and then make at least one of the computing device 2600, the computing device 400A, the computing device 400B, and the server 350 perform the steps of any one of the methods for non-invasively estimating blood glucose as described above.

Thereby, the device 2700 provided in the present disclosure can be used to transmit the plurality of ECG waveforms of the user that have been measured to at least one of the computing device 2600, the computing device 400A, the computing device 400B, and the server 350, such that at least one of the computing device 2600, the computing device 400A, the computing device 400B, and the server 350 can estimate the blood glucose value of the user based on the plurality of ECG waveforms that have been measured by the device 2700.

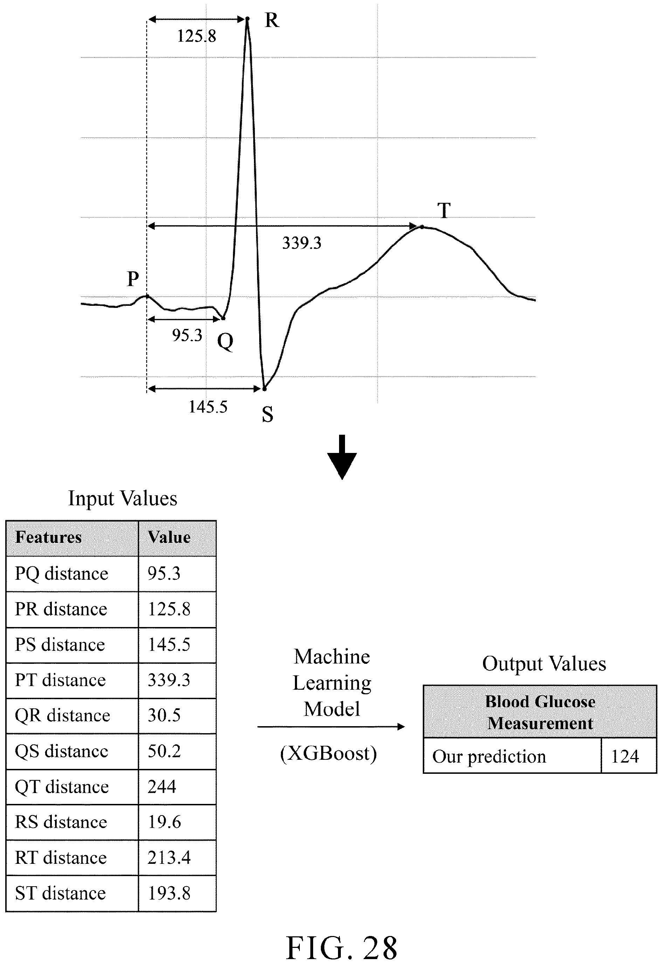

Referring to FIG. 28 is a schematic diagram illustrating that the blood glucose value of the user is estimated based on the plurality of ECG waveforms that have been received from the user according to an embodiment of the present disclosure.

The method for non-invasively estimating blood glucose can be carried out by the computing device as described above. In particular, the computing device as described above can estimate the blood glucose value of the user by utilizing the plurality of ECG waveforms of the user, especially the peak distance. For example, the peak distance can be calculated based on the plurality of ECG waveforms as shown in FIG. 28, and then the blood glucose value of the user can be estimated based on the peak distance. As shown in FIG. 28, the peak distance can be input into the machine learning model, i.e., an extreme gradient boosting (XGBoost) algorithm, and then the blood glucose value of the user can be estimated by the machine learning model. As shown in FIG. 28, the blood glucose value of the user that is estimated is 124 mg/dL, and the actual blood glucose value of the user that is measured by a blood glucose meter is 128 in this case. Since the result of the estimation for the blood glucose value is close to the actual blood glucose value, the present disclosure can be used to not only provide a user with a means for non-invasively estimating the blood glucose value of the user, but also to estimate the blood glucose value of the user more accurately by utilizing the plurality of ECG waveforms, especially the peak distance described above.

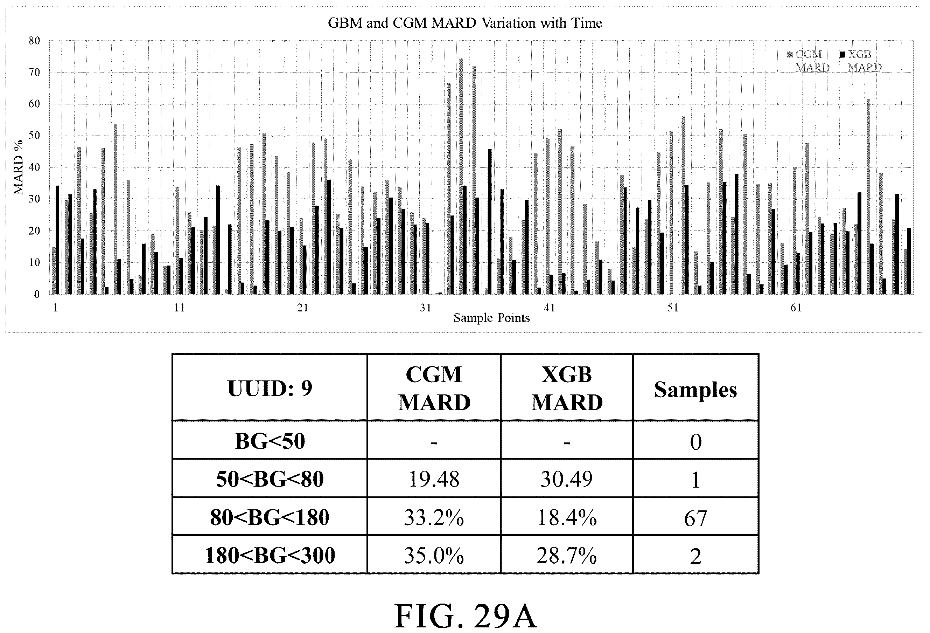

Referring to FIG. 29A, FIG. 29A is a schematic diagram illustrating a comparison result for the blood glucose value that is estimated by the present disclosure compared to another device.

As shown in FIG. 29A, "UUID: 48" is the ID of the subject, "CGM MARD" is the error between the real blood glucose value and the monitoring blood glucose value by another device, and "XGB MARD" is the error between the real blood glucose value and the estimated blood glucose value by the device provided in the present disclosure. The comparison results show that the results of the estimation of "XGB MARD" are more accurate than the monitoring results of "CGM MARD". In some embodiments, "MARD" can be calculated by an equation, $MARD = \frac{1}{N}\sum_{i=1}^{N}\left|\frac{BGi-Compi}{Compi}\right|$, wherein the "BGi" is the estimated blood glucose value, the "Compi" is the real blood glucose value, and the "N" is the number of sampling points. For example, when the estimated blood glucose value is {105, 95, 85} and the real blood glucose value is {100, 90, 90}, "MARD" can be calculated and then the calculated result of the "MARD" is 5.37%. (

$$MARD = \frac{1}{3}\sum_{i=1}^{3}\left|\frac{BGi-Compi}{Compi}\right| = \frac{1}{3}\left(\left|\frac{105-100}{100}\right| + \left|\frac{95-90}{90}\right| + \left|\frac{85-90}{90}\right|\right) = 5.37\%$$

)

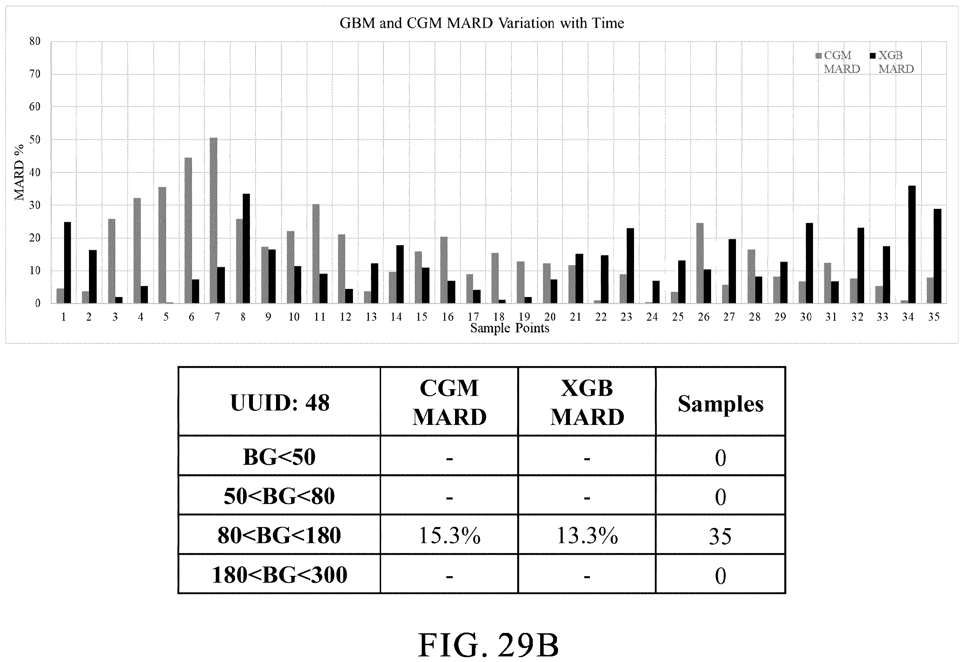

Referring to FIG. 29B, FIG. 29B is a schematic diagram illustrating another comparison result for the blood glucose value that is estimated by the present disclosure compared to another device.

As shown in FIG. 29B, "UUID: 9" is the ID of the subject (i.e., another subject), "CGM MARD" is another device for monitoring the blood glucose value of the user, and "XGB MARD" is the device provided in the present disclosure. The comparison results also show that the results of the estimation of "XGB MARD" are more accurate than the monitoring results of "CGM MARD".

In some embodiments, the steps of the method for non-invasively estimating blood glucose as described above may be stored in a non-transitory computer-readable storage medium in a series of particular codes or a series of particular instruction sets, the non-transitory computer-readable storage medium may be, for example, a hard disk, a CD-ROM, a magnetic disk, or a USB disk, but is not limited thereto. The non-transitory computer-readable storage media, when loaded and executed by a computer with the stored codes or instruction sets, enables the method for non-intrusively estimating blood glucose as described above.

While the present disclosure has been described by means of specific embodiments, numerous modifications and variations could be made thereto by those skilled in the art without departing from the scope and spirit of the present disclosure set forth in the claims. Therefore, the protection of this application shall be as defined in the claims instead of the contents disclosed in the specification.

**Claims**

1. A method for non-invasively estimating blood glucose, which is suitable for estimating a blood glucose value of a user by a computing device (400A, 400B, 2600), the method comprising:

   receiving a plurality of electrocardiogram (ECG) waveforms of the user;
   extracting at least two first ECG features from each of the plurality of ECG waveforms of the user;
   determining a first feature peak position corresponding to each of the first ECG features, respectively;
   calculating at least one peak distance between first feature peak positions for each of the plurality of ECG waveforms; and
   estimating the blood glucose value of the user based on the peak distance,
   wherein the first ECG features are selected from the group consisting of a P-wave, a Q-wave, an R-wave, an S-wave, a T-wave, and a U-wave.

2. The method for non-invasively estimating blood glucose according to claim 1, wherein estimating the blood glucose value of the user based on the peak distance comprises:

   normalizing the peak distance;
   inputting the normalized peak distance into a machine learning model (500); and
   outputting the blood glucose value of the user by the machine learning model (500),
   wherein the blood glucose value of the user is estimated based on the normalized peak distance by the machine learning model (500).

3. The method for non-invasively estimating blood glucose according to claim 1, wherein estimating the blood glucose value of the user based on the peak distance comprises:

normalizing the peak distance;
inputting the normalized peak distance into a neural network model (510); and
outputting the blood glucose value of the user by the neural network model (510),
wherein the blood glucose value of the user is estimated based on the normalized peak distance by the neural network model (510).

4. The method for non-invasively estimating blood glucose according to claim 1, further comprising:
calculating a calibrated blood glucose value of the user based on the blood glucose value of the user by an equation of calibrated blood glucose value.

5. The method for non-invasively estimating blood glucose according to claim 1, further comprising:

calculating at least one peak-to-peak slope between first feature peak positions for each of the plurality of ECG waveforms,
wherein estimating the blood glucose value of the user is further based on the peak-to-peak slope.

6. The method for non-invasively estimating blood glucose according to claim 1, further comprising:

extracting at least three second ECG features from each of the plurality of ECG waveforms of the user; and
calculating at least one occurrence rate based on the second ECG features,
wherein estimating the blood glucose value of the user is further based on the occurrence rate, and
wherein the second ECG features are selected from the group consisting of a P-wave, a Q-wave, an R-wave, an S-wave, a T-wave, and a U-wave.

7. The method for non-invasively estimating blood glucose according to claim 6, wherein calculating the occurrence rate based on the second ECG features comprises:

determining an occurrence interval in each of the plurality of ECG waveforms of the user;
calculating a first peak-to-peak value and a second peak-to-peak value within the occurrence interval for each of the plurality of ECG waveforms based on the second ECG features;
calculating a peak-to-peak ratio of the first peak-to-peak value to the second peak-to-peak value for each of the plurality of ECG waveforms;
comparing the peak-to-peak ratio with a default ratio and generating a comparison result, for each of the plurality of ECG waveforms; and
calculating the occurrence rate based on the plurality of the comparison results.

8. The method for non-invasively estimating blood glucose according to claim 6, wherein estimating the blood glucose value of the user comprises:

normalizing the peak distance;
inputting the normalized peak distance and the occurrence rate into a machine learning model (500); and
outputting the blood glucose value of the user by the machine learning model (500),
wherein the blood glucose value of the user is estimated based on the normalized peak distance and the occurrence rate by the machine learning model (500).

9. The method for non-invasively estimating blood glucose according to claim 6, wherein estimating the blood glucose value of the user comprises:

normalizing the peak distance;
inputting the normalized peak distance and the occurrence rate into a neural network model (510); and
outputting the blood glucose value of the user by the neural network model (510),
wherein the blood glucose value of the user is estimated based on the normalized peak distance and the occurrence rate by the neural network model (510).

10. The method for non-invasively estimating blood glucose according to claim 1, further comprising:

extracting at least four third ECG features from each of the plurality of ECG waveforms of the user; and
calculating at least one amplitude ratio based on the third ECG features,
wherein estimating the blood glucose value of the user is further based on the amplitude ratio, and
wherein the third ECG features are selected from the group consisting of a P-wave, a Q-wave, an R-wave, an S-wave, a T-wave, and a U-wave.

11. The method for non-invasively estimating blood glucose according to claim 10, wherein calculating the amplitude ratio based on the third ECG features comprises:

calculating a third peak-to-peak value, a fourth peak-to-peak value, a fifth peak-to-peak value, and a sixth peak-to-peak value for each of the plurality of ECG waveforms based on the third ECG features;
calculating a first average value of the third peak-to-peak value and the fourth peak-to-peak value;
calculating a second average value of the fifth peak-to-peak value and the sixth peak-to-peak value; and
calculating the amplitude ratio of the first average value to the second average value.

12. The method for non-invasively estimating blood glucose according to claim 10, wherein estimating the blood glucose value of the user comprises:

normalizing the peak distance and the amplitude ratio, respectively;
inputting the normalized peak distance and the normalized amplitude ratio into a machine learning model (500); and
outputting the blood glucose value of the user by the machine learning model (500),
wherein the blood glucose value of the user is estimated based on the normalized peak distance and the normalized amplitude ratio by the machine learning model (500).

13. The method for non-invasively estimating blood glucose according to claim 10, wherein estimating the blood glucose value of the user comprises:

normalizing the peak distance and the amplitude ratio, respectively;
inputting the normalized peak distance and the normalized amplitude ratio into a neural network model (510); and
outputting the blood glucose value of the user by the neural network model (510),
wherein the blood glucose value of the user is estimated based on the normalized peak distance and the normalized amplitude ratio by the neural network model (510).

14. The method for non-invasively estimating blood glucose according to claim 1, further comprising:

extracting at least one fourth ECG feature from each of the plurality of ECG waveforms of the user; and
calculating at least one sharpness result based on the fourth ECG feature,
wherein estimating the blood glucose value of the user is further based on the sharpness result, and
wherein the fourth ECG feature is selected from the group consisting of a P-wave, a Q-wave, an R-wave, an S-wave, a T-wave, and a U-wave.

15. The method for non-invasively estimating blood glucose according to claim 14, wherein calculating the sharpness result based on the fourth ECG feature comprises:

determining a fourth feature peak position corresponding to the fourth ECG feature, respectively;
calculating a first slope and a second slope based on the fourth feature peak position, respectively;
calculating a first sharpness based on the first slope; and
calculating a second sharpness based on the second slope.

16. The method for non-invasively estimating blood glucose according to claim 14, wherein estimating the blood glucose value of the user comprises:

normalizing the peak distance and the sharpness result, respectively;
inputting the normalized peak distance and the normalized sharpness result into a machine learning model (500); and

outputting the blood glucose value of the user by the machine learning model (500),
wherein the blood glucose value of the user is estimated based on the normalized peak distance and the normalized sharpness result by the machine learning model (500).

17. The method for non-invasively estimating blood glucose according to claim 14, wherein estimating the blood glucose value of the user comprises:

normalizing the peak distance and the sharpness result, respectively;
inputting the normalized peak distance and the normalized sharpness result into a neural network model (510); and
outputting the blood glucose value of the user by the neural network model (510),
wherein the blood glucose value of the user is estimated based on the normalized peak distance and the normalized sharpness result by the neural network model (510).

18. The method for non-invasively estimating blood glucose according to claim 1, further comprising:

evaluating the quality of the plurality of ECG waveforms of the user;
outputting an immediate blood glucose value of the user when the plurality of ECG waveforms of the user is evaluated as a normal ECG signal; and
outputting a previous blood glucose value of the user when the plurality of ECG waveforms of the user is evaluated as a noise signal,
wherein the immediate blood glucose value of the user is the blood glucose value that is estimated immediately, and
wherein the previous blood glucose value of the user is the blood glucose value that is estimated previously.

19. The method for non-invasively estimating blood glucose according to claim 1, further comprising:

determining whether the number of the plurality of ECG waveforms of the user is less than a default value; and
re-receiving the plurality of ECG waveforms of the user when the number of the plurality of ECG waveforms of the user is less than the default value.

20. A computing device (2600) for non-invasively estimating blood glucose, which is suitable for signally connecting with an electrocardiogram (ECG) measuring device in order to receive a plurality of ECG waveforms of a user from the ECG measuring device, the computing device (2600) comprising:

a storage module (2610); and
a blood glucose estimating module (2620), configured to be signally connected with the storage module (2610);
wherein a plurality of codes is stored in the storage module (2610), and
wherein the blood glucose estimating module (2620) performs the steps of the method for non-invasively estimating blood glucose according to any one of claims 1 to 19 after the blood glucose estimating module (2620) executes the plurality of codes stored in the storage module (2610).

21. A device (2700) for non-invasively measuring electrocardiogram (ECG) signal, which is suitable for electrically coupling with a computing device (400A, 400B, 2600) in order to output a plurality of ECG waveforms of a user to the computing device (400A, 400B, 2600), the device (2700) comprising:

a measuring module (2710), having a plurality of electrodes that electrically connect to the user; and
a signal transmitting module (2720), configured to be electrically coupled with the measuring module (2710), and
wherein the measuring module (2710) is configured to be used to measure the plurality of ECG waveforms of the user, and
wherein the signal transmitting module (2720) is configured to be used to transmit the plurality of ECG waveforms of the user to the computing device (400A, 400B, 2600) and then make the computing device (400A, 400B, 2600) be able to perform the steps of the method for non-invasively estimating blood glucose according to any one of claims 1 to 19.

22. A non-transitory computer readable storage medium having codes recorded thereon for a computing device (400A, 400B, 2600) to perform, after executing the codes, a method for non-invasively estimating blood glucose according to any one of claims 1 to 19.

First Example:

Second Example:

FIG. 1

One of the plurality of ECG waveforms

R-wave

P-wave

T-wave

U-wave

Q-wave

S-wave

FIG. 2

## Calculating at least one peak distance :

FIG. 3

ECG Measuring Device 300

## Computing Device 400A

Receiving Unit 402

Extracting Unit 404

Determining Unit 406

Calculating Unit 408

Estimating Unit 410

ECG Waveforms
Database 452

Blood Glucose Value
Database 454

Machine Learning
Model 500

Neural Network
Model 510

Convolutional Neural
Network Model 520

## FIG. 4

Determining Unit 406

Calculating Unit 408

Peak Distance Calculator 502

Occurrence Rate Calculator 504

Amplitude Ratio Calculator 506

Calibrated Blood Glucose Value
Calculator 508

Estimating Unit 410

FIG. 5

Receiving a plurality of ECG waveforms  S610

Extracting at least two first ECG features  S620

Determining a first feature peak position corresponding to each of the first ECG features, respectively  S630

Calculating at least one peak distance  S640

Estimating the blood glucose value of the user based on the peak distance  S650

FIG. 6

S650

Normalizing the peak distance — S710A

Inputting the normalized peak distance into a machine learning model — S720A

Estimating the blood glucose value of the user by the machine learning model — S730A

Outputting the blood glucose value of the user by the machine learning model — S740A

FIG. 7A

S650

Normalizing the peak distance — S710B

Inputting the normalized peak distance into a neural network model — S720B

Estimating the blood glucose value of the user by the neural network model — S730B

Outputting the blood glucose value of the user by the neural network model — S740B

FIG. 7B

Data (Features & Labels): X

$Tree_1\{X, \theta_1\}$  $Tree_2\{X, \theta_2\}$  $Tree_3\{X, \theta_3\}$  $Tree_n\{X, \theta_n\}$

$f_1(X, \theta_1)$  Residual  $f_2(X, \theta_2)$  Residual  $f_3(X, \theta_3)$  Residual  $f_n(X, \theta_n)$

$$\sum_{i=1}^{n} f_i(X, \theta_i)$$

FIG. 8A

EP 4 574 023 A1

FIG. 8B

EP 4 574 023 A1

FIG. 8C

Normalized
ECG signal

(Batch, 1, Signal)

**Conv. 1**
kernel: k1
stride: s1
padding: p1
output: y1
**Max pool.**
**Activation fn.**
**Batch norm.**

(Batch, y1, Signal)

**Conv. 2**
kernel: k2
stride: s2
padding: p2
output: y2
**Max pool.**
**Activation fn.**
**Batch norm.**

(Batch, y2, Signal)

**Conv. 3**
kernel: k3
stride: s3
padding: p3
output: y3
**Max pool.**
**Activation fn.**
**Batch norm.**

(Batch, y3, Signal)

# FIG. 8D

Receiving a plurality of ECG waveforms — S610

Extracting at least two first ECG features — S620

Determining a first feature peak position corresponding to each of the first ECG features, respectively — S630

Calculating at least one peak distance — S640

Estimating the blood glucose value of the user based on the peak distance — S650

Calculating a calibrated blood glucose value of the user based on the blood glucose value of the use — S910

FIG. 9

Receiving a plurality of ECG waveforms — S610

Extracting at least two first ECG features — S620

Determining a first feature peak position corresponding to each of the first ECG features, respectively — S630

Calculating at least one peak distance — S640

Calculating at least one peak-to-peak slope — S1010

Estimating the blood glucose value of the user based on the peak distance and the peak-to-peak slope — S1020

FIG. 10

| Receiving a plurality of ECG waveforms | S610 |

| Extracting at least two first ECG features | S620 |

| Extracting at least three second ECG features | S1110 |

| Determining a first feature peak position corresponding to each of the first ECG features, respectively | S630 |

| Calculating at least one peak distance | S640 |

| Calculating at least one occurrence rate | S1120 |

| Estimating the blood glucose value of the user based on the peak distance and the occurrence rate | S1130 |

FIG. 11

S1120

S1210

Determining an occurrence interval

S1220

Calculating a first peak-to-peak value and
a second peak-to-peak value within the
occurrence interval, respectively

S1230

Calculating a peak-to-peak ratio of the first
peak-to-peak value to the second
peak-to-peak value, respectively

S1240

Comparing the peak-to-peak ratio with
a default ratio, respectively

S1250

Generating a comparison result, respectively

S1260

Calculating the occurrence rate based on
the plurality of the comparison results

FIG. 12

Calculating at least one occurrence rate:

FIG. 13

S1130

S1410A

Normalizing the peak distance

S1420A

Inputting the normalized peak distance and the occurrence rate into a machine learning model

S1430A

Estimating the blood glucose value of the user by the machine learning model

S1440A

Outputting the blood glucose value of the user by the machine learning model

FIG. 14A

S1130

S1410B

Normalizing the peak distance

S1420B

Inputting the normalized peak distance
and the occurrence rate into a neural
network model

S1430B

Estimating the blood glucose value of
the user by the neural network model

S1440B

Outputting the blood glucose value of
the user by the neural network model

FIG. 14B

Receiving a plurality of ECG waveforms — S610

Extracting at least two first ECG features — S620

Extracting at least four third ECG features — S1510

Determining a first feature peak position corresponding to each of the first ECG features, respectively — S630

Calculating at least one peak distance — S640

Calculating at least one amplitude ratio — S1520

Estimating the blood glucose value of the user based on the peak distance and the amplitude ratio — S1530

FIG. 15

S1520

S1610

Calculating a third peak-to-peak value,
a fourth peak-to-peak value, a fifth peak-to-peak
value and a sixth peak-to-peak value

S1620

Calculating a first average value of the
third peak-to-peak value and the fourth
peak-to-peak value

S1630

Calculating a second average value of the
fifth peak-to-peak value and the sixth
peak-to-peak value

S1640

Calculating the amplitude ratio of the first
average value to the second average value

FIG. 16

Calculating at least one amplitude ratio:

FIG. 17A

Calculating at least one amplitude ratio:

FIG. 17B

S1530

S1810A

Normalizing the peak distance and the
amplitude ratio, respectively

S1820A

Inputting the normalized peak distance
and the normalized amplitude ratio into
a machine learning model

S1830A

Estimating the blood glucose value of
the user by the machine learning model

S1840A

Outputting the blood glucose value of
the user by the machine learning model

FIG. 18A

S1530

S1810B

Normalizing the peak distance and the amplitude ratio, respectively

S1820B

Inputting the normalized peak distance and the normalized amplitude ratio into a neural network model

S1830B

Estimating the blood glucose value of the user by the neural network model

S1840B

Outputting the blood glucose value of the user by the neural network model

FIG. 18B

| | |
|---|---|
| Receiving a plurality of ECG waveforms | S610 |

↓

| | |
|---|---|
| Extracting at least two first ECG features | S620 |

↓

| | |
|---|---|
| Extracting at least one fourth ECG feature | S1910 |

↓

| | |
|---|---|
| Determining a first feature peak position corresponding to each of the first ECG features, respectively | S630 |

↓

| | |
|---|---|
| Calculating at least one peak distance | S640 |

↓

| | |
|---|---|
| Calculating at least one sharpness result | S1920 |

↓

| | |
|---|---|
| Estimating the blood glucose value of the user based on the peak distance and the sharpness result | S1930 |

FIG. 19

S1920

S2010

Determining a fourth feature peak position corresponding to the fourth ECG feature, respectively

S2020

Calculating a first slope and a second slope based on the fourth feature peak position, respectively

S2030

Calculating a first sharpness based on the first slope

S2040

Calculating a second sharpness based on the second slope

FIG. 20

Calculating the sharpness result:

FIG. 21

S1930

Normalizing the peak distance and the
sharpness result, respectively
S2210A

Inputting the normalized peak distance
and the normalized sharpness result into
a machine learning model
S2220A

Estimating the blood glucose value of
the user by the machine learning model
S2230A

Outputting the blood glucose value of
the user by the machine learning model
S2240A

FIG. 22A

S1930

S2210B

Normalizing the peak distance and the
sharpness result, respectively

S2220B

Inputting the normalized peak distance
and the normalized sharpness result into
a neural network model

S2230B

Estimating the blood glucose value of
the user by the neural network model

S2240B

Outputting the blood glucose value of
the user by the neural network model

FIG. 22B

ECG Measuring Device 300

Computing Device 400B

Receiving Unit 402

Extracting Unit 404

Determining Unit 406

Calculating Unit 408

Estimating Unit 410

Evaluating Unit 412

Counting Unit 414

Outputting Unit 416

ECG Waveforms
Database 452

Blood Glucose
Value Database 454

Machine Learning
Model 500

Neural Network
Model 510

Convolutional Neural
Network Model 520

FIG. 23

Receiving a plurality of ECG waveforms — S610

Evaluating the quality of the plurality of ECG waveforms — S2410

NS

NM

Outputting a previous blood glucose value of the user

S2420

Outputting an immediate blood glucose value of the user

S2430

FIG. 24

Receiving a plurality of ECG waveforms — S610

determining whether
the number of the plurality of ECG
waveforms of the user is less than
a default value — S2510

Y

N

Extracting at least two first ECG features — S620

Determining a first feature peak position
corresponding to each of the first
ECG features, respectively — S630

Calculating at least one peak distance — S640

Estimating the blood glucose value of
the user based on the peak distance — S650

FIG. 25

```
┌─────────────────────┐  ┌─────────────────────┐  ┌─────────────────────┐
│                     │  │   ECG monitoring    │  │                     │
│  ECG Sensor 310     │  │    device 320       │  │    Server 330       │
│                     │  │                     │  │                     │
└─────────────────────┘  └─────────────────────┘  └─────────────────────┘
```

Computing device 2600

Storage module 2610

Blood glucose estimating module 2620

Displaying device 380

Server 390

FIG. 26

device 2700

Measuring module 2710

Signal transmitting module 2720

Computing device
400A、400B、2600

Server 350

FIG. 27

Input Values

| Features | Value |
|---|---|
| PQ distance | 95.3 |
| PR distance | 125.8 |
| PS distance | 145.5 |
| PT distance | 339.3 |
| QR distance | 30.5 |
| QS distance | 50.2 |
| QT distance | 244 |
| RS distance | 19.6 |
| RT distance | 213.4 |
| ST distance | 193.8 |

Machine
Learning
Model

(XGBoost)

Output Values

| Blood Glucose Measurement | |
|---|---|
| Our prediction | 124 |

FIG. 28

GBM and CGM MARD Variation with Time

| UUID: 9 | CGM MARD | XGB MARD | Samples |
|---|---|---|---|
| BG<50 | - | - | 0 |
| 50<BG<80 | 19.48 | 30.49 | 1 |
| 80<BG<180 | 33.2% | 18.4% | 67 |
| 180<BG<300 | 35.0% | 28.7% | 2 |

FIG. 29A

EP 4 574 023 A1

GBM and CGM MARD Variation with Time

| UUID: 48 | CGM MARD | XGB MARD | Samples |
|---|---|---|---|
| BG<50 | - | - | 0 |
| 50<BG<80 | - | - | 0 |
| 80<BG<180 | 15.3% | 13.3% | 35 |
| 180<BG<300 | - | - | 0 |

FIG. 29B

EP 4 574 023 A1

EP 4 574 023 A1

Europäisches Patentamt
European Patent Office
Office européen des brevets

# EUROPEAN SEARCH REPORT

**Application Number**

EP 24 15 0461

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CORDEIRO RENATO ET AL: "Hyperglycemia Identification Using ECG in Deep Learning Era",<br>SENSORS, [Online]<br>vol. 21, no. 18,<br>18 September 2021 (2021-09-18), page 6263,<br>XP093134571,<br>CH<br>ISSN: 1424-8220, DOI: 10.3390/s21186263<br>Retrieved from the Internet:<br>URL:https://www.ncbi.nlm.nih.gov/pmc/articles/PMC8472987/pdf/sensors-21-06263.pdf><br>[retrieved on 2024-02-16]<br>* abstract *<br>* Section Introduction *<br>* Section Dataset *<br>* Section Features extraction *<br>* Section Normalization *<br>* Section Models and Metrics *<br>* Section Hardware and Software *<br>* Section Challenge *<br>* figure 3; table 2 *<br>-----<br>-/-- | 1-22 | INV.<br>A61B5/00<br>A61B5/145<br>A61B5/0245<br><br><br>TECHNICAL FIELDS SEARCHED (IPC)<br><br>A61B<br>G16H<br>G06F |

The present search report has been drawn up for all claims

| Place of search | Date of completion of the search | Examiner |
|---|---|---|
| The Hague | 7 March 2024 | Lai, Marco |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 3**

Europäisches
Patentamt

European
Patent Office

Office européen
des brevets

# EUROPEAN SEARCH REPORT

**Application Number**

EP 24 15 0461

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FELLAH ARBI KHADIDJA ET AL: "Blood glucose estimation based on ECG signal", PHYSICAL AND ENGINEERING SCIENCES IN MEDICINE, [Online] vol. 46, no. 1, 3 January 2023 (2023-01-03), pages 255-264, XP093134029, Cham ISSN: 2662-4729, DOI: 10.1007/s13246-022-01214-3 Retrieved from the Internet: URL:https://link.springer.com/content/pdf/10.1007/s13246-022-01214-3.pdf> [retrieved on 2024-02-23] * abstract * * Section Introduction * * Section Dataset * * Section Signal segmentation * * Section Features extraction * * Section BGC estimation * * Section Methodology * | 1-22 | |
| A | WO 2020/072989 A2 (MEDTRONIC INC [US]) 9 April 2020 (2020-04-09) * paragraph [0115] * | 1-15 | **TECHNICAL FIELDS SEARCHED (IPC)** |
| A | Ostrander Leon D.: "Electrocardiographic Changes after Glucose Ingestion", , 1 July 1964 (1964-07-01), pages 67-76, XP093134263, Retrieved from the Internet: URL:https://www.ahajournals.org/doi/pdf/10.1161/01.CIR.30.1.67 [retrieved on 2024-02-22] * Section Results, ST-segment and T-wave Changes after Oral Glucose Administration * | 1-15 | |

-/--

The present search report has been drawn up for all claims

| Place of search | Date of completion of the search | Examiner |
|---|---|---|
| The Hague | 7 March 2024 | Lai, Marco |

EPO FORM 1503 03.82 (P04C01)

page 2 of 3

Europäisches
Patentamt

European
Patent Office

Office européen
des brevets

**EUROPEAN SEARCH REPORT**

Application Number

EP 24 15 0461

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2022/346676 A1 (PECCHIA LEANDRO [GB] ET AL) 3 November 2022 (2022-11-03) <br> * paragraph [0009] * <br> * paragraph [0066] * <br> * paragraph [0130] * <br> ----- | 1-15 | |
| A | Rad Mohammad Assadian ET AL: "Hyperglycemia-Induced T-Wave Oversensing as a Cause of Cardiac Resynchronization Therapy (CRT) Failure", Journal of Tehran University Heart Center, 1 January 2012 (2012-01-01), pages 30-32, XP093134445, Tehran Retrieved from the Internet: URL:https://www.ncbi.nlm.nih.gov/pmc/articles/PMC3466884/pdf/jthc-7-30.pdf [retrieved on 2024-02-23] * abstract * ----- | 1-15 | |
| A | ANDELLINI MARTINA ET AL: "Artificial intelligence for non-invasive glycaemic-events detection via ECG in a paediatric population: study protocol", HEALTH AND TECHNOLOGY, [Online] vol. 13, no. 1, 23 January 2023 (2023-01-23), pages 145-154, XP093134025, Berlin/Heidelberg ISSN: 2190-7188, DOI: 10.1007/s12553-022-00719-x Retrieved from the Internet: URL:https://www.ncbi.nlm.nih.gov/pmc/articles/PMC9899724/pdf/12553_2022_Article_719.pdf> [retrieved on 2024-02-23] * abstract * ----- | 1-15 | **TECHNICAL FIELDS SEARCHED (IPC)** |

The present search report has been drawn up for all claims

| Place of search | Date of completion of the search | Examiner |
|---|---|---|
| The Hague | 7 March 2024 | Lai, Marco |

**page 3 of 3**

## ANNEX TO THE EUROPEAN SEARCH REPORT
## ON EUROPEAN PATENT APPLICATION NO.

EP 24 15 0461

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2024

| Patent document cited in search report | | Publication date | Patent family member(s) | | Publication date |
|---|---|---|---|---|---|
| WO 2020072989 | A2 | 09-04-2020 | CN | 112789083 A | 11-05-2021 |
| | | | EP | 3860710 A2 | 11-08-2021 |
| | | | JP | 7333811 B2 | 25-08-2023 |
| | | | JP | 2022503565 A | 12-01-2022 |
| | | | KR | 20210072755 A | 17-06-2021 |
| | | | US | 2020108260 A1 | 09-04-2020 |
| | | | US | 2023330425 A1 | 19-10-2023 |
| | | | WO | 2020072989 A2 | 09-04-2020 |
| US 2022346676 | A1 | 03-11-2022 | EP | 4021300 A1 | 06-07-2022 |
| | | | GB | 2586788 A | 10-03-2021 |
| | | | US | 2022346676 A1 | 03-11-2022 |
| | | | WO | 2021038229 A1 | 04-03-2021 |

EPO FORM P0459

For more details about this annex : see Official Journal of the European Patent Office, No. 12/82